# EUROPEAN PATENT SPECIFICATION

(11) **EP 3 194 623 B1**
(45) Date of publication and mention of the grant of the patent: **05.01.2022**
(21) Application number: 15831338.7
(22) Date of filing: 11.08.2015
(51) Int. Cl.: G01N 33/50, C12N 5/073, C12N 5/0735, C12Q 1/6897

(54) **SYSTEMS AND METHODS TO DETECT STEM CELL STRESS AND USES THEREOF**
SYSTEME UND VERFAHREN ZUR ERKENNUNG VON STRESS VON STAMMZELLEN UND VERWENDUNGEN DAVON
SYSTÈMES ET MÉTHODES DE DÉTECTION DU STRESS DANS LES CELLULES SOUCHES ET LEURS UTILISATIONS

(30) Priority: 12.08.2014 US 201462036549 P; 11.05.2015 US 201562159675 P
(43) Date of publication of application: 26.07.2017
(73) Proprietor: Wayne State University, Detroit, MI 48202 (US)
(72) Inventor: PUSCHECK, Elizabeth, Detroit, Michigan 48202 (US); RAPPOLEE, Daniel A., Detroit, Michigan 48202 (US)
(74) Representative: Forresters IP LLP
(86) International application number: PCT/US2015/044701
(87) International publication number: WO 2016/025510

(56) References cited:
- WO-A1-2011/026222
- WO-A1-2011/029798
- WO-A2-2007/117573
- JP-A- 2011 087 475
- US-A1- 2013 004 959
- US-A1- 2013 302 815
- PUSCHECK E E ET AL: "SAPK activity is a better marker than AMPK activity for measuring stressful O2 levels in stem cells", FERTILITY AND STERILITY, vol. 100, no. 3, 1 September 2013 (2013-09-01), XP028702306, ISSN: 0015-0282, DOI: 10.1016/J.FERTNSTERT.2013.07.483
- GIOVANNA G. LARA ET AL: "Fluid Flow Modulation of Murine Embryonic Stem Cell Pluripotency Gene Expression in the Absence of LIF", CELLULAR AND MOLECULAR BIOENGINEERING, vol. 6, no. 3, 1 September 2013 (2013-09-01), pages 335-345, XP055446029, Boston ISSN: 1865-5025, DOI: 10.1007/s12195-013-0287-6
- WU ET AL.: 'Combinatorial Signals of Activin/Nodal and Bone Morphogenic Protein Regulate the Early Lineage Segregation of Human Embryonic Stem Cells' JOURNAL OF BIOLOGICAL CHEMISTRY vol. 283, no. 36, 05 September 2008, pages 24991 - 25002, XP055399271 DOI: 10.1074/JBC.M803893200

## Description

### CROSS REFERENCE TO RELATED APPLICATIONS

This application claims priority to U.S. Provisional Application No. 62/036,549, filed on August 12, 2014, and U.S. Provisional Application No. 62/159,675, filed on May 11, 2015.

### FIELD OF DISCLOSURE

The present disclosure provides systems and methods (S/M) to detect stress in stem cells. The S/M of the invention, including assays and high throughput screens, can be used to identify compounds or other potential stressors that can negatively affect development potential.

### BACKGROUND OF DISCLOSURE

Stem cells are unspecialized cells that are able to renew themselves through cell division for long periods. Stem cells can be subdivided and classified on the basis of their potency. A totipotent stem cell is produced from fusion between an egg and a sperm. Stem cells produced by the 1^{st} few divisions of the fertilized egg cell are also totipotent stem cells. These cells can grow into any type of cell that makes up the developing organism, extra-embryonic tissues (e.g., yolk sac and placenta) and embryo. Pluripotent stem cells are descendants of totipotent cells that can differentiate into any cell type except for placental cells. Multipotent stem cells are descendants of pluripotent stem cells that can produce only cells of a closely related family of cells (e.g. blood cells such as red blood cells, white blood cells and platelets, or all placental lineages). Progenitor (sometimes called unipotent) cells can produce only one cell type, but have the property of self-renewal which distinguishes them from non-stem cells.

Many compounds are embryotoxic and exposure to them may interfere with stem cell and resulting embryo development and induce abnormal embryogenesis and malformations, if not pregnancy termination. As one example, pharmaceutical compounds administered to pregnant women can be embryotoxic. Further, compounds encountered within the environment, or cosmetics also can be harmful. Thus, there is a need to test compounds and other potential stressors for potentially negative effects on developing stem cells and embryos.

Currently-available toxicological stress tests typically adopt one of two approaches. The 1^{st} approach is to differentiate stem cells by removing growth factors that support potency to test stress effects during production of differentiated, functional cell lineages. A drawback of this approach is that single lineage tests may find toxicants for only that particular lineage. The second approach is to measure outright lethality and morbidity of stem cells in the presence of potency-maintaining growth factors. This approach, however, does not identify compounds or events that stress, but do not kill stem cells. That is, this approach does not identify lower, submorbid stress doses that lead to maladaptive runting and toxic responses to the embryo that do not necessarily kill stem cells.

Lara et al., Cell. Mol. Bioeng. (2013) 6: 335-345, discloses that the expression of Oct4 and Sox2 is increased, the expression of Nanog is decreased, and the expression of Rex1 did not change when shear stress is applied in the absence of LIF to murine embryonic stem cells (ESCs), that have previously been cultured in the presence of LIF.

Puschek et al., Fertil. Steril. (2013) 100 (Supplement): S456, discloses the cultivation of murine trophoblast stem cells (TSCs) in the presence of FGF-4. After the reduction of oxygen in the presence of FGF-4, the stress response is measured by determining the activities of AMPK and SAPK.

### SUMMARY OF DISCLOSURE

**The** invention is defined in the claims. Any subject-matter which is described herein, but which is not claimed, does not form part of the invention. The current disclosure provides S/M to detect stress in stem cells where the detected stressor might affect all stem cell lineages and might not kill the stem cells. Testing for stress before differentiation occurs is of great benefit because stress at this stage can affect all later-developing cell lineages. Thus, the S/M provide a unifying test.

**The** S/M also can detect stressors that act at sub-morbid runting doses that slow growth and negatively impact and imbalance differentiation of stem cells. Thus, the S/M provide for more sensitive stress detection that does not require cell death to observe stress. Imbalances of differentiation forced on cultured stem cells measure organismal responses not needed for adaptation to culture. Thus stress induced differentiation should reduce or replace the need for slower and more costly gestational animal *in vivo* reproductive toxicology tests.

**The** S/M are based on the finding that stressed stem cells (i) slow their growth and (ii) differentiate under physiological conditions that would normally maintain their toti- or pluri-potency. That is, they differentiate earlier than they normally would. Early differentiation of stem cells depletes the number of stem cells available to differentiate later in the development process, reducing the number of stem cells available to form organ systems that arise later during development. This type of early, stress-induced differentiation is referred to as "prioritized differentiation." Prioritized differentiation negatively affects development potential.

**The** disclosure provides numerous S/M to detect stress in stem cells by measuring potency factors (and markers) and prioritized differentiation factors (and markers). "Potency factors" can be thought of as "brake pedals" for differentiation. That is, their expression and activity is associated with toti- or pluri-potency. "Differentiation factors" can be thought of as "accelerator pedals" for differentiation. That is, their expression and activity is associated with normal differentiation that occurs when potency-maintaining extracellular growth factors are removed. Under stress conditions, however, potency factors can decrease and differentiation factors can increase even in the presence of potency-maintaining extracellular growth factors. This type of differentiation is referred to as "compensatory" or "prioritized" differentiation. That is, under compensatory or prioritized differentiation, stem cells can be induced by stress from a toti- or pluripotent state into a more differentiated/committed cell type despite the presence of growth factors that maintain potency. Stress also slows stem cell growth and induces differentiation in fewer-than-normal stem cells to further deplete stemness to "compensate" for fewer cells. Compensatory or prioritized differentiation of stem cells despite potency-maintaining growth factors also prioritizes differentiation to emphasize creating sufficient 1^{st} lineage differentiation at the sacrifice of later lineages. Decrease in nuclear potency factors and an increase in 1^{st} lineage determining nuclear differentiation factors provide (early lineage>later lineage) "markers" that are evidence that differentiation is enabled, but their activity does not necessarily lead to or cause the potent or differentiated state. The differentiated state requires sufficient loss of nuclear potency factors and/or gain of nuclear differentiation nuclear factors. But, different stressors may more strongly release the brake pedal (decrease potency) or engage the accelerator pedal.

Potency factors and/or markers include: Oct4; Sox2; Nanog; Rex1; TEAD4 (TEA domain family member 4); Errβ (estrogen receptor-related-β); Cdx2 (caudal homeobox domain); Id2; Klf5 (Krueppel-like factor 5); PDCD2 (programmed cell death 2); CARM1(coactivator-associated-arginine methyltransferase); Sall4 (Spalt-like transcription factor); SSEA1; SSEA3; SSEA4; TRA-1-60; TRA-1-81; and Elf5 (E74-like factor 5 (ets domain transcription factor).

Prioritized differentiation factors and/or markers include: Lrp2; Dab2; Fgf5 (fibroblast growth factor 5); Pdgfra (Platelet-derived growth factor receptor A); Sox17 (SRY box containing gene 17); Gata4/6; PAX8 (Paired box 8); NEFL (Neurofilament protein light chain); TSHR (thyroid stimulating hormone receptor); Brachyury; Laminin; AFP (a-feto-protein); Nestin; Goosecoid; MEK1 (mitogen-activated protein kinase kinase 1); MEK2; AKT(RAC-alpha serine/threonine-protein kinase/protein kinase B); PL1 (Placental lactogen-1); Plf (proliferin); PL2 (placental lactogen-2; Hand1; Stra13; Tpbpa; Mash2 (mouse achaete scute 2); Gcm1 (glial cells missing 1); Tfeb; Ctsq (Cathepsin Q); AMPK; SAPK/JNK (stress-activated protein/jun Kinase); Cubulin; TBP (TATA binding protein); Vimentin; Snail; GSK3; and JAK/STAT.

The S/M can be used to assess compounds and other environmental conditions for stress effects on stem cells. The S/M can be used to screen compounds and other environmental conditions for stress effects on stem cells before the compounds or other environmental conditions are developed, commercialized or released into the workplace or atmosphere. The S/M of the invention include assays and high throughput screens (HTS).

### BRIEF DESCRIPTION OF THE FIGURES

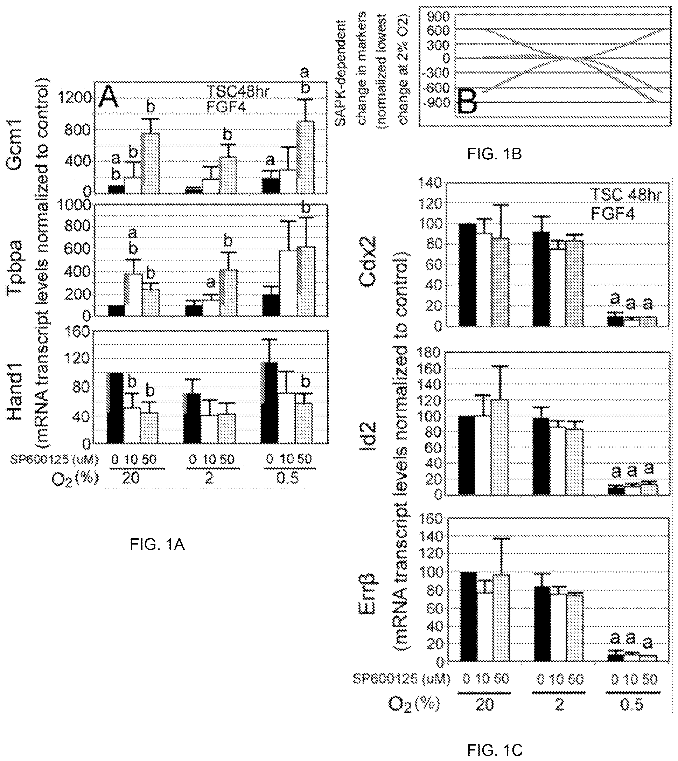

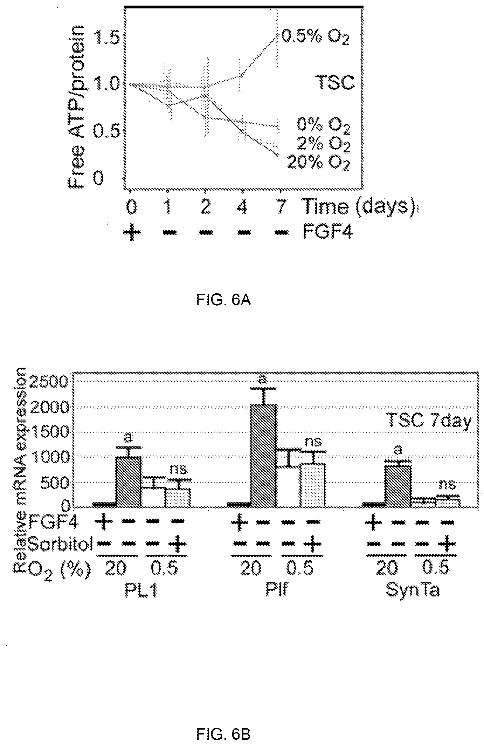

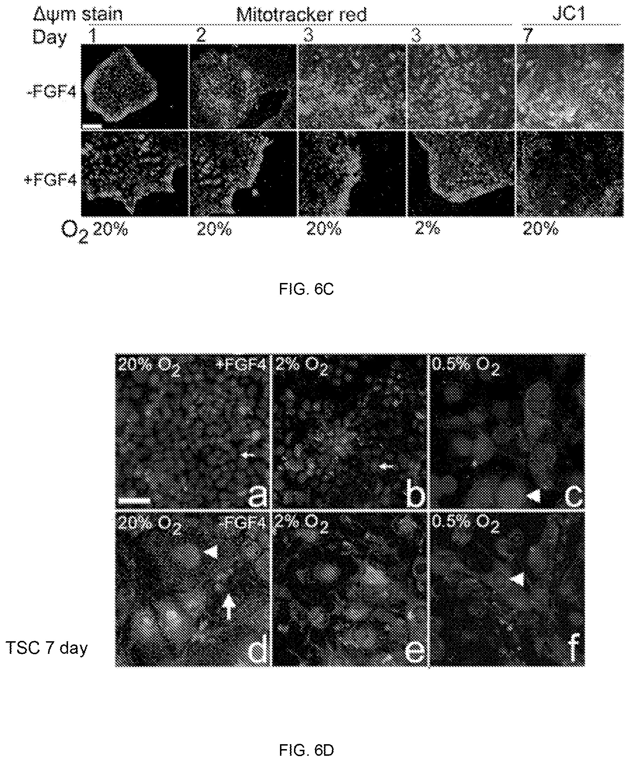

**FIGs.** 1A-1C. SAPK mediates decreased Gcm1 and Tpbpa and increased Hand1 at all O₂ levels. There are greater effects as O₂ deviates from the stemness optimum of 2% O₂. Trophoblast stem cells (TSCs) were passaged and cultured at 20, 2, or 0.5% O₂ overnight. FGF4 was replenished the next day at time zero, and SAPK inhibitor SP600125 (10 µm or 50 µM) was co-incubated with TSCs for 2 days at the indicated O₂ levels ± SP600125. Markers were quantitated using qPCR. (FIG. 1B) is a graph derived from data in (FIG. 1A), where the highest inhibited group was subtracted from the uninhibited group at each O₂ level (the subtracted difference is shown as a color inset in the histogram bars of highest expression of each marker). The difference at 2% O₂ was set to a baseline of "0" as it was previously shown that 2% O₂ was the optimal level that produces the least stress (activated SAPK) and highest proliferation (Zhou (*et als.,* omitted from citations), 32:475, 2011 (Zhou, 2011)). In (FIG. 1C) Cdx2, Id2, and Errβ decreased at 0.5% 02 in the presence of FGF4, but SAPK inhibitor had no effect at any O₂ level.

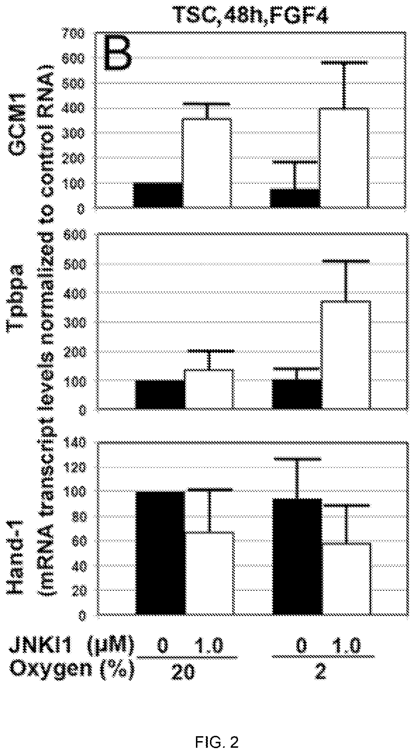

**FIG.** 2. SAPK mediates suppression of Gcm1 and TPBPa, and induces Hand1 at all O₂ levels, but there are greater effects for these lineage choices at 0.5% O₂ and 20% O₂ and the least effect is seen at 2% O₂ where it has been reported that SAPK activity is lowest. TSCs were grown at 20% O₂, then passed and switched to 20, 2, or 0.5% O₂ overnight. FGF4 was replenished the next day at time zero, JNKI1 (1 µM) was preincubated with TSC 3 hr prior to time zero, then cells were cultured for 2 days at the indicated O₂ levels with or without the two SAPK inhibitors. RNA was isolated and quantitated using qPCR.

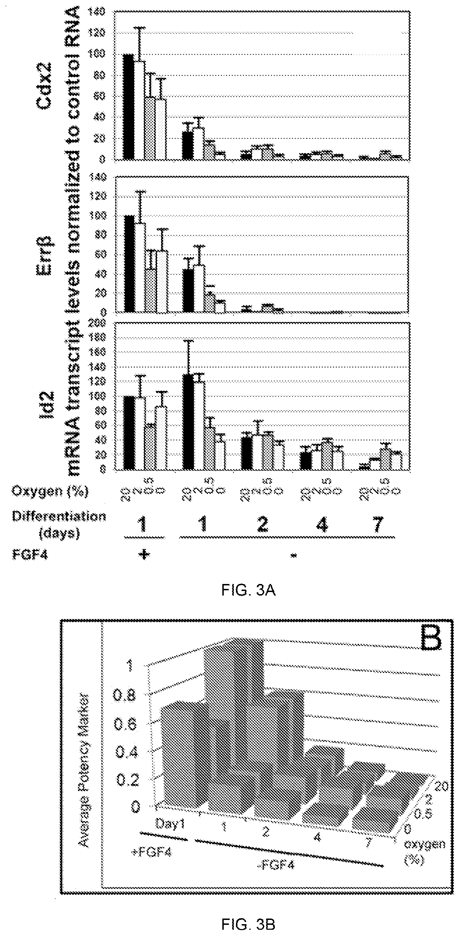

**FIG.** 3A and FIG. 3B. Nuclear differentiation factors have complex time- and O₂ level-dependent regulation over 7 days of FGF4 removal and TSC differentiation. TSCs were grown at 20%, then passed and switched to 20, 2, 0.5, or 0% O₂ for one day and RNA was isolated to emulate previous studies. FGF4 was removed after one day, then cells were cultured for 1, 2, 4, or 7 days, and RNA was isolated and quantitated using real time TaqMan qPCR. (FIG. 3A) shows histograms for loss of mRNA for four nuclear differentiation markers, Gcm1, Tfeb, Hand1, and Mash2, and one nonnuclear marker Tpbpa (n = 3 biological replicates, error bars show standard error of the mean). (FIG. 3B) shows a three dimensional histogram of time in the x axis, O₂ levels in the y axis, and average levels of the four nuclear differentiation factors and one nonnuclear lineage marker in the z axis.

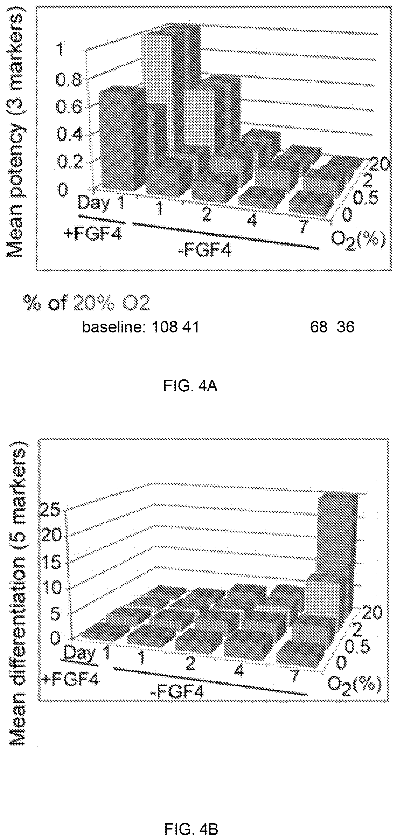

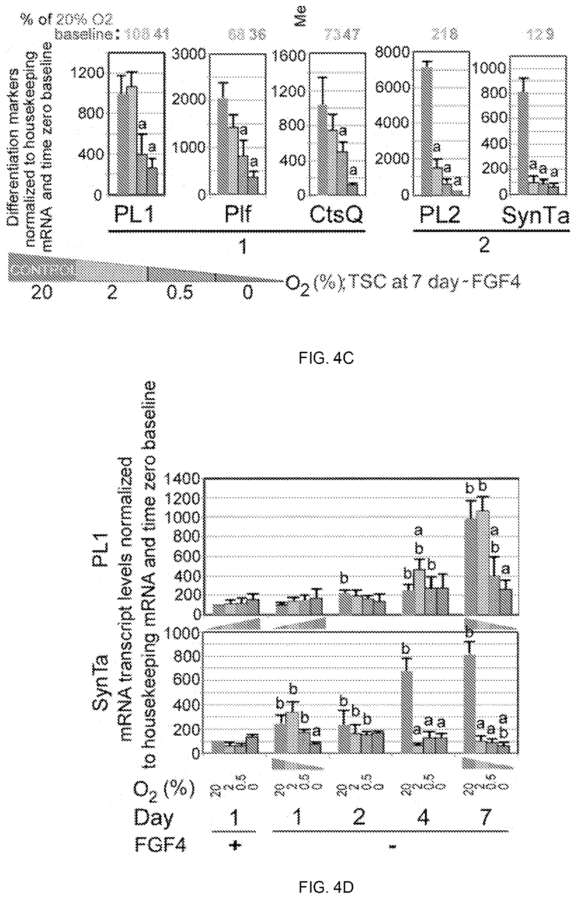

**FIGs.** 4A-4D. Multipotency factors undergo rapid loss on day 1 at 0.5% O₂ with FGF4 present, but the least loss after 7 days of FGF4 absence. Terminal differentiation is lowest at 0.5% O₂ and highest at 20% O₂ after 7 days, and labyrinthine placental lineages are dependent on 20% O₂. (FIG. 4A) shows averages of loss of a mean of three potency factors, (FIG. 4B) the gain of a mean of five differentiation factors during seven days of differentiation caused by removal of FGF4 dependent on four O₂ levels, and (FIG. 4C) shows the five differentiation factors averaged in (B), but only at highest differentiation at day 7. There are three types of O₂-dependence from left to right; (1) PL1 has no difference between 20 and 2, but decreases at hypoxic 0.5% O₂ to 41% O₂. (2) Plf and Ctsq marker loss increased at each O₂ level; 2-0.5-0% O₂. (3) Two markers, PL2 and SynTa have the greatest O₂-dependence, decreasing greatly to 21 and 12% at 2% O₂ and 8 and 9% at 0.5% O₂, respectively. (FIG. 4D) shows the oxygen-dependent accumulation of mRNA of a marker of the 1^{st} lineage (PL1) and a later lineage (synTA). Note that synTA always requires highest O₂ but PL1 is induced by hypoxic stress early but requires highest O₂ levels at day 7.

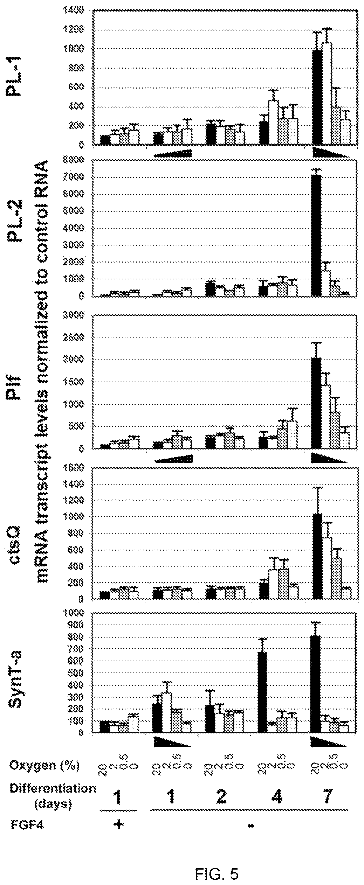

FIG. 5. Although greatest multipotency factor loss and differentiation factor gain initiate at 0.5% O₂ with FGF4 for 24 h, terminal differentiation is lowest at 0.5% O₂ and highest at 20% O₂ after 7 days when multipotency is lowest.
**TSCs** were grown at 20% O₂, then passed and switched to 20, 2, or 0.5 O₂ overnight. RNA was isolated and five terminal differentiation marker mRNAs were quantitated using real time TaqMan qPCR. The filled in triangles below PL1, Plf and SynTa show tendencies of all three to be dependent on high O₂ at 7 days, but early lineage markers PI1 and Plf are induced by hypoxia at day 1, but not SynTa
**FIGs.** 6A-6D. (FIG. 6A) TSCs that differentiated without FGF4 for 7 days had the highest ATP levels at 0.5% O₂ and the lowest ATP in the rank order 20%<2%<0% compared with baseline stem cells at 20% O₂ + FGF4. TSCs were cultured in 0-20% O₂ for 0-7 days ± FGF4 as indicated, rapidly frozen, and assayed for free ATP (n=4). (FIG. 6B) At 7 days, FGF4 absence increased terminal differentiation markers 8-20 fold at 20% O₂, but lower differentiation levels at 0.5% O₂ were not increased by a sorbitol dose, which induces high levels of differentiation at 20% O₂. TSCs were cultured for 7 days ± FGF4 at 20% or 0.5% O₂ ± 100 mM sorbitol. mRNA was purified and reverse transcribed, then analyzed for relative amounts of 3 terminal differentiation markers that were normalized to housekeeping mRNAs. Triplicate experiments are shown, with mean ± s.e.m. (error bars). Sorbitol does not significantly increase differentiation markers at 0.5% O₂ (FIG. 6C) Mitochondrial membrane charge increased between 2-3 after FGF4 removal. (FIG. 6D) Correspondingly, at 7 days, mitochondrial Δ_{Ψm} increased only at O₂≥2% with FGF4 removal. TSCs were cultured for 7 days at 20% O₂ (a,d), 2% O₂ (b,e), or 0.5% O₂ (c,f) with FGF4 (a-c) or without FGF4 (d-f) and stained with JC1. Micron bar in micrograph (a) indicates 50µM. Green fluorescence indicates monomeric stain and low membrane charge and red fluorescence indicates polymeric stain and high charge. For original color fluorescence figures see (Xie, Stem Cell Research, 13:478, 2014 (Xie, 2014)).

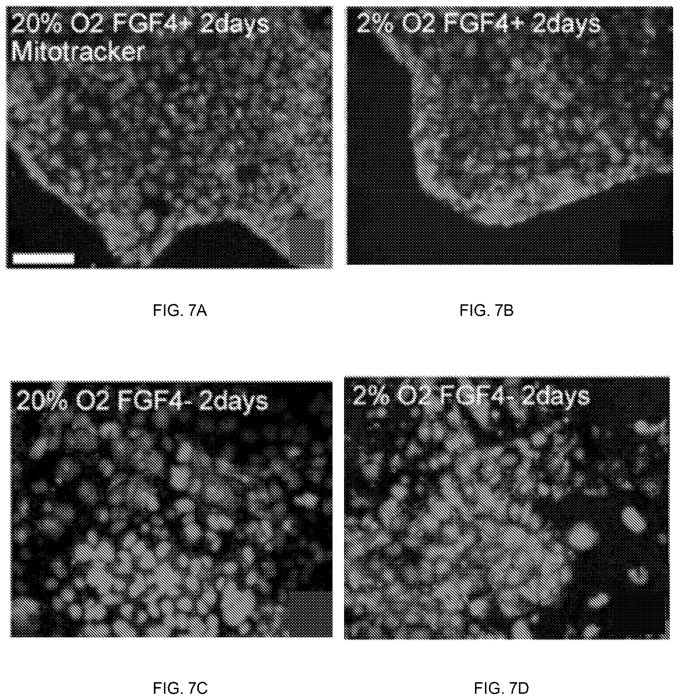

**FIGs.** 7A-7D. FGF4 maintains inactive mitochondria at 20%, 2%, or 0.5% O₂ (FIG. 7A) and mitochondria display higher mitochondrial membrane potential levels (ΔΨₘ) at 20% > 2% but remain depolarized at 0.5% O₂ after FGF4 removal. TSCs were cultured for 2 days at 20% (FIG. 7A, FIG. 7C) or 2% O₂ (FIG. 7B, FIG. 7D) with FGF4 (FIG. 7A, FIG. 7B) or without FGF4 (FIG. 7C, FIG. 7D) and then stained for ΔΨₘ using the MitoTracker dye (A). Micron bars in micrograph A indicate 50 µM.

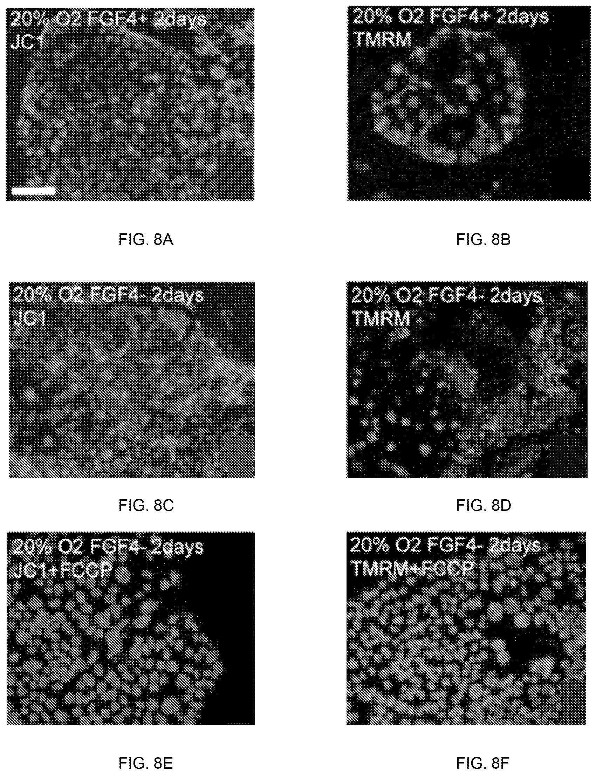

**FIGs.** 8A-8F. To confirm mitochondrial inactivity, TSCs were cultured for 2 days at 2% O₂ (FIG. 8A, FIG. 8C, FIG. 8E) or 20% O₂ (FIG. 8B, FIG. 8D, FIG. 8F) with FGF4 (FIG. 8A, FIG. 8B) or without FGF4 (FIG. 8C-FIG. 8F) and then stained with the probe JC1 or TMRM as indicated, in the presence or the absence of uncoupler FCCP as a control. Micron bars in micrograph A indicate 50 µM.

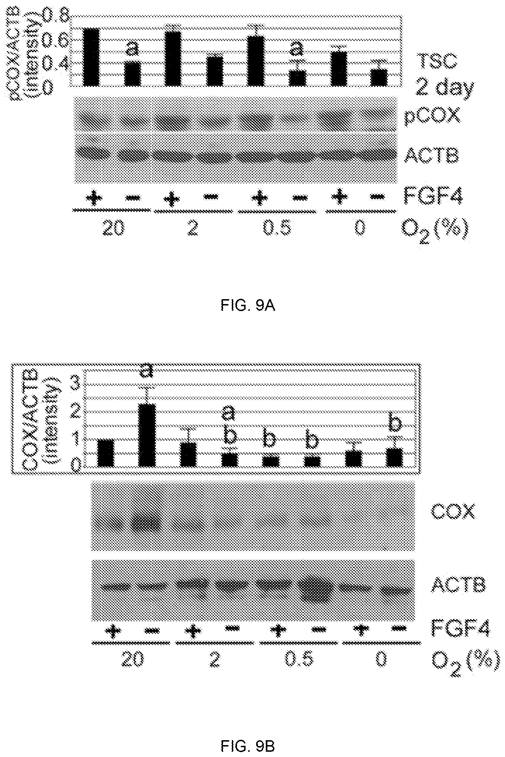

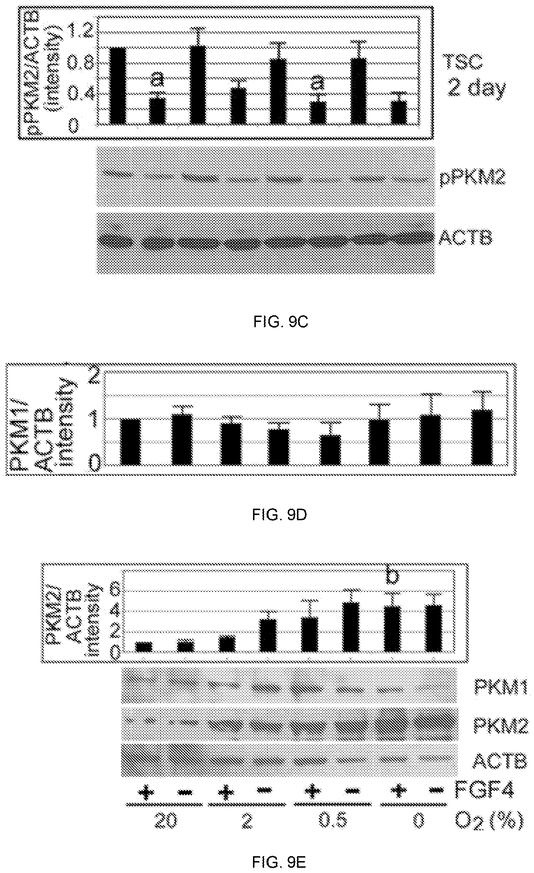

**FIGs.** 9A-9E. FGF4 maintained COX subunit I and PKM2 in the phosphorylated state at all O₂ levels, but total COX and PKM2 levels are regulated by hypoxia and did not change with FGF4 removal. TSCs were cultured for 2 days ± FGF4 at 0-20% O₂ and assayed by immunoblot for phosphorylated cytochrome c oxidase (pCOX) using a phospho-specific antibody (FIG. 9A), total COX (FIG. 9B), actin b (ACTB) and, pPKM2 (FIG. 9C), PKM1 (FIG. 9D), or PKM2 (FIG. 9E). Histograms show the pCOX, total COX levels, PKM1, PKM2, and pPKM2 normalized to ACTB, with bars showing the average of 3 experiments ± s.e.m.

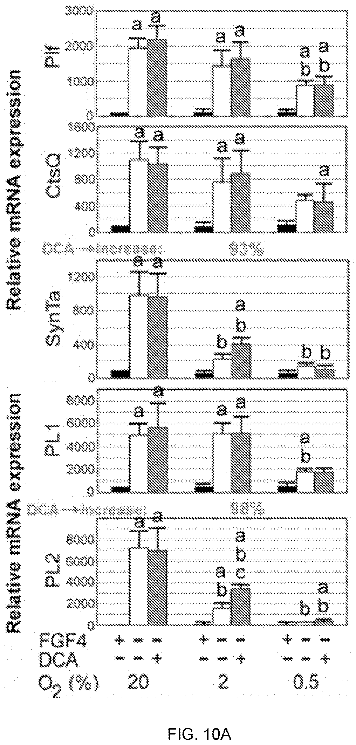

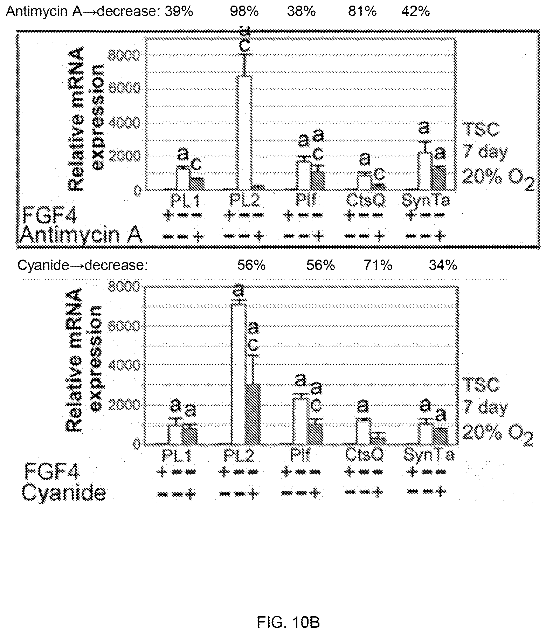

**FIGs.** 10A and 10B. In TSCs cultured for 7 days at 20% O₂, all terminal differentiated lineages were O₂ sensitive, but PL2 and CtsQ were most inhibited by the mitochondrial inhibitors cyanide and antimycin A, and PL2 was most highly induced by the mitochondrial activator DCA at 2% O₂. (FIG. 10A) TSCs were incubated for 7 days ± FGF4 at 20%, 2%, or 0.5% O₂ with the mitochondrial activator DCA. TSCs were cultured for 7 days at 20% O₂, ± FGF4, and with mitochondrial inhibitors antimycin A or cyanide (FIG. 10B). The cells were then lysed and assayed by qPCR for the relative levels of 5 terminal differentiation markers PL1, PL2, Plf, CtsQ, and synTa. The experiment was repeated 3 times and bars show the average ± s.e.m., (a) indicates no significant difference and (b) indicates a significant difference (p<0.05) between TSCs with FGF4 removed without or with mitochondrial agonist or antagonist. Where changes in markers are significant (b), the increase or decrease due to mitochondrial agonist or antagonist is shown above the histogram bar.

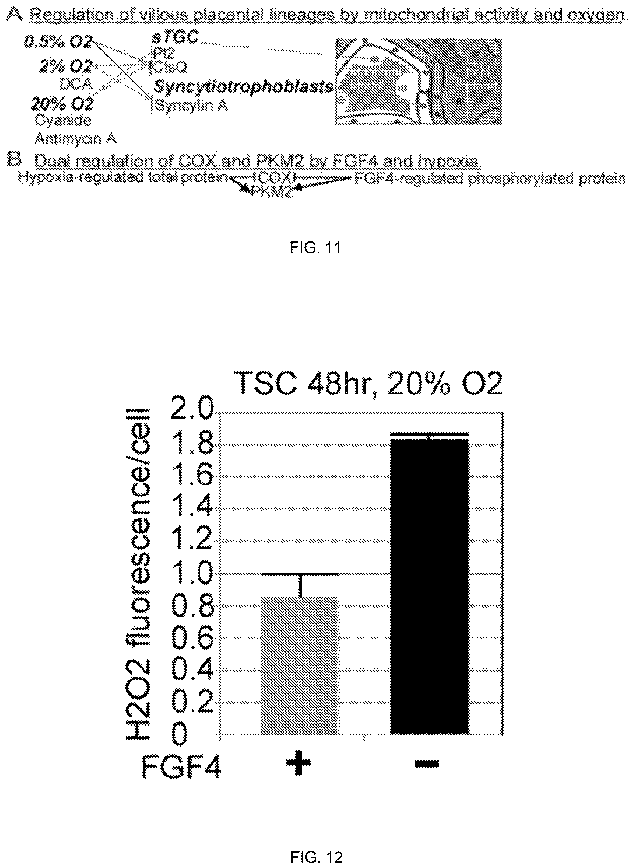

FIG. 11. Diagram showing that hypoxia, mitochondrial agonists, and antagonists affect 3 markers of 2 differentiated lineages at the surface of the labyrinthine placenta. COX and PKM2 total protein levels are regulated by hypoxia and fine-tuned by phosphorylation induced by FGF4.
FIG. 12. TSCs produce twice as much ROS (H₂O₂) 48 hr after FGF4 removal.

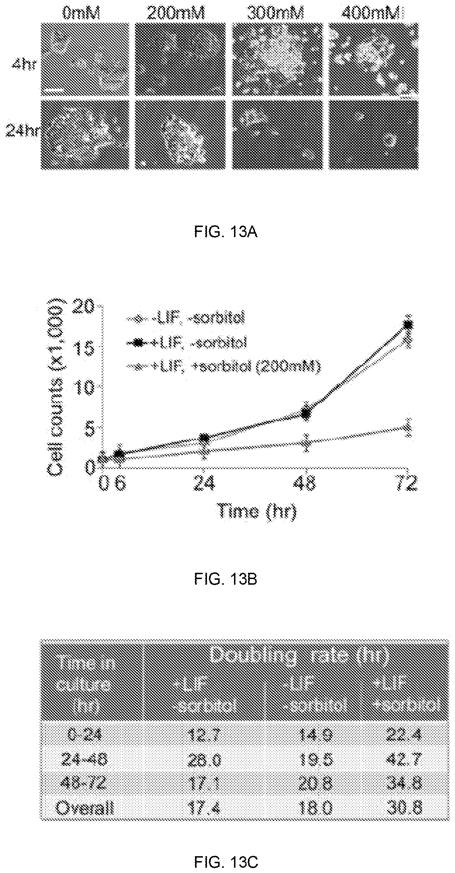

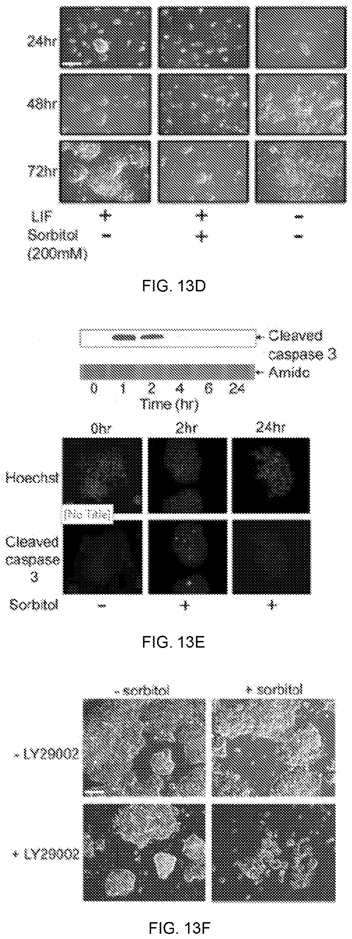

FIGs. 13A-13F. Hyperosmotic stress effects on cell proliferation in murine embryonic stem cells (mESCs). (FIG. 13A) mESCs were cultured in the presence of 0-400 mM sorbitol for either 4 or 24 hr and transmitted light micrographs were taken. (FIG. 13B) mESCs were cultured ± leukemia inhibitory factor (LIF) and ± 200 mM sorbitol for 0 - 72 hr. mESCs were trypsinized and counted with a hemocytometer following trypan blue exclusion. Error flags represent standard error of the mean, n ≥ 3. (FIG. 13C) Doubling rates were calculated and tabulated from cell counts in (FIG. 13B). (FIG. 13D) mESCs were incubated in the presence of LIF ± 200 mM sorbitol for 24, 48, or 72 hr and transmitted light micrographs were taken. (FIG. 13E) mESCs were cultured in the presence of 200 mM sorbitol for 0-24 hr. In some wells, cells were lysed, and proteins were fractionated using sodium dodecyl sulfate-polyacrylamide gel electrophoresis (SDS-PAGE), blotted, and probed for the presence of the caspase 3 cleavage product (n = 3). Time-matched cells were fixed, and stained for the cleavage product of caspase 3 with Hoechst staining of the nuclei. (FIG. 13F) mESCs were cultured for 24 hr ± sorbitol (200 mM), ± phosphoinositide 3-kinase (PI3K) inhibitor, and LY294002 (25 µM); transmitted light micrographs were taken. All micrographs were taken at 100XScale bar = 50 mm.

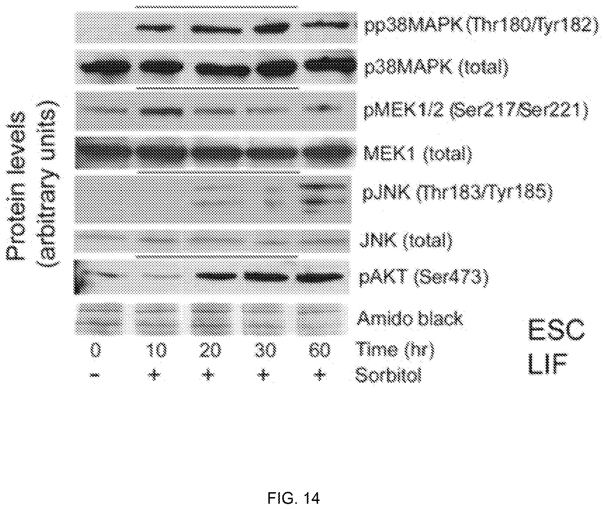

FIG. 14. Activation of p38, MEK1/2, c-Jun N-terminal kinase (JNK), and AKT by hyperosmolarity. mESCs were incubated in 200 mM sorbitol for 0-60 min to detect kinetics of enzyme activation. Cells were lysed and proteins were fractionated using SDS-PAGE, blotted, and probed for phosphop38 (Thr180/Tyr182), phospho-JNK (Thr183/Tyr185), phospho-MEK1/2 (Ser217/221), or phospho-AKT (Ser473). Blots are representative of triplicate experiments. Phospho-proteins and their loading controls are grouped between black lines.

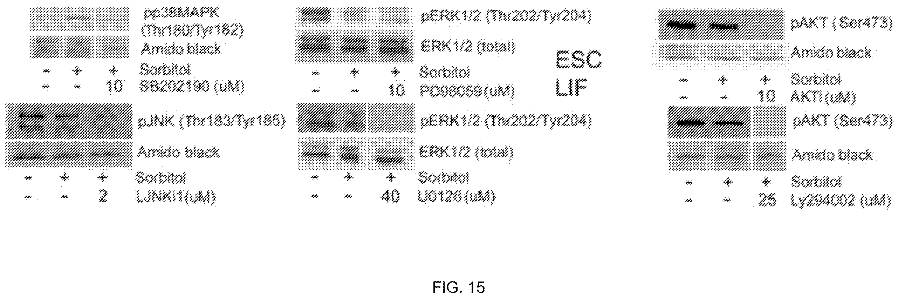

FIG. 15. Efficacy of enzyme inhibitors during hyperosmotic stress. mESCs were incubated for 4 hr in the presence of 200 mM sorbitol with or without one of the enzyme inhibitors SB202190 (p38), PD98059 (MEK1), U0126 (MEK1/2), LJNKi-1 (JNK), AKTi (AKT), or LY294002 (PI3K). Cells were lysed and proteins were fractionated using SDS-PAGE, blotted, and probed for phospho-p38 (Thr180/Tyr182), phospho-JNK (Thr183/Tyr185), phospho-ERK1/2 (Thr202/Tyr204), or phospho-AKT (Ser473). Blots are representative of triplicate experiments.

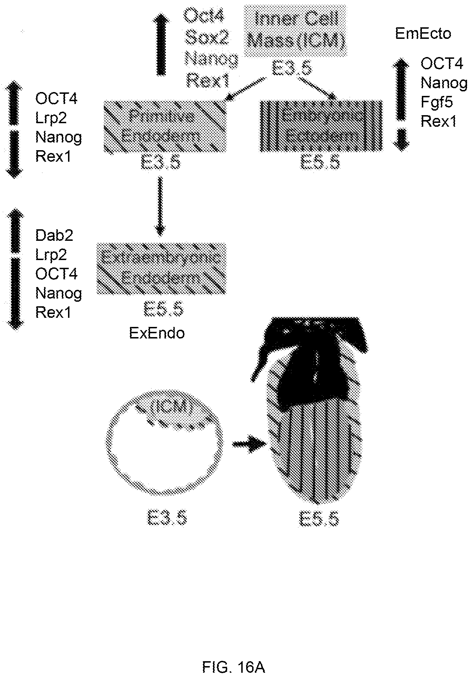

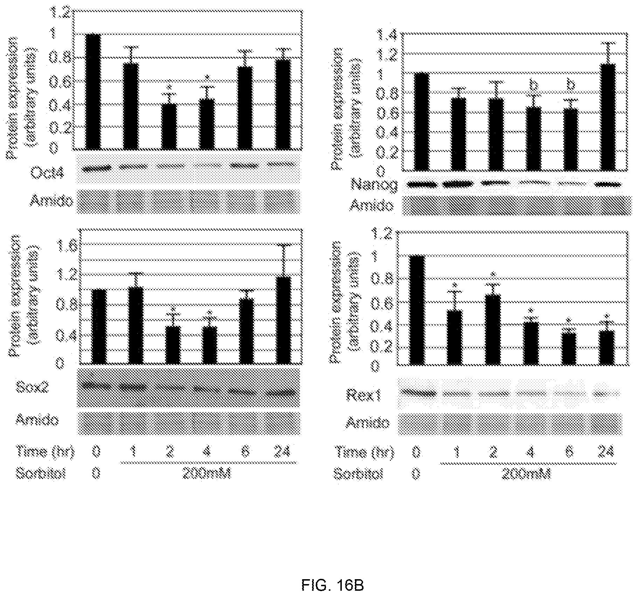

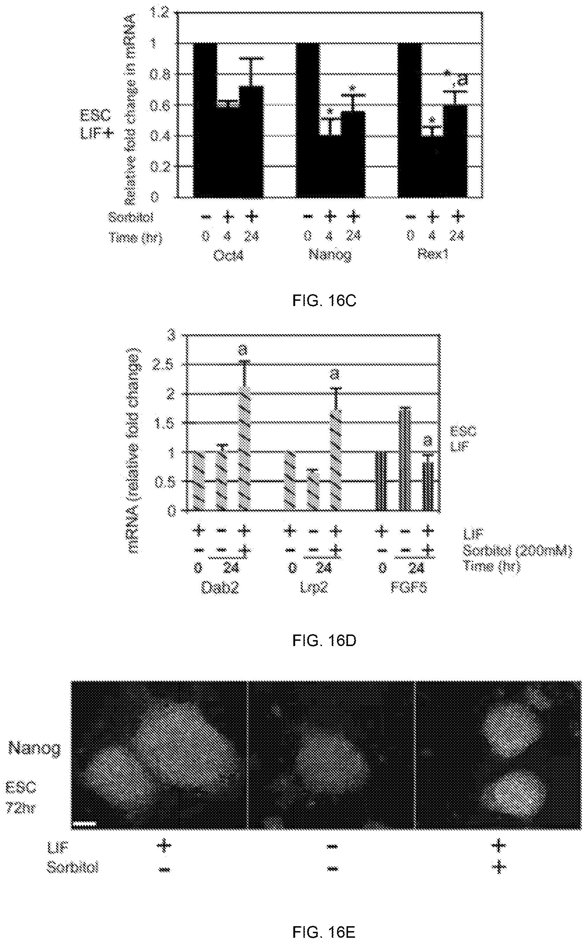

FIGs. 16A-16E. Hyperosmotic stress in mESCs impacts transcription factor expression at protein and mRNA levels. (FIG. 16A) The inner cell mass (ICM) of the blastocyst coexpresses Oct4/Rex1/Nanog in pluripotent cells at E3.5. After E3.5 a Rex1 subpopulation delaminates from the ICM and expresses primitive endoderm (PrEndo) marker, Lrp2, at E3.5 and extraembryonic endoderm (ExEndo) marker, Dab2, at E4.5. At E4.75, Rex1 decreases in the remaining cells of the ICM and, by E5.5, fibroblast growth factor (FGF)5 is expressed in embryonic ectoderm (EmEcto). Upward arrow indicates that expression is maintained or increasing; downward arrow means that expression is decreasing or absent. (FIG. 16B) mESCs were incubated in 200 mM sorbitol for 0-24 hr and lysed. Proteins were fractionated using SDS-PAGE, blotted, and probed for Oct4, Sox2, Nanog, or Rex1. Histograms show relative expression of each protein when normalized to amido black. (FIG. 16C) Total RNA was isolated and subjected to reverse transcription to form complementary DNA (cDNA). Real-time quantitative PCR (RTqPCR) was performed with Oct4, Nanog, and Rex1 primers. Histograms represent relative fold change in mRNA expression using the ddCt method. (FIG. 16D) mESCs in monolayer were incubated ± sorbitol and ± LIF for 24 hr. Dab2, Lrp2, and Fgf5 mRNA transcript levels were examined by RT-qPCR. Histogram shows the relative fold changes when compared with time zero, no stress. Error flags are the standard error of the mean (n = 3). "a" indicates significant difference for sorbitol +LIF compared with LIF- at 24 hr. (P < 0.05, ANOVA and Student-Newman-Keuls post hoc tests). (FIG. 16E) mESCs were treated with sorbitol for 72 h, fixed, and probed for Nanog. Micrographs taken at 40 X Scale bar = 50 µm. Error bars represent standard error of the mean (n = 3); "*" denotes a significant difference from the 0 hr untreated control; "a" denotes significant difference when compared with 4 hr + sorbitol time point. "b" Indicates that the expression nadir (variable in Nanog) was significantly different from the unstressed mESCs at time zero (4 and 6 hr were chosen to represent the nadir in this histogram). ANOVA+ Student-Newman-Keuls post hoc tests, P < 0.05. In (FIG. 16A) and (FIG. 16D) diagonal lines indicate primitive endoderm starting at E3.5 through extraembryonic endoderm at E5.5 and vertical lines indicate primitive ectoderm starting at E5.5.

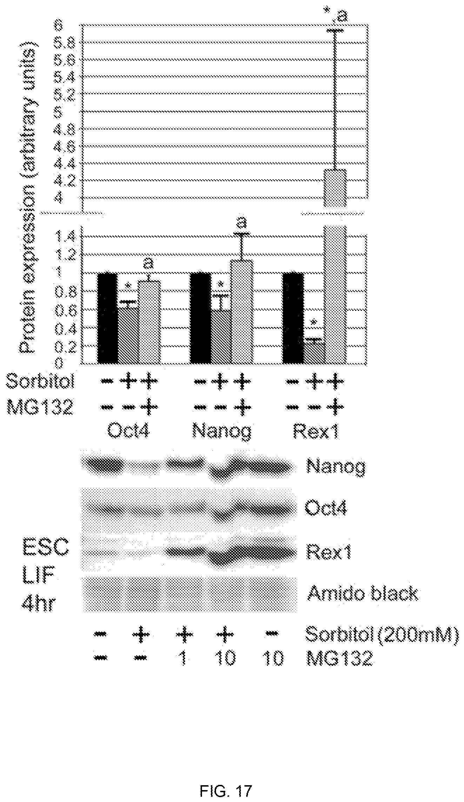

FIG. 17. MG132 proteasome inhibitor effects on Oct4, Nanog, and Rex1 during sorbitol stimulation of mESCs. mESCs were treated for 4 hr with 0 or 200 mM sorbitol ± 10 µM MG132 and then lysed. Total cellular protein was fractionated using SDS-PAGE, blotted, and probed for Oct4, Nanog, or Rex1. Error bars represent standard error of the mean (n = 3); "*" denotes a significant difference from the untreated control; "a" denotes significant difference when compared with 4 hr + sorbitol time point. ANOVA and Student-Newman-Keuls post hoc tests: Oct4 P < 0.002; Nanog P < 0.005; and Rex1 P < 0.001.

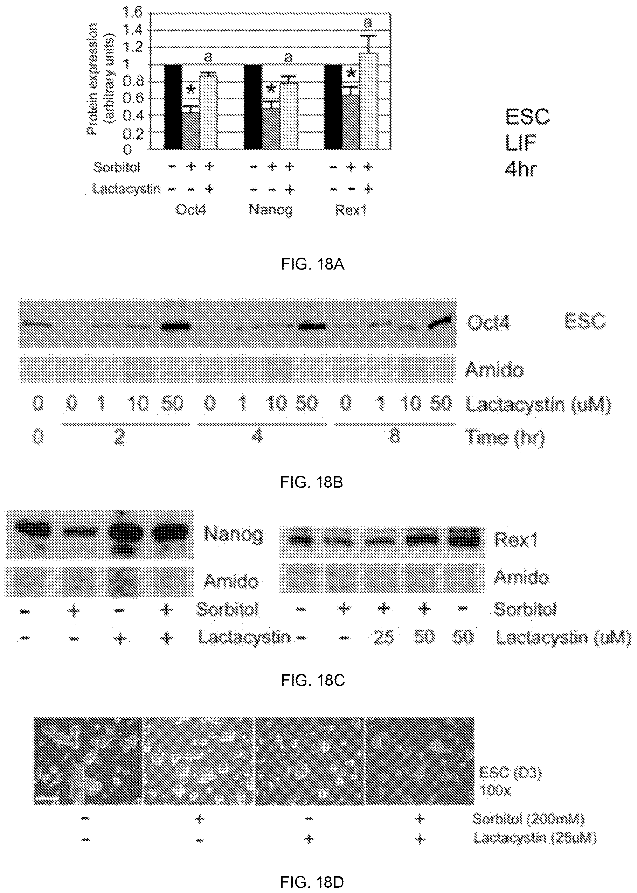

FIGs. 18A-18D. Lactacystin effects on Oct4, Nanog, Rex1 during sorbitol stimulation of mESCs. mESCs were treated for 4 hr ± 200 mM sorbitol ± 25, 50µM lactacystin, then photographed before lysing. FIG. 18A, FIG. 18B, FIG. 18C)Total cellular protein was fractionated using SDS-PAGE, blotted, and probed for Oct4, Nanog, or Rex1. Error bars represent standard error of the mean (n=3); '*' denotes a significant difference from the untreated control; 'a' denotes significant difference when compared to 4 hr+sorbitol timepoint. ANOVA + Student-Newman-Keuls post hoc tests, Oct4 p=0.000; Nanog p=0.035; Rex1 p=0.11. FIG. 18D) mESCs were stressed with or without lactacystin and micrographs were taken at 100x. Scale bar = 50µm.

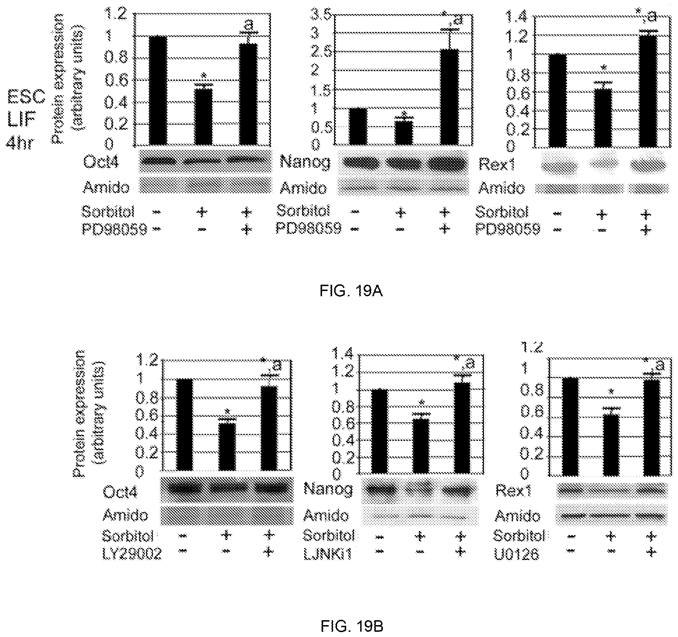

FIGs. 19A and 19B. Enzymes activated during 4 hr of hyperosmotic stress initiate the differentiation program in mESCs. mESCs were incubated in 200 mM sorbitol for 0-4 hr with or without the presence of (FIG. 19A) PD98059 (10 µM), or (FIG. 19B) LY294002 (25 µM), L-JNKi-1 (2 µM), or U0126 (40 µM). mESCs were lysed, and proteins were fractionated using SDS-PAGE, blotted, and probed for Oct4, Nanog, or Rex1. Histograms show relative expression of each protein when normalized to amido black expression. Error flags represent standard error of the mean (n ≥ 3). ' '*' ' denotes significant difference from unstressed mESCs. "a" indicates significant difference from stress-only time point. ANOVA and Student-Newman-Keuls post hoc tests (P<0.05).

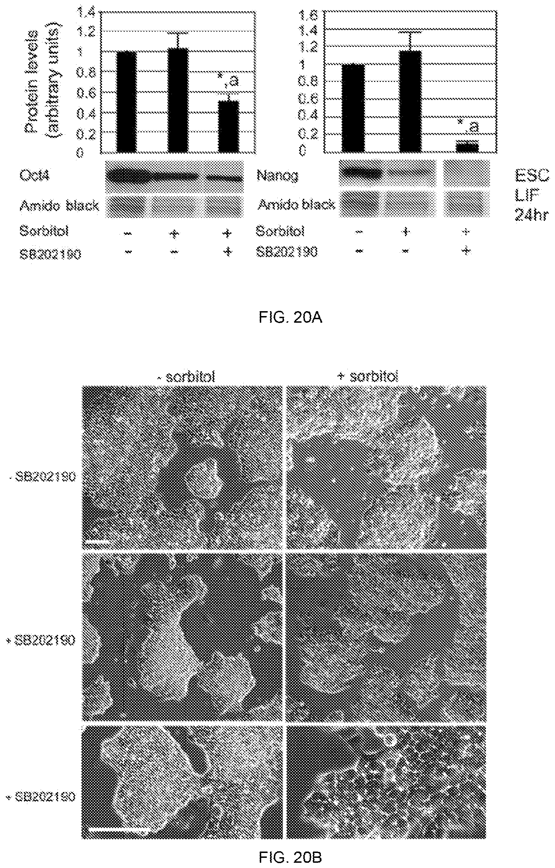

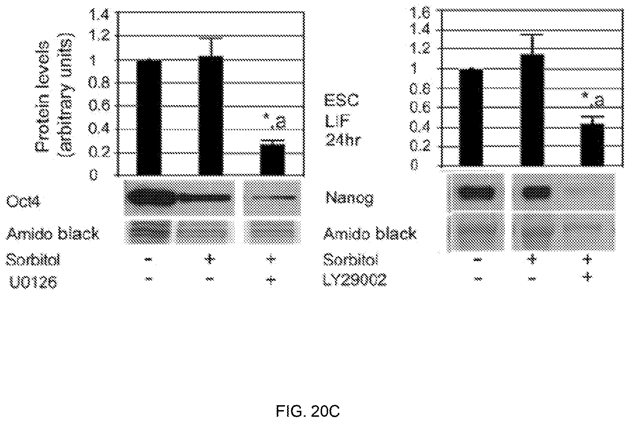

FIGs. 20A-20C. p38MAPK rescues pluripotency of mESCs during 24 hr of hyperosmotic stress. (FIG. 20A) mESCs were incubated ± 200 mM sorbitol for 0-24 hr and ± p38 inhibitor SB202190 (10 µM). mESCs were lysed, and proteins were fractionated using SDS-PAGE, blotted, and probed for Oct4 or Nanog. (FIG. 20B) Phase micrographs of mESCs following 24 hr of culture in the presence/absence of stress and p38MAPK inhibitor, SB202190. Scale bars = 50 µm. (FIG. 20C) mESCs were incubated ± 200 mM sorbitol for 0-24 hr ± either U0126 or LY294002 (25 µM). mESCs were lysed and proteins were fractionated using SDS-PAGE, blotted, and probed for Oct4 or Nanog. Histograms show relative expression of each protein when normalized to amido black expression. Error flags represent standard error of the mean (n ≥ 3). ' '*' ' denotes significant difference from unstressed mESCs. ' 'a' ' indicates significant difference from stress-only time point. ANOVA and Student-Newman-Keuls post hoc tests (P < 0.05).

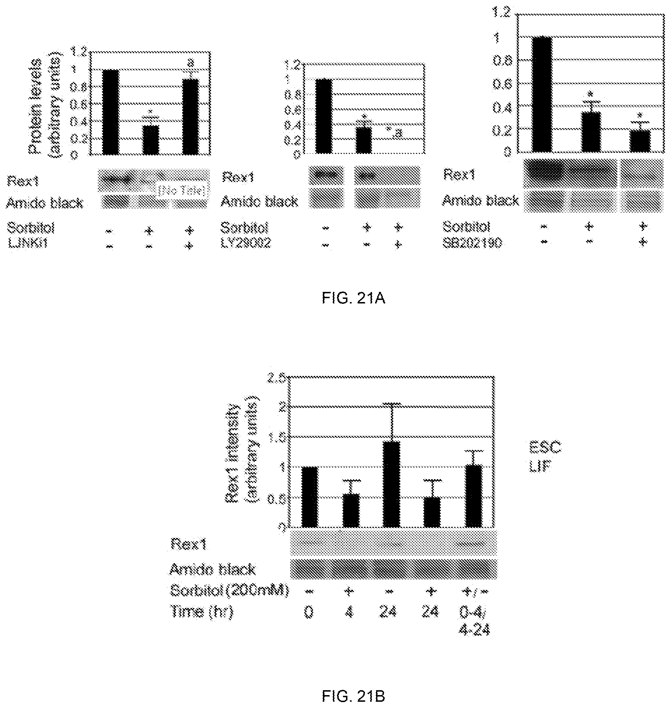

FIGs. 21A and 21B. JNK-dependent signaling suppressed Rex1 expression during 24 hr of hyperosmotic stress. (FIG. 21A) mESCs were incubated ± 200 mM sorbitol for 0-24 hr ± (A) p38MAPK inhibitor SB202190 (10 µM) or PI3K inhibitor LY294002 (25 µM); or JNK inhibitor L-JNKi-1 (2 µM). mESCs were lysed, and proteins were fractionated using SDS-PAGE, blotted, and probed for Rex1. Histograms show relative expression of each protein when normalized to amido black expression. Error flags represent standard error of the mean (n ≥ 3). ' '*' ' denotes significant difference from unstressed mESCs. ' 'a' ' indicates significant difference from stress-only time point. ANOVA and Student-Newman-Keuls post hoc tests (P < 0.05). (FIG. 21B) To test the reversibility of the Rex1 loss during sorbitol stress, mESCs were incubated - 200 mM sorbitol for 0-4 hr and then sorbitol was removed from some dishes for the remaining 20 hr (lane 5). Histogram is representative of two independent experiments.

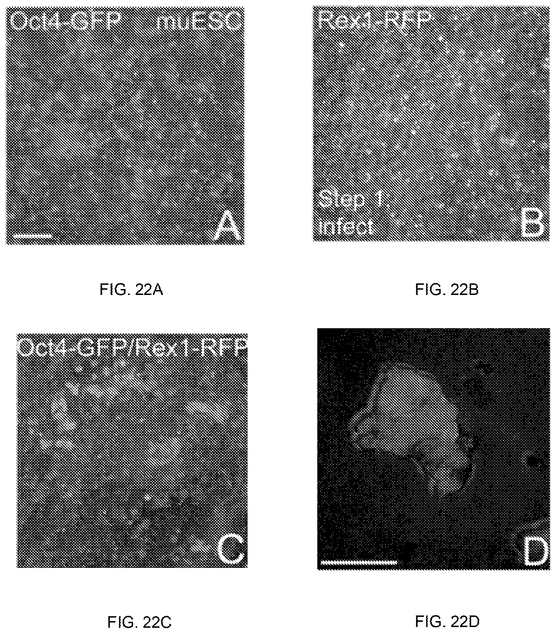

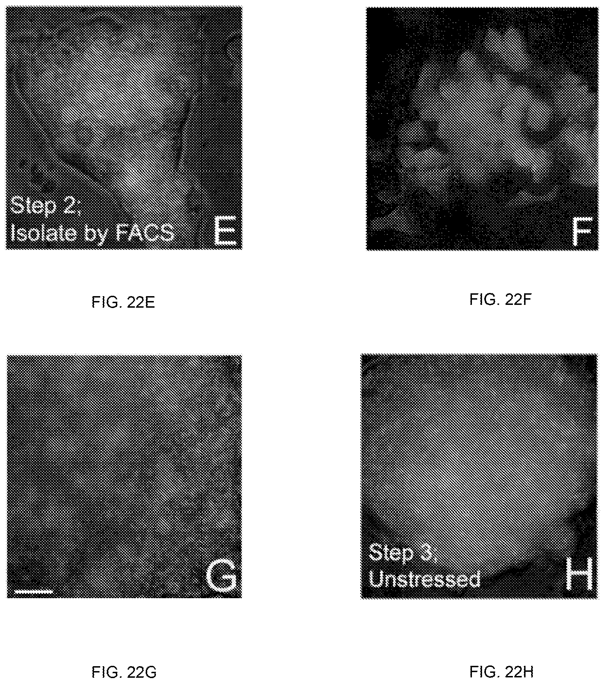

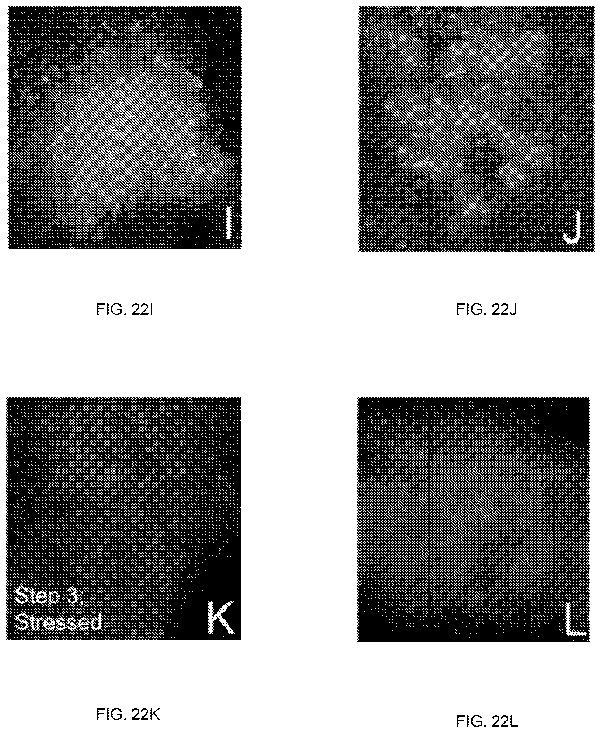

FIGs. 22A-22L. Viable pluripotency activity reporters respond to stress treatment as previously reported for endogenous pluripotency reporter protein levels. ESCs were lentivirus-infected (Step 1; FIG. 22A, FIG. 22B, FIG. 22C), FACS-selected (Step 2; FIG. 22D, FIG. 22E, FIG. 22F), and stress-tested (Step 3) without (FIG. 22G, FIG. 22H, FIG. 22I) or with 200mM sorbitol for three days (FIG. 22J, FIG. 22K, FIG. 22L) using pluripotency reporter Oct4-GFP ESCs (FIG. 22A, FIG. 22D, FIG. 22G, FIG. 22J), Rex1-RFP ESCs (FIG. 22B, FIG. 22E, FIG. 22H, FIG. 22K), or ESCs with both reporters (FIG. 22C, FIG. 22F, FIG. 22I, FIG. 22L). ESCs were imaged by epifluorescence microscopy and transmitted light (phase contrast) with fluorescence superimposed. Micron bars in FIG. 22A, FIG. 22D, and FIG. 22G indicate 100µm as FIG. 22A-C, FIG. 22G-I and FIG. 22J-L were micrographed at 10X and FIG. 22D-F were micrographed at 20X.

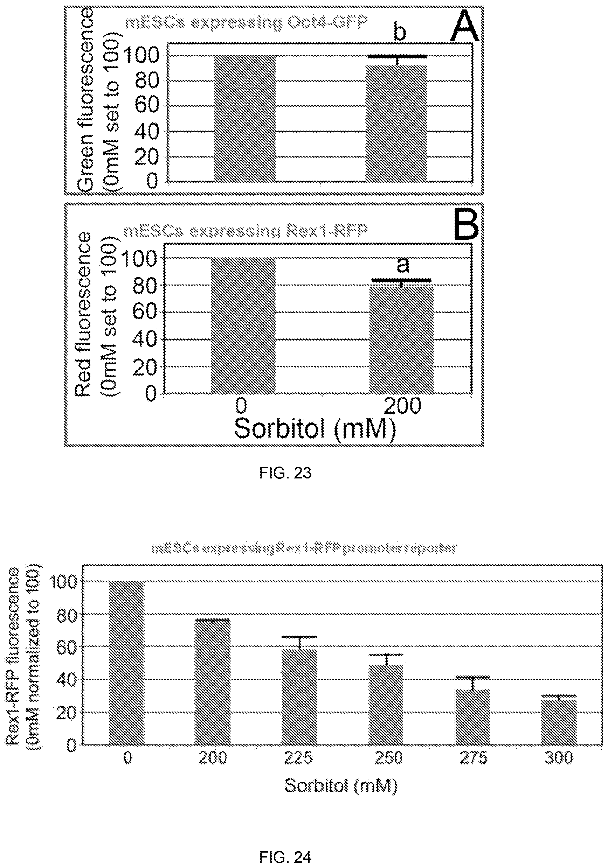

FIGs. 23A and 23B. Stress induces loss of Rex1-RFP but not Oct4-GFP after days of 200mM sorbitol. Viable pluripotency reporter Rex1-RFP ESCs (FIG. 23A) or Oct4-GFP ESCs (FIG. 23B) were cultured in 94-well plates to 30% confluence. Cells were then treated with 200mM of sorbitol as an osmotic stress for 3 days. Fluorescence was determined from live ESCs using a plate reader from 200mM sorbitol treated cells was normalized with the non-sorbitol treated control cells. Four independent experiments were carried out and plotted here. By t test, Oct4-GFP fluorescence in FIG. 23A was not significantly different (b) with stress P=0.327 and in FIG. 23B Rex1-RFP fluorescence was significantly different with stress (a) P<0.019.
FIG. 24. Viable Rex1 promoter reporter responds with stress treatment in a dose-dependent manner in flow cytometry assay. mESCs expressing Rex1 promoter reporter were cultured in 12-well plates till 30% confluent. Cells were then treated with 200-300mM of sorbitol in 25mM increments for 3 days. After trypsinization cell suspensions were assayed by flow cytometry. Histograms shown here are from three independent experiment and (a) indicates significant decrease in Rex1-RFP fluorescence at all sorbitol doses from 200-300mM as determined by ANOVA followed by Dunnett two-sided post hoc test, n=3, P<0.01.

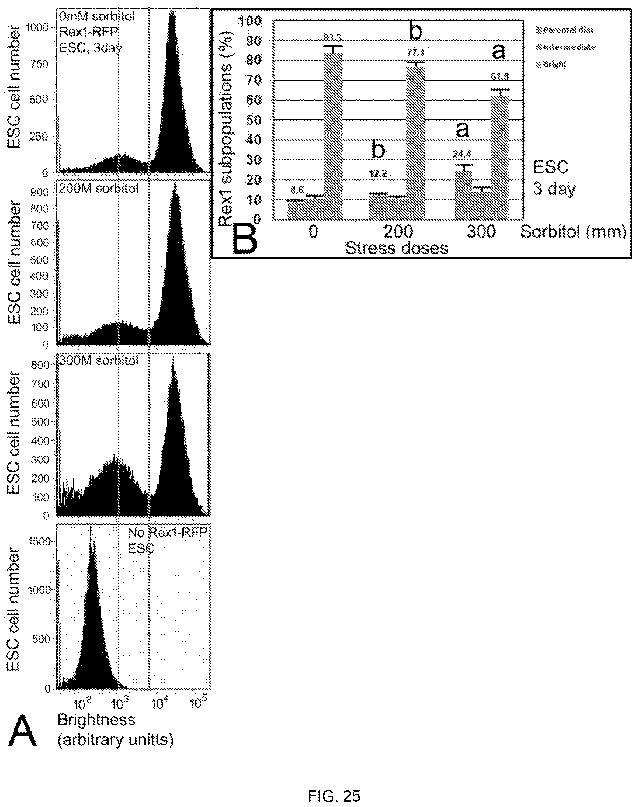

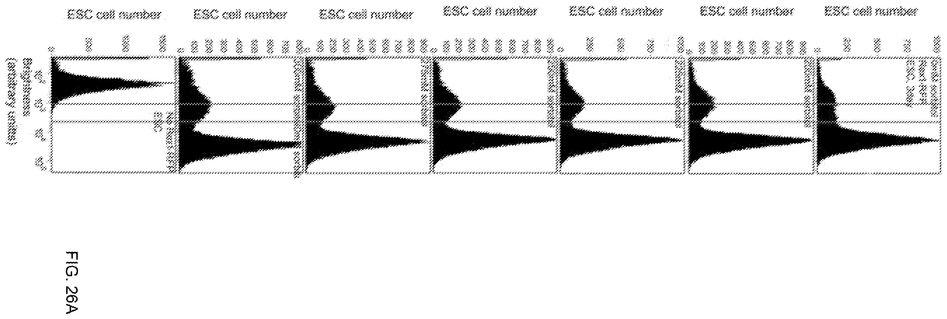

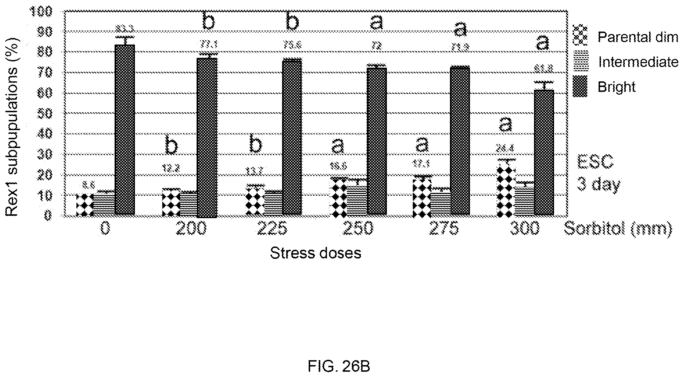

FIGs. 25A and 25B. Rex1 promoter RFP reporter ESCs respond to stress treatment with a dose-dependent 2.8-fold increase in the low Rex1 activity subpopulation and a 20% decrease in high activity Rex1 subpopulation. Rex1-RFP mESCs expressing Rex1 promoter reporter were cultured in 12-well plates until 30% confluent, treated with OmM, 200mM, and 300mM sorbitol for 3 days, trypsinized, and cell suspensions were assayed by flow cytometry. Arbitrary thresholds (vertical red lines) were drawn at 5x103 on the X-axis between the middle of the two peaks and at 2 standard deviations above the mean of the parental (before infection) dim population shown in the bottom panel. Areas in parental dim, intermediate dim, and bright were defined by the 2 vertical red lines and were quantified as pixels using Image J from data in (FIG. 25A) to give histograms in (FIG. 25B). The histogram bars (FIG. 25B) are derived from three independent experiments (see also FIG. 26A and 26B) with errors bars showing s.e.m, One-way ANOVA was performed followed by Dunnett's post hoc test; (a) indicates significantly smaller bright cell subpopulation and greater parental dim cells at 300mM sorbitol (a) (P<0.05), but no significant decrease in bright cell subpopulation or increase in parental dim cells at 200mM sorbitol (b).significant.
FIGs. 26A and 26B. Viable Rex1 promoter reporter responds with stress treatment in a dose-dependent manner in flow cytometry assay. mESCs expressing Rex1 promoter reporter were cultured in 12-well plates till 30% confluence. Cells were then treated with 200-300 mM of sorbitol in 25 mM increments for 3 days. After trypsinization, the cell suspensions were subjected for flow cytometry assay. An arbitrary threshold was drawn at 5x10³ on the x-axis and another near the low point between the bright peak and the dimmer peak to its left (FIG. 26A). This created three areas of non-transgenic Parental ESC dim at the level of ESCs that do not express Rex1-RFP (bottom graph of the four). Intermediate and bright groups were also defined by the two vertical red lines. Area sizes of the two peaks were quantified using Image J. The histogram bars (FIG. 26B) are derived by three independent experiments, one of which is shown in (FIG. 26A), errors bars showing s.e.m, One-way ANOVA was performed followed by Dunnett's post hoc test using OmM as reference for parental dim, intermediate dim, and bright separately. (a) indicates significant decrease in bright subpopulations and increase in parental dim subpopulations at 250-300mM sorbitol (P<0.01) and (b) indicates no significance at 200-225mM sorbitol for changes in Rex1-RFP bright or parental dim subpopulations.

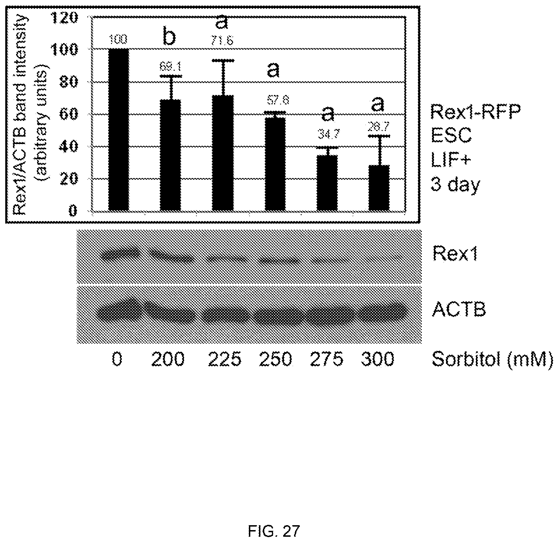

FIG. 27. Rex1 promoter RFP have a stress dose-dependent loss of endogenous Rex1 protein corresponding to dose-dependent loss of Rex1 activity shown in FIG. 24. (A) Rex1-RFP mESCs expressing cultured until 30% confluent, treated with 0mM, 200-300mM sorbitol in 25mM increments for 3 days (as in FIG. 24), lysed, size fractionated by SDS-PAGE, blotted, probed for Rex1 and ACTB proteins and analyzed for changes compared to time zero and for stress dose responses. The histogram bars are derived from three independent experiments, one of which is shown in (A), shown are X+/-s.e.m., n=3, (a) is P<0.01 and (b) indicates not significant. (B). One-way ANOVA was performed followed by Dunnett's post hoc test; (a) indicates significantly lower endogenous Rex1 at 225-300mM sorbitol (P<0.05) but not at 200mM sorbitol (b).

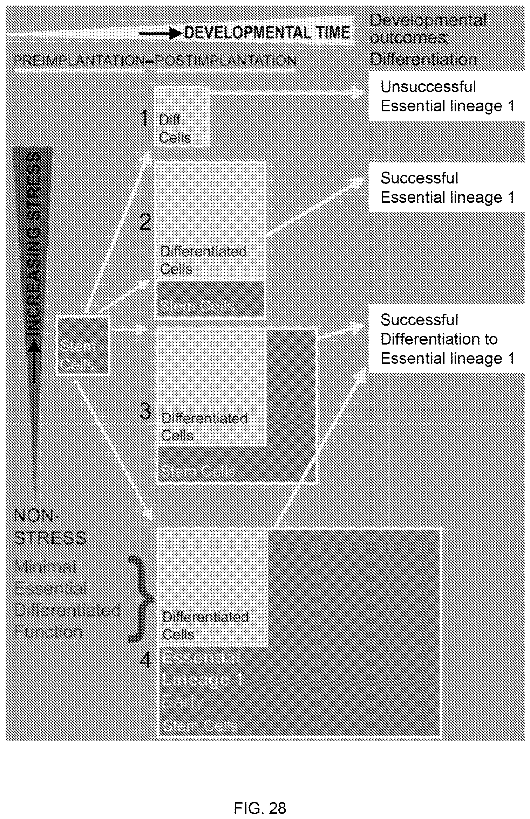

FIG. 28. Partial validation of the Theory of Compensatory Differentiation whereby stress *decreases in size* the normal levels of stem cell expansion and, in the nonmorbid stress range where doubling rates were near-normal, the diminished potency group that is enabled to differentiate is *constant in size.* Previous modeling [18-19] of the Theory of Compensatory Differentiation was based on biochemical averages of mTSCs and mESCs undergoing stress dose-dependent diminished stem cell population expansion and simultaneous potency factor protein decrease and 1^{st} lineage differentiation factor increase. A modified Venn diagram from previous modeling is presented here and analyzed with respect to supporting flow cytometry data for changes in potency subpopulation sizes derived from Table 8 and discussed in the text. For convenience, Table 8 is reproduced here:

| **Table 1.** | **Stress induces % potency loss but total subpopulation to differentiation is constant.** | | | | | | | | | | | | | |
|---|---|---|---|---|---|---|---|---|---|---|---|---|---|---|
| **Sorbitol (mM)** | | 0 | | 200 | | 225 | | 250 | | 275 | | 300 | | LIF+ |
| | | % | # | % | # | % | # | % | # | % | # | % | # | |
| **Bright** | | 833 | | 77.1 | | 756 | | 72 | | 71.9 | | 61.8 | | |
| **Intermediate** | | 8.1 | | 10.7 | | 10.7 | | 11.4 | | 11 | | 13.8 | | |
| **Dim** | | 86 | | 12.2 | | 13.7 | | 16.6 | | 17.1 | 264 | 24.4 | | |
| **Intermediate+ Dim** | | 16.7 | | 22.9 | | 24.4 | | 28 | | 28.1 | | 28.1 | | |
| %Dim+intermediate# | | | | | | | | | | | | | | |
| X | | | | | | | | | | | | | | |
| Cell# | 16.7 x 460 | | | 22.9 x 432 | | 24.4 x 407 | | 28 x 356 | | 28.1 x 264 | | 28.2 x 161 | | |
| | 7683 | | | 9893 | | 9930 | | 9968 | | 7418 | | 4540 | | |
| Total % / # | | 100 | 460 | 100 | 432 | 100 | 407 | 100 | 356 | 100 | 264 | 100 | 161 | |

| Sorbitol | | Duration | | Relative Cell # | | Doubling rate | | | Intermediate and very dim (%very low potency) x cell number | | | | | |
|---|---|---|---|---|---|---|---|---|---|---|---|---|---|---|
| | | | | OUTSIDE BOX++ | | | | | INSIDE BOX cell number++ | | | | | |
| 0 | | 0 | | 100 | | | | | | | | | | |
| 0 | | 72 | | 460 | | 32.7hr | | | | | | | | |
| 200 | | 72 | | 432 | | 34,1 hr | | | 9893 | | | | | |
| 225 | | 72 | | 407 | | 35.6hr | | | 9930 | | | | | |
| 250 | | 72 | | 356 | | 39.3hr | | | 9968 | | | | | |
| 275 | | 72 | | 264++ | | 51.4hr | | | 7418 | | | | | |
| 300 | | 72 | | 162 | | 103.4hr | | | 4540 | | | | | |

| | | | | | | | | | | | | | | |
|---|---|---|---|---|---|---|---|---|---|---|---|---|---|---|
| # Cell counts for Figure 4A multiplied by total number of intermediate and dim Rex1 activity populations to ascertain. ++/++/++ The Inside box (dark green) data and outside box (light green) data refer to the Venn diagram in Figure 6. The data in red show the non-linear range where adaptation breaks down and the previously defined cellular toxicity becomes dominant. | | | | | | | | | | | | | | |

### DETAILED DESCRIPTION

**The** invention is defined in the claims. Any subject-matter which is described herein, but which is not claimed, does not form part of the invention. The present disclosure provides systems and methods (S/M) to detect stress in stem cells and uses thereof. The described S/M can detect sub-morbid stress in stem cells that can lead to stem cell depletion and predict later organismal toxicity and morbidity.

**"Stem** cells" are cells that are totipotent, pluripotent, or multipotent and are capable of differentiating into one or more different cell types. Stem cells may be derived from any organism, e.g. any mammalian species, e.g. human, primate, equine, bovine, porcine, canine, feline, etc. In certain embodiments (CE), they are derived from a human or a mouse.

As indicated earlier, a totipotent stem cell can give rise to all of the embryonic and extraembryonic tissues of an organism. An example of a totipotent stem cell is a fertilized oocyte.

A pluripotent stem cell can give rise to progeny that can undergo differentiation, under appropriate conditions, into cell types that collectively demonstrate characteristics associated with cell lineages from all of the three germinal layers (endoderm, mesoderm, and ectoderm). Pluripotent stem cells contribute to all tissues of a prenatal, postnatal or adult animal. Cell pluripotency is a continuum, ranging from the completely pluripotent cell that can form every cell of the embryo proper, e.g., ESCs and induced pluripotent stem cells (iPSCs), to the incompletely or partially pluripotent cell that can form cells of all three germ layers but may not exhibit germline transmission or ability to generate a whole organism. A standard art-accepted test to establish pluripotency of a cell population is ability to form a teratoma in 8-12 week old SCID mice. Expression of certain combinations of molecular markers are also pluripotent stem cell characteristics. For example, human pluripotent stem cells express markers including: SSEA-3, SSEA-4, TRA-1-60, TRA-1-81, TRA-2-49/6E, ALP, Sox2, E-cadherin, UTF-1, Oct4, Rex1, and Nanog.

A multipotent stem cell is capable of differentiating into at least two differentiated cell types.

S/M for obtaining totipotent, pluripotent and multipotent stem cells from humans and numerous other animals (e.g., monkeys, mice) are described in, e.g., US Pat. Nos. 5,453,357; 5,523,226; 5,589,376; 5,340,740; and 5,166,065; Evans, Theriogenology, 33(1):125, 1990; Notarianni, J. Reprod. Fertil., 41(Suppl.):51, 1990; Giles, Mol. Reprod. Dev., 36:130, 1993; Sukoyan, Mol. Reprod. Dev., 33:418, 1992; Sukoyan, Mol. Reprod. Dev., 36:148, 1993; Iannaccone, Dev. Biol., 163:288, 1994; Evans & Kaufman, Nature, 292:154, 1981; Martin, Proc. Natl. Acad. Sci. USA, 78:7634, 1981; Doetschman, Dev. Biol., 127:224, 1988; Graves & Moreadith, Mol. Reprod. Dev., 36:424, 1993; and Bradley, Nature, 309:255, 1984.

Particular examples of stem cells include embryonic stem cells; cord blood stem cells; umbilical cord matrix stem cells; tissue-specific stem cells; and placental TSCs. Progenitor cells are also stem cells.

Embryonic stem cells (ESCs) are stem cells derived from the inner cell mass (ICM) of an early stage embryo known as a blastocyst. Human embryos reach the blastocyst stage 4-5 days post fertilization, at 50-150 cells. ESCs include those derived from an embryo of up to 7 days after fertilization. ESCs are pluripotent and have the capability of proliferating indefinitely in culture, under conditions that allow their proliferation without differentiation.

S/M for isolating ESCs are described in, e.g., US Pat. Nos. 6,090,622; 5,843,780; 5,340,740; and 5,656,479; PCT WO00/27995; PCT WO99/27076; Matsui, Cell, 70:841, 1992; Thomson, Science, 282:114, 1998; Shamblott, Proc. Natl. Acad. Sci. USA, 95:13726, 1998; Reubinoff, Nat. Biotech., 18:399, 2000; Pain, Development, 122:2339, 1996; Wheeler, Reprod. Fertil. Dev., 6:563, 1994; and Shim, Biol. Reprod., 57:1089, 1997. Primate ESCs may be obtained by, e.g., the methods in US Pat. Nos. 5,843,780 and 6,200,806. Primate and human stem cells may also be obtained from commercial sources such as WiCell, Madison, WI.

S/M for isolating cord blood stem cells or umbilical cord matrix stem cells are described in, e.g., Brunstein, Br. J. Haematol., 137(I):20, 2007; Sun, Biochem. Biophys. Res. Commun., 354(4):919, 2007; Riodan, Transl. Med., 30:5, 2007; and Weiss, Stem Cell Rev., 2(2):155, 2006.

Mouse placental TSCs, like mouse ESCs, are derived from a blastocyst arising at E3.5 (3.5 days after fertilization). The growth factor fibroblast growth factor (FGF)4 is synthesized by the ICM (Rappolee, Development, 120:2259, 1994) and this is necessary to maintain the adjacent polar trophectoderm, source of TSCs, in a stem cell state (Chai, Dev. Biol., 198:105, 1998 (Chai, 1998)). The isolation of mouse TSCs was 1^{st} reported in 1998 (Tanaka, Science, 282:2072, 1998 (Tanaka, 1998)) and used FGF4, the FGF4 cofactor heparin, and conditioned medium from mouse embryonic fibroblasts (MEF-CM) to supply additional necessary cofactors. It has been shown that TGFB family members activin and TGFB1 can replace MEF-CM. Erlebacher, Dev. Biol., 275:158, 2004). In culture, removal of FGF4 enables differentiation of TSC to produce several different lineages that are defined by expression of Hand1, Stra13, PL1, Proliferin, Gcm1, SyncytinA, Ctsq, PL2, Tfeb, Tpbpa, Mash2. During FGF4 removal TSCs lose the potency factors TEAD4, Cdx2, Id2, and ErrB (reviewed in Puscheck, Adv Exp Med Biol, 843:77, 2015).

Many types of stress cause potency loss and differentiation increase despite the presence of FGF4. Thus stress destroys potency in two ways, by diminishing normal stem cell growth and by causing differentiation of the remaining fewer cells to compensate for lower cell numbers.

Stem cells used within the scope of the present disclosure can be cultured according to any appropriate method known in the art. An appropriate medium for stem cell culture includes 80% Dulbecco's modified Eagle's medium (DMEM; no pyruvate, high glucose formulation, Gibco BRL), with 20% fetal bovine serum (FBS; Hyclone), 0.1 µM β-mercaptoethanol (Sigma), and 1% non-essential amino acid stock (Gibco BRL). DMEM Knockout can also be used. The culture can further be supplemented with Knock-out serum replacement (KSP). Cultures can also include one or more carbon sources. In CE, the cultures include L-analyl-L-glutamine. The cultures can include serum or can be serum-free. In CE, stem cells can be cultured with an ALK5 inhibitor and one of a MEK or Erk inhibitor, optionally with a GSK3 inhibitor and/or LIF. Mek and GSK3 inhibitors in one media are called 2i (two inhibitor media) and have been shown to aid in maintaining the ground state of ESCs. Marks, Cell, 149:590, 2012. In CE, the culture media include basal media components for cell survival (e.g., vitamins and minerals, optionally in an isotonic condition).

In CE, stem cells can be cultured in contact with feeder cells. Exemplary feeder cells include fibroblast cells, e.g., mouse embryonic fibroblast (MEF) cells, or x-ray inactivated CF1 feeder cells. Methods of culturing stem cells on feeder cells are known in the art.

In CE, stem cells are cultured in the absence of feeder cells. Stem cells, e.g., can be attached directly to a solid culture surface (e.g., a culture plate), e.g., via a molecular tether. Exemplary molecular tethers include matrigel, an extracellular matrix (ECM), ECM analogs, laminin, fibronectin, or collagen.

**The** disclosure is based in part on the finding that stress stimuli (e.g., toxicological compounds, drugs under development, environmental pollutants) cause stem cells to differentiate despite the presence of growth factors and an environment that is meant to sustain proliferation and potency of stem cells of the early conceptus (both ESCs and TSCs). The unique exponential growth phase normally occurs for days after fertilization and before implantation into the uterus, and then one to several weeks after implantation (depending on the mammalian species). During the exponential growth phase ESCs and TSCs increase population size under normal conditions, but have diminished population growth under stressed conditions. Paradoxically, the strategy of both ESCs and TSCs during stress is to compensate for stress-diminished population size by further depleting stem cell number by differentiating to the 1^{st} lineage at the expense of later lineages Thus, compensatory differentiation is linked to prioritized differentiation when stress induces the differentiation to 1^{st} lineage and suppresses later lineages. Compensatory and prioritized differentiation can occur at lower levels of stress that do not cause toxicity (cell death) in individual stem cells, but additively depletes stem cells so that later cell lineages (e.g. heart, liver, etc.) have insufficient stem cells for development. Thus later lineages may be depleted even though toxicants tested may not directly affect these later lineages. Moreover, the described S/M provide assays that are more sensitive and earlier in ontogeny. The S/M can be practiced in vitro, replacing animals needed in toxicological assays.

In CE, the assays and high throughput screens (HTS) of the invention can be used to identify compounds or other potential stressors that can negatively affect development potential. "Developmental potential" refers to the capacity of a stem cell (e.g., pluripotent cell) to divide and differentiate and/or the capacity of an embryo to grow according to expected developmental milestones when compared to an average non-stressed stem cell and/or healthy embryo. The S/M can also provide early indicators of miscarriage or at-risk pregnancy; indicator of infertility and/or the effect of therapeutics on fertility; indicator of ESC stress and placental insufficiency as the issue for the infertility; early detection and prediction of diseases of placental deficiency; an in vitro toxicology screen for compounds that may have teratogenic potential.

As stated earlier, potency factors can be thought of as "brake pedals" and differentiation factors can be thought of as "accelerator pedals" for differentiation. Normal differentiation occurs when extracellular potency-maintaining growth factors (e.g. FGF4 for mouse TSCs or LIF for mouse ESCs) are removed. Stress-induced compensatory and prioritized differentiation occurs despite the presence of these extracellular growth factors. In the stress response, intracellular stress enzymes (e.g. SAPK/JNK, and AMPK) not necessary for normal development induce differentiation using molecular mechanisms that can be used to measure magnitude and toxicological significance of stress:
(i) potency factor or potency marker decrease, (ii) 1^{st} lineage prioritized differentiation factor (e.g., intracellular enzymes) or prioritized (1^{st} lineage) differentiation marker increase, and (iii) later lineage factor or marker decrease. As stated previously, "factors" as used herein are intracellular proteins that lead to a potent or prioritized differentiated state. "Markers" need not lead to the state, but are indicative of its presence. Classification as a factor or marker is not mutually exclusive. The distinction is made largely for descriptive purposes.

Potency factors and/or markers for ESC include Oct4; Sox2; Nanog; Rex1; Klf5; PDCD2; CARM1; Sall4); SSEA1; SSEA3; SSEA4; TRA-1-60; and TRA-1-81. Potency factors and/or markers for TSC include TEAD4; Errβ; Cdx2; Id2; and Elf5.

Differentiation factors and/or markers for ESC include Lrp2; Dab2; Fgf5; Pdgfra; Sox17; Gata4/6; PAX8; NEFL; TSHR; Brachyury; Laminin; AFP; Nestin; Goosecoid; Cubulin; TBP; Vimentin; and Snail. Differentiation factors and/or markers for TSC include PL1; Plf; PL2; Hand1; Stra13; Tpbpa; Mash2; Gcm1; Tfeb; and Ctsq.

Factors and markers with enzyme activity include MEK1; MEK2; AKT; PI3K; AMPK; SAPK/JNK; p38MAPK; GSK3; and JAK/STAT. Sometimes these factors and markers can override brake pedals and accelerator pedals, during runting stress. In CE, MEK1; MEK2; AKT; AMPK; SAPK/JNK; GSK3; and JAK/STAT can be classified as differentiation factors or markers.

Particular factors and markers are described in more detail herein, with additional description regarding its identity as a factor and/or a marker. As examples discussed more fully below for ESCs, Oct4, Nanog, and Rex1 are potency factors that decrease with stress and prioritized differentiation. Pdgfra, Sox17, Dab2, Lrp2, and GATA4/6 are prioritized differentiation markers that increase with 1^{st} lineage. These markers increase with stress-induced differentiation. Brachyury and FGF5 are later lineage prioritized differentiation markers that decrease with stress and prioritized differentiation. AMPK, SAPK and JNK are intracellular stress enzymes that increase with stress and mediate compensatory and prioritized differentiation.

The Oct4 gene, also known as Oct3 or POU5F1, belongs to the POU (Pit-Oct-Unc) transcription factor family. The Oct4 protein (octamer-binding transcription factor 4) can activate the expression of target genes through binding the octameric motif (5'-ATTTGCAT-3). There are two human Oct4 isoforms proteins produced by alternative splicing.

Oct4 functions in protein binding, miRNA binding, DNA binding, sequence-specific DNA binding, ubiquitin protein ligase binding, transcription factor activity, and transcription factor binding. Oct4 is important for early embryogenesis, for embryonic stem cell pluripotency, and is required for maintaining pluripotency. Oct4 is primarily expressed in ESCs, where it is essential in maintaining potency. In later mammalian development, Oct4 expression is confined to the developing germ cells. Oct4 is conserved in human (SEQ ID NO:8), chimpanzee (SEQ ID NO:9), mouse (SEQ ID NO:10), and many other animals.

The SOX2 gene, also known as SRY (sex determining region Y)-box2, encodes a protein that is a member of the SRY-related HMG-box (SOX) family of transcription factors. SOX2 is an intronless gene that lies within an intron of another gene called the SOX2 overlapping transcript (SOX2OT). The transcription factor encoded by SOX2 forms a trimeric complex with OCT4 on DNA and controls the expression of a number of genes involved in embryonic development such as YES1, FGF4, UTF1, and ZFP206. SOX2 is involved in the regulation of embryonic development, stem-cell maintenance in the central nervous system, and the determination of cell fate. The SOX2 gene is conserved in human (SEQ ID NO:1), chimpanzee (SEQ ID NO:2), mouse (SEQ ID NO:3), and many other animals.

The Nanog gene encodes the homeobox protein Nanog. Nanog is thought to function in concert with other factors such as Oct 4 and SOX2. The homeobox protein Nanog binds to the DNA consensus sequence 5'-TAAT[GT][GT]-3' or 5'-[CG][GA][CG]C[GC]ATTAN[GC]-3' (SEQ ID NO:4).

Nanog functions as a transcription regulator involved in inner cell mass and ESC proliferation and self-renewal. Nanog imposes pluripotency on ESC and prevents their differentiation towards extraembryonic endoderm and trophectoderm lineages. When overexpressed, Nanog promotes cells to enter into S phase and proliferate. Nanog is also associated with ovarian germ cell tumors and sacrococcygeal teratoma. The Nanog gene is conserved in human (SEQ ID NO:5), chimpanzee (SEQ ID NO:6), mouse (SEQ ID NO:7), and many other animals.

The Rex1 gene, also called ZFP42 is expressed primarily in undifferentiated stem cells, both in the embryo and the testicles of adults. The Rex1 protein is 310 amino acids long, and has four closely spaced zinc fingers at 188-212, 217-239, 245-269, and 275-299.

Rex1 functions in sequence-specific DNA binding transcription factor activity and is involved in ESC self-renewal. The Rex1 gene is conserved in human (SEQ ID NO:11), chimpanzee (SEQ ID NO:12), mouse (SEQ ID NO:13), and many other animals.

The Errβ gene is also known as estrogen-related receptor beta (ESRRB) and NR3B2. This gene encodes the Steroid hormone receptor ERR2 protein which is similarity to the estrogen receptor. A similar gene in mouse plays an essential role in placental development. The Errβ gene is conserved in human (SEQ ID NO:50), chimpanzee (SEQ ID NO:51), mouse (SEQ ID NO:52), and many other animals.

TEAD4 (TEA domain family member 4) is a member of the transcriptional enhancer factor (TEF) family of transcription factors and is essential for initiating the TSC lineage. It is preferentially expressed in the skeletal muscle, and binds to the M-CAT regulatory element found in promoters of muscle-specific genes to direct gene expression. Alternatively spliced transcripts encode different isoforms. TEAD4 (TEA domain family member 4) plays a key role in the control of organ size and tumor suppression. The TEAD4 gene is conserved in human (SEQ ID NO:70), chimpanzee (SEQ ID NO:72), mouse (SEQ ID NO:71), and many other animals.

The Caudal Type Homeobox 2, or Cdx2, gene is also known as Cdx3 and CDX2/AS. This gene is a member of the caudal-related homeobox transcription factor gene family.

The encoded protein is a major regulator of intestine-specific genes involved in cell growth and differentiation. This protein also plays a role in early embryonic development of the intestinal tract and is essential in establishing TSCs. The Cdx2 gene is conserved in human (SEQ ID NO:44), Rhesus monkey (SEQ ID NO:45), mouse (SEQ ID NO:46), and many other animals.

The Id2 gene is also known as the cell growth-inhibiting gene 8 (GIG8), Id2A, Id2H, and bHLHb26. The Id2 gene encodes for a protein called DNA-binding protein inhibitor ID-2. That protein belongs to the inhibitor of DNA binding family, members of which are transcriptional regulators that contain a helix-loop-helix (HLH) domain but not a basic domain.

Members of this protein family inhibit the functions of basic helix-loop-helix transcription factors in a dominant-negative manner by suppressing their heterodimerization partners through the HLH domains. This protein plays a role in negatively regulating cell differentiation and specifically inhibits TSC differentiation in humans and mouse. The Id2 gene is conserved in human (SEQ ID NO:47), chimpanzee (SEQ ID NO:48), mouse (SEQ ID NO:49), and many other animals.

The SAPK gene is also known as JNK2, MAPK9, p54a, JNK2A, JNK2B, SAPK1a, JNK2BETA, p54aSAPK, and JNK2α. SAPK encodes the protein mitogen-activated protein (MAP) kinase 9 (MAPK9) which is a member of the MAP kinase family. Several alternatively spliced transcript variants encoding distinct isoforms have been reported. The different isoforms vary in the use of an alternate internal coding exon of the same length.

MAP kinases act as an integration point for multiple biochemical signals, and are involved in a wide variety of cellular processes such as proliferation, differentiation, transcription regulation, and development. MAPK9 targets specific transcription factors, and thus mediates immediate-early gene expression in response to various cell stimuli. MAPK9 is closely related to MAPK8, both of which are involved in UV radiation-induced apoptosis. This gene and MAPK8 are also known as c-Jun N-terminal kinases. Studies of this gene's mouse counterpart suggest a key role in T-cell differentiation. The SAPK gene is conserved in human (SEQ ID NO:38), chimpanzee (SEQ ID NO:39), mouse (SEQ ID NO:40), and many other animals.

The JNK gene, also known as MAPK8, encodes the C-jun-amino-terminal kinase-interacting protein 3 enzyme. This enzyme is a member of the MAP kinase family which includes MAPK9. Five alternatively spliced transcript variants encoding distinct isoforms have been reported.

JNK mediates immediate-early gene expression in response to cell stress stimuli by phosphorylating specific transcription factors. The activation of this kinase by tumor-necrosis factor alpha (TNF-α) is found to be required for TNF-α induced apoptosis. This kinase is also involved in UV radiation induced apoptosis. The MAPK8 gene is conserved in human (SEQ ID NO:41), chimpanzee (SEQ ID NO:42), mouse (SEQ ID NO:43), and many other animals.

The MEK1 gene is also known as mitogen-activated protein kinase kinase 1 (MAPKK1), CFC3, and PRKMK1. MAPKK1 has been shown to interact with C-Raf, Phosphatidylethanolamine binding protein 1, MAP2K1IP1, GRB10, MAPK3, MAPK8IP3, MAPK1, MP1, and MAP3K1.

The protein encoded by MEK1 is a member of the dual specificity protein kinase family, which acts as a mitogen-activated protein (MAP) kinase. This protein kinase lies upstream of MAP kinases and stimulates the enzymatic activity of MAP kinases upon wide variety of extra-and intracellular signals. As an essential component of the MAP kinase signal transduction pathway, this kinase regulates many processes such as proliferation, differentiation, transcription regulation, and development. The MEK1 gene is conserved in human (SEQ ID NO:53), chimpanzee (SEQ ID NO:54), mouse (SEQ ID NO:55), and many other animals.

The MEK2 gene is also known as mitogen-activated protein kinase kinase 2 (MAPKK2), MAP2K2, CFC4, MKK2, and PRKMK2. The protein encoded by this gene is a dual specificity protein kinase that belongs to the MAP kinase family.

This kinase plays a critical role in mitogen growth factor signal transduction. It phosphorylates and activates MAPK1/ERK2 and MAPK2/ERK3. The activation of this kinase is dependent phosphorylation by MAP kinase kinases. The MEK2 gene is conserved in human (SEQ ID NO:56), chimpanzee (SEQ ID NO:57), mouse (SEQ ID NO:58), and many other animals.

The AKT gene is also known as v-akt murine thymoma viral oncogene homolog 1, AKT1, PKB, PRKBA, PKB α, and RAC α. There are three isoforms of Akt; Akt 1, 2 and 3 (also known as PKB-α, -β, and -γ). The protein encoded by this gene is called RAC-alpha serine/threonine-protein kinase.

AKT is involved in regulating many processes including metabolism, proliferation, cell survival, and growth. The regulation is mediated through serine and/or threonine phosphorylation of many downstream, substrates. Over 100 substrate candidates have been reported. The AKT gene is conserved in human (SEQ ID NO:59), chimpanzee (SEQ ID NO:60), mouse (SEQ ID NO:61), and many other animals.

The placental Lactogen gene (PL1) is also known as PL, CSH1, CSA, CS-1, CSMT, hCS-1, and hCS-A. The protein encoded by this gene is a member of the somatotropin/prolactin family of hormones. The PL1 gene is expressed mainly in the placenta and is produced by the 1^{st} lineage differentiating from mouse TSC utilizes multiple transcription initiation sites.

PL1 plays an important role in growth control. The encoded protein is produced only during pregnancy and is involved in stimulating lactation, fetal growth, and metabolism. The human PL1 gene (SEQ ID NO:62) has orthologs in chimpanzee (SEQ ID NO:63), and many other animals.

The Plf gene is also known as, Prl2c2 (prolactin family 2, subfamily c, member 2), Plf1, MRP-1, and PLF-1. This gene encodes the anterior pituitary hormone prolactin which is also known as luteotropic hormone or luteotropin.

Prolactin (Prl), is a growth regulator for many tissues. It is essential for promoting lactation in the mammary glands. The Prl gene is conserved in human (SEQ ID NO:64), chimpanzee (SEQ ID NO:65), mouse (SEQ ID NO:66), and many other animals.

The protein encoded by the Lrp2 gene is called low density lipoprotein-related protein 2. The Lrp2 protein is also known as. Lrp2 is a multi-ligand endocytic receptor glycoprotein with a large amino-terminal extracellular domain, a single transmembrane domain, and a short carboxy-terminal cytoplasmic tail that is found in the plasma membrane of many absorptive epithelial cells..

Lrp2 is expressed in many different tissues but primarily in absorptive epithelial tissues such as the kidney. The extracellular ligand-binding-domains of the protein bind diverse macromolecules including albumin, apolipoproteins B/ E, and lipoprotein lipase. Lrp2 is important for the reuptake of numerous ligands, including lipoproteins, sterols, vitamin-binding proteins, and hormones. The Lrp2 gene is conserved in human (SEQ ID NO:23), chimpanzee (SEQ ID NO:24), mouse (SEQ ID NO:25), and many and other animals.

The DAB2 gene was initially named DOC2 (Differentially expressed in Ovarian Cancer) and is distinct from the DOC2A and DOC2B genes. Dab2 is expressed in normal ovarian epithelial cells, but is decreased in ovarian carcinomas, suggesting a role as a tumor suppressor. The Dab2 gene is conserved in human (SEQ ID NO:20), chimpanzee (SEQ ID NO:21), mouse (SEQ ID NO:22), and many other animals.

The Fgf5 gene is a member of the fibroblast growth factor (FGF) family that encodes the Fibroblast Growth Factor 5 protein.

FGF family members possess broad mitogenic and cell survival activities, and are involved in a variety of biological processes, including embryonic development, cell growth, cell proliferation, morphogenesis, cell differentiation, tissue repair, tumor growth, and invasion. This gene is as an oncogene, transforming transfected mammalian cells. The Fgf5 gene is conserved in human (SEQ ID NO:32), chimpanzee (SEQ ID NO:33), mouse (SEQ ID NO:34), and many other animals.

The Pdgfra (platelet-derived growth factor receptor, alpha polypeptide) gene codes for the alpha-type platelet-derived growth factor receptor protein. In addition to Pdgfra, there is another isoform Pdgfrb. The two isoforms can form homo- or heterodimers. Pdgfra functions as a tyrosine-protein kinase that acts as a cell-surface receptor for Pdgfa, Pdgfb, and Pdgfc. The α-type platelet-derived growth factor receptor protein plays an important role in the regulation of embryonic development, cell proliferation, survival, and chemotaxis. The Pdgfra gene is conserved in human (SEQ ID NO:14), chimpanzee (SEQ ID NO:15), and many other animals. The homologous gene in mice (SEQ ID NO:16) includes a protein tyrosine kinase conserved domain rather than the protein kinase domain found in the human gene.

The cubulin gene is also known as CUBN, IFCR, MGA1, and gp280. Cubulin is located within the epithelium of intestine and kidney. Cubulin acts as a receptor for intrinsic factor-vitamin B12 complexes. Cubulin plays a role in lipoprotein, vitamin and iron metabolism, by facilitating their uptake. This gene plays an important role in the development of the peri-implantation embryo through internalization of APOA1 and cholesterol. It also binds to LGALS3 at the maternal-fetal interface. The cubulin gene is conserved in human (SEQ ID NO:67), mouse (SEQ ID NO:68), and many other animals.

The Sox17 (SRY (sex determining region Y)-box 17) gene encodes a member of the SOX (SRY-related HMG-box) family of transcription factors. The encoded protein acts as a transcriptional regulator after forming a complex with other proteins. Sox17 binds target promoter DNA and bends the DNA by binding to the sequences 5'-AACAAT-'3 or 5'-AACAAAG-3'.

SOX17 is involved in the regulation of embryonic development and in the determination of cell fate. The SOX17 gene is conserved in human (SEQ ID NO:17), chimpanzee (SEQ ID NO:18), mouse (SEQ ID NO:19), and many other animals.

The GATA6 (GATA binding protein 6 or transcription factor GATA-6) gene encodes a protein that is a member of a small family of zinc finger transcription factors. The GATA6 protein preferentially binds (A/T/C)GAT(A/T)(A) of the consensus binding sequence.

GATA6 is expressed during early embryogenesis and localizes to endo- and meso-dermally derived cells during later embryogenesis and plays an important role in the regulation of cellular differentiation and organogenesis during vertebrate development such as gut, lung, and heart development. The GATA6 gene is conserved in human (SEQ ID NO:26), chimpanzee (SEQ ID NO:27), mouse (SEQ ID NO:28), and many other animals.

GATA4 is involved in embryogenesis and in myocardial differentiation and function and testicular development. It is a transcriptional activator that binds to the consensus sequence 5'-AGATAG-3'. The GATA4 gene is conserved in human (SEQ ID NO:73), mouse (SEQ ID NO:74), and many other animals.

The T gene encodes the Brachury protein which is an embryonic nuclear transcription factor that binds to a specific DNA element, the palindromic T-site. Brachury binds through a region in its N-terminus called the T-box. Two transcript variants encoding different isoforms have been found for this gene. The gene brachyury appears to have a conserved role in defining the midline of a bilateral organism, and thus the establishment of the anterior-posterior axis; this function is apparent in chordates and mollusks. Brachury effects transcription of genes required for mesoderm formation and differentiation and is localized to notochord-derived cells. The T gene is conserved in human (SEQ ID NO:29), chimpanzee (SEQ ID NO:30), mouse (SEQ ID NO:31), and many other animals.

The AMPK genes are also known as PRKAA1/2 and AMPKa1/2. The protein encoded by this gene belongs to the ser/thr protein kinase family. The functional enzyme is composed of α-(catalytic) and β/γ-(regulatory) subunits. The AMPK genes encode proteins called 5'-prime-AMP-activated protein kinase which is the catalytic subunit. The 5'-AMP-activated protein kinase catalytic subunit α1 has a magnesium cofactor and is 559 amino acids long with a molecular weight of 64,009 Da. Alternatively spliced transcript variants encoding distinct isoforms have been observed.

AMPK is a cellular energy sensor. AMPK regulates key metabolic enzymes through phosphorylation. This kinase activity is activated by stimuli that increase the AMP/ATP ratio. The AMPK gene is conserved in human (SEQ ID NO:35), chimpanzee (SEQ ID NO:36), mouse (SEQ ID NO:37), and many other animals.

PAX8 encodes a member of the paired box (PAX) family of transcription factors involved in functional differentiation and maintenance of thyroid-related cells. The PAX8 gene is conserved in human (SEQ ID NO:75), chimpanzee (SEQ ID NO:77), mouse (SEQ ID NO:76), and many other animals.

The NEFL gene encodes neurofilaments in the axoskeleton. They maintain neuronal caliber and also play a role in intracellular transport to axons and dendrites. The NEFL gene is conserved in human (SEQ ID NO:78), chimpanzee (SEQ ID NO:80), mouse (SEQ ID NO:79), and many other animals.

The TSHR gene encodes membrane proteins that serve as receptors for thyrothropin and thyrostimulin. These proteins are involved in thyroid cell metabolism. Different transcript variants have been identified. The TSHR gene is conserved in human (SEQ ID NO:81), chimpanzee (SEQ ID NO:83), mouse (SEQ ID NO:82), and many other animals.

The laminin gene encodes proteins that mediate attachment, migration and organization of cells into embryonic tissues. The proteins function by interacting with the extracellular matrix. The laminin gene is conserved in human (SEQ ID NO:84), ), chimpanzee (SEQ ID NO:86), mouse (SEQ ID NO:85) and many other animals.

The AFP gene encodes alpha-fetoprotein, a plasma and intracellular protein produced by the yolk sac and liver during fetal development. AFP, the fetal counterpart of serum albumin and binds copper, nickel, fatty acids and bilirubin. The AFP gene is conserved in human (SEQ ID NO:87), chimpanzee (SEQ ID NO:89), mouse (SEQ ID NO:88), and many other animals.

The NES gene encodes a member of the intermediate filament protein family and is expressed primarily in nerve cells. It is required for survival, renewal and mitogen-stimulated proliferation of neural progenitor cells. The NES gene is conserved in human (SEQ ID NO:90), chimpanzee (SEQ ID NO:92), mouse (SEQ ID NO:91), and many other animals.

The Goosecoid (GSC) gene: encodes a member of the bicoid subfamily of the paired (PRD) homeobox family of proteins. The encoded protein acts as a transcription factor and plays a role in spatial programming within discrete lineage compartments during organogenesis. The GSC gene is conserved in human (SEQ ID NO:93), chimpanzee (SEQ ID NO:95), mouse (SEQ ID NO:94), and many other animals.

Oct4 and Rex1 are markers of the 1^{st} ESC progenitors that arise in the embryo within 3.5 days (E3.5) after fertilization. Upon differentiation to any lineage, Rex1 is decreased (e.g., lost) and Oct4 is decreased after commitment to the 1^{st} differentiated lineage of extraembryonic ectoderm (ExEndo). Thus, the presence of Oct4 and Rex1 indicates stem cell potency. In contrast, Pdgfra is a growth factor receptor and Dab2 a lipid nutrient-acquiring protein that are both activated immediately upon 1^{st} differentiation of ESC to ExEndo. However, Pdgfra is expressed in all ExEndo lineages and Dab2 only in 1^{st} lineage and its maturing descendants. Thus, the presence of Pdgfra and Dab2 indicate loss of stem cell potency under conditions wherein potency would normally be maintained (e.g., that a stress has occurred).

Three embryonic genes decrease and then rapidly increase when ESCs are stressed. These 3 genes are Sox2, Oct4 and Nanog. There is a 4^{th} gene, Rex1, whose expression after stress decreases but does not increase thereafter as Sox2, Oct4 and Nanog do. Once expression for Rex1 decreases, this decrease indicates that stress has occurred. In CE, the stress leads to reduced stem cell potency and development potential. Within these embodiments, stress can be detected by time course evaluation of Sox2, Oct4 and Nanog expression and/or time course or single time point evaluation of Rex1.

In CE, the terminal differentiation of stem cells leads to production of gene products of parenchymal differentiation that distinguishes the 1^{st} lineage available to ESCs and TSCs, and from lineages that normally arise later from these two stem cell types. Thus the careful measure of the ratio of 1^{st} /later lineage intracellular and secreted products predicts stressed stem cells and pregnancies. Specifically for stressed TSCs, compensatory and prioritized differentiation predicts that earliest pregnancy tests will have higher or similar 1^{st} lineage hormone (e.g., PL1), and decreased later lineage hormones from maternal blood at the earliest times.

The occurrence of a stress can be detected by measuring the expression or activity of potency and/or differentiation factors that cause the up- or down-regulation of a marker. The expression or activity of these factors can identify the occurrence of stress-induced compensatory and prioritized differentiation. For example, increased expression of PL1 or Plf is associated with an up-regulation of SAPK's expression or activity. As a further example, the continued down-regulation of Rex1 following stress is mediated by an up-regulation of JNK's expression or activity. Further relationships between the factors and markers are described in detail in accompanying Examples 1, 2 and 3.

As is understood by one of ordinary skill in the art, "up-regulation" and "down-regulation" of gene or protein expression, and protein activity can be measured against a relevant control condition including relative to the expression or activity of an unstressed stem cell or relative to differentiation mediated by removal of growth factors that maintain stemness in culture (e.g., normal differentiation control). In CE, up-regulation or down-regulation will be a statistically-significant difference (e.g., p<0.05) from a relevant control condition.

In CE, stem cells are assessed by measuring gene expression. In such embodiments, the stem cell parameter is a gene expression level or profile. Determining the expression of one or more genes, e.g., obtaining an expression profile) may be made by measuring nucleic acid transcripts, e.g. mRNAs, of genes of interest, e.g. a nucleic acid expression profile; or by measuring levels of one or more different proteins/polypeptides that are expression products of one or more genes of interest, (e.g. a proteomic expression profile). Thus, the terms "expression profile" and "expression evaluation" can be used broadly to include a gene expression profile at the nucleic acid level or a gene expression profile at the protein level.

In CE, expression of genes may be evaluated by obtaining a nucleic acid expression profile, where the amount or level of one or more nucleic acids in a sample is determined. The nucleic acid sample includes a plurality or population of distinct nucleic acids that includes the expression information of the genes of interest of the stem cell being assessed. The nucleic acid may include RNA or DNA nucleic acids, e.g., mRNA, cRNA, cDNA etc., so long as the sample retains the expression information of the stem cell from which it is obtained. The sample may be prepared in a number of different ways, e.g., by mRNA isolation from a cell, where the isolated mRNA is used as is, amplified, employed to prepare cDNA, cRNA, etc., as performed in the differential expression art. The sample may be prepared from a single cell, e.g. a stem cell or a population of stem cells of interest.

The expression profile may be generated from the initial nucleic acid sample using many appropriate protocols. One representative protocol is array-based gene expression profile generation protocols. Such protocols can be hybridization assays in which a nucleic acid that displays "probe" characteristics for each of the genes to be assayed/profiled in the profile to be generated is employed.

Specific hybridization technologies which may be used to generate expression profiles are described in, e.g., US Pat. Nos. 5,143,854; 5,288,644; 5,324,633; 5,432,049; 5,470,710; 5,492,806; 5,503,980; 5,510,270; 5,525,464; 5,547,839; 5,580,732; 5,661,028; and 5,800,992; as well as WO 95/21265; WO 96/31622; WO 97/10365; WO 97/27317; EP 373 203; and EP 785 280. In these S/M, an array of "probe" nucleic acids that includes a probe for each of the phenotype determinative genes whose expression is being assayed is contacted with target nucleic acids. Contact is carried out under hybridization conditions, e.g., stringent hybridization conditions, and unbound nucleic acid is then removed.

In CE, stringent hybridization conditions include e.g., 2 hr to 4 days incubation at 42°C using a DIG-labeled DNA probe (prepared by, e.g., using a DIG labeling system; Roche Diagnostics GmbH, 68298 Mannheim, Germany) in a solution such as DigEasyHyb solution (Roche Diagnostics GmbH) or a solution including 50% formamide, 5XSSC (150 mM NaCl, 15 mM trisodium citrate), 0.02% sodium dodecyl sulfate, 0.1% N-lauroylsarcosine, and 2% blocking reagent (Roche Diagnostics GmbH), followed by washing the filters twice for 5 to 15 minutes in 2XSSC and 0.1% SDS at room temperature and then washing twice for 15-30 minutes in 0.5XSSC and 0.1% SDS or 0.1XSSC and 0.1% SDS at 65-68°C.

Probes can also be designed to have a % sequence identity that is complementary to wild-type gene sequences that lead to conditions that are compatible to produce binding pairs of nucleic acids, e.g., surface bound and solution phase nucleic acids, of sufficient complementarity to provide for the desired level of specificity in the assay while being less compatible to the formation of binding pairs between binding members of insufficient complementarity to provide for the desired specificity. In CE, the probes will have 90% to 100% sequence identity in 1% stepwise increments (e.g., 90%, 91%, 92% up to 100%) that identity a sequence that is complementary to the wild-type sequence being assessed.

"% sequence identity" refers to a relationship between two or more sequences, as determined by comparing the sequences. In the art, "identity" also means the degree of sequence relatedness between sequences as determined by the match between strings of such sequences. "Identity" (often referred to as "similarity") can be readily calculated by appropriate methods, including those described in: Computational Molecular Biology (Lesk, ed.) Oxford University Press, NY 1988; Biocomputing: Informatics and Genome Projects (Smith, ed.) Academic Press, NY 1994; Computer Analysis of Sequence Data, Part I (Griffin and Griffin, eds.) Humana Press, NJ 1994; Sequence Analysis in Molecular Biology (Von Heijne, ed. Academic Press 1987); and Sequence Analysis Primer (Gribskov & Devereux, eds. Oxford University Press, NY 1992. Preferred methods to determine sequence identity are designed to give the best binding between the sequences tested. Methods to determine sequence identity and similarity are codified in publicly available computer programs. Sequence alignments and percent identity calculations may be performed using the Megalign program of the LASERGENE bioinformatics computing suite (DNASTAR, Inc., Madison, Wisconsin). Multiple alignment of the sequences can also be performed using the Clustal method of alignment (Higgins and Sharp CABIOS, 5, 151-153 (1989) with default parameters (GAP PENALTY=10, GAP LENGTH PENALTY=10). Relevant programs also include the GCG suite of programs (Wisconsin Package Version 9.0, Genetics Computer Group (GCG), Madison, Wisconsin); BLASTP, BLASTN, BLASTX (Altschul, J. Mol. Biol., 215:403 199); DNASTAR (DNASTAR, Inc., Madison, Wisconsin); and the FASTA program incorporating the Smith-Waterman algorithm (Pearson, Comput. Methods Genome Res., [Proc. Int. Symp.] 1994, Meeting Date 1992, 111. Editor(s): Suhai, Sandor. Publisher: Plenum, New York, N.Y.. Within the context of this disclosure it will be understood that where sequence analysis software is used for analysis, the results of the analysis are based on the "default values" of the program referenced. As used herein "default values" will mean any set of values or parameters which originally load with the software when 1^{st} initialized.

Resultant patterns of hybridized nucleic acid provides information regarding expression for each of the genes that have been probed, where the expression information is whether or not the gene is expressed and, typically, at what level, where the expression data or expression profile (e.g., in the form of a transcriptome), may be both qualitative and quantitative.

Alternatively, non-array based S/M for quantitating the level of one or more nucleic acids in a sample may be employed, including those based on amplification protocols, e.g., Polymerase Chain Reaction (PCR)-based assays, including quantitative PCR, reverse-transcription PCR (RT-PCR), real-time PCR, etc.

In CE, expression of genes may be evaluated by obtaining a proteomic expression profile, where the amount or level of one or more proteins/polypeptides in the sample is determined. Where the expression profile is proteomic expression profile, i.e. a profile of one or more protein levels in a sample, any convenient protocol for evaluating protein levels may be employed wherein the level of one or more proteins in the assayed sample is determined. While a variety of different manners of assaying for protein levels can be used, one representative protocol is enzyme-linked immunosorbent assays (ELISA). Alternatively, non-ELISA based-S/M for measuring proteins levels in a sample, such as mass spectrometry, proteomic arrays, xMAP^{™} microsphere technology, flow cytometry, western blotting, and immunohistochemistry can be used.

The disclosure includes modified stem cells (which are not part of the invention) with reporter transgene systems that are used to identify compounds or other potential environmental stem cell stressors that cause forced, compensatory or prioritized differentiation and reduced development potential.

In CE of the disclosure, reporter genes include a nucleic acid sequence encoding a reporter. The reporter transgene is inserted via homologous recombination into the stem cell genome so that the reporter gene is in operable combination with the promoter of a factor or marker (e.g., potency or differentiation) of interest. "In operable combination" refers to the linkage of nucleic acid sequences in a manner such that one or more proteins can be produced. A promoter is a regulatory element that facilitates the initiation of transcription of a coding region of a nucleic acid sequence in operable combination (used interchangeably with operably linked) with it. A promoter is typically, though not necessarily, located 5' (i.e., upstream) of a nucleotide sequence of interest whose transcription into mRNA it controls, and provides a site for specific binding by RNA polymerase and other transcription factors for initiation of transcription. With this approach, when a factor or marker gene can be expressed, it is expressed as a reporter.

A "gene" refers to a nucleotide sequence that encodes a protein (e.g., a reporter protein or other proteins described herein). This definition includes various sequence polymorphisms, mutations, and/or sequence variants. In particular embodiments, the sequence polymorphisms, mutations, and/or sequence variants do not affect the function of the encoded protein. The term "gene", depending on the context, refer only to coding sequences or to coding sequences in combination with regulatory regions such as promoters, enhancers, and termination regions. The term further can include all introns and other DNA sequences spliced from the mRNA transcript, along with variants resulting from alternative splice sites. Nucleic acid sequences encoding proteins can be DNA or RNA that directs the expression of the protein or RNA. These nucleic acid sequences may be a DNA sequence that is transcribed into RNA or an RNA sequence that is translated into protein. The nucleic acid sequences include both the full-length nucleic acid sequences as well as non-full-length sequences derived from the full-length protein or RNA. The sequences can also include degenerate codons of the native sequence or sequences that may be introduced to provide codon preference. In addition to particular sequences provided, gene sequences to encode reporter proteins and other proteins are available in publicly available databases and publications.

In CE of the disclosure, a gene of interest encodes any of the potency factors, potency markers, compensatory or prioritized differentiation factors or markers disclosed herein. In CE of the disclosure, a reporter gene encodes any reporter disclosed herein.

The disclosure is not limited to the use of any particular reporter gene. Indeed, the use of a variety of reporters can be used. In CE, the disclosure provides S/M for transfecting stem cells with reporter genes encoding fluorescent proteins, wherein the expression of the fluorescent proteins provides an expression assay in stem cells without having to sacrifice the cells. Fluorescent reporters are known and can be obtained commercially at, e.g., Promega Corporation, Invitrogen, Clontech, Stratagene, BD Biosciences Pharmingen, or Evrogen JSC. Additional examples of fluorescent proteins can be found at, e.g., US Patent No. 6,884,620 and 6,645,761. The disclosure is not limited by the fluorescent protein-encoding gene, and any fluorescent encoding gene can be used.

In CE of the disclosure, the gene encoding the fluorescent protein can be optimized for mammalian expression by codon optimization for mammalian translational machinery. In CE, the disclosure provides S/M for transfecting stem cells with reporter genes encoding enzymatic proteins, wherein the expression of the enzymatic proteins provides a viable expression assay without sacrificing the stem cells. For example, the expression of reporter enzymatic proteins provide light output upon substrate utilization. Examples of such enzymatic proteins that produce light as a byproduct of substrate utilization include, luciferase and β-lactamase. Luciferase encoding reporter genes (e.g. Photinus sp. (GenBank Accession No. AY738222), Renilla sp. (GenBank Accession No. AF025844), Pyrophorus sp. (GenBank Accession No. AY258591 and AY258593), etc.) are commercially available from Promega Corporation, Stratagene and Clontech, e.g., and β-lactamase encoding reporter genes are available from Invitrogen, for example.

In CE of the disclosure, modified stem cells may be transfected with a vector including a wild-type promoter or a synthetic promoter made up of repeated response elements to a given single transcription factor (as done with Rex1 and Oct4 in Li (2015). Submitted to Stem Cells and Development. Rapid development and validation of a Rex1 promoter-RFP (red fluorescent reporter) viable potency activity reporter quantifies stress-induced ESC potency loss and supports the theory of compensatory differentiation. In these embodiments, the vector will integrate into the stem cell genome and on-going cellular events will interact with the inserted promoter. When conditions occur that would cause transcription of the genes associated with the wild-type promoter, expression of the inserted reporter will also occur.

The ability of certain viruses to infect stem cells or enter stem cells via receptor-mediated endocytosis, and to integrate into the stem cell genome and express genes stably and efficiently have made them attractive candidates for the transfer of foreign nucleic acids into stem cells. Non-limiting examples of viral vectors that can be used include adenoviral vectors and retroviral vectors. Adenoviral vectors include those constructs containing adenovirus sequences sufficient to (a) support packaging of a construct and (b) express a cell-specific construct that has been cloned therein. Retroviral vectors integrate their genes into stem cell genomes and are capable of transferring a large amount of foreign genetic material, infecting a broad spectrum of species and cell types (Miller, Am. J. Clin. Oncol." 15(3):216, 1992). Lentiviruses are complex retroviruses, which, in addition to the common retroviral genes gag, pol, and env, contain other genes with regulatory or structural function. See, e.g., Naldini, Science, 272(5259):263, 1996; Zufferey, Nat. Biotechnol., 15(9):871, 1997; US Pat. Nos. 6,013,516 and 5,994,136). Some examples of lentivirus include the Human Immunodeficiency Viruses: HIV-1ad nHIV-. Lentiviral vectors have been generated by multiply attenuating the HIV virulence genes, e.g., the genes env, vif, vpr, vpu and nef are deleted making the vector biologically safe. Recombinant lentiviral vectors are capable of infecting stem cells and can be used for *ex vivo* gene transfer and expression of nucleic acid sequences. For example, recombinant lentivirus capable of infecting cells wherein a suitable host cell is transfected with ≥ vectors carrying the packaging functions, namely gag, pol and env, as well as rev and tat is described in US Pat. No. 5,994,136.

Promoters used in embodiments described herein may be "endogenous" (e.g., wild-type), "exogenous" or "heterologous." An "endogenous" promoter is one that is naturally operably linked with a given gene in the genome. An "exogenous" promoter is one that is not native to the wild-type genome. A "heterologous" promoter is a type of exogenous promoter that shares at least 90% sequence identity with an endogenous promoter.

In CE of the disclosure, reporter gene constructs include a selectable marker. The disclosure is not limited to the use of any particular selectable marker. An exemplary selectable marker includes dominant selectable markers such as the bacterial aminoglycoside 3' phosphotransferase gene (also referred to as the neo gene) that confers resistance to the drug G418 in mammalian cells. Other selectable markers are used in stem cell lines that lack the relevant enzyme activity. Examples of non-dominant selectable markers include the thymidine kinase (tk) gene that is used in conjunction with tk-deficient cell lines and the mammalian hypoxanthine-guanine phosphoribosyl transferase (hprt) gene, which is used in conjunction with hprt-deficient cell lines. A review of the use of selectable markers in mammalian cell lines is provided in Sambrook, J. Molecular Cloning: A Laboratory Manual, 2nd ed., Cold Spring Harbor Laboratory Press, New York (1989) pp. 16.9-16.15.

Gene constructs including vectors and reporter genes described herein can be inserted or "knocked-in" at a desired locus in a stem cell. Methods for generating gene constructs for use in generating knock-in cell lines and techniques for generating such cell lines are described in, e.g., Sambrook 1989, Molecular Cloning, A Laboratory Manual, 3rd ed., Cold Spring Harbor Laboratory; Yoo, Neuron, 37:383, 2003; and Lin, Human Molecular Genetics, 10:137, 2001. Exemplary knock-in methods include *ex vivo* transfection (see, e.g., Wilson, Science, 244:1344, 1989; and Nabel & Baltimore, Nature, 326:711, 1987), optionally with Fugene6 (Roche) or Lipofectamine (Invitrogen); injection (see, e.g., US Pat. Nos. 5,994,624; 5,981,274; 5,945,100; 5,780,448; 5,736,524; 5,702,932; 5,656,610; 5,589,466; and 5,580,859); microinjection (see, e.g., Harland and Weintraub, J. Cell. Biol., 101:1094, 1985; US Pat. No. 5,789,215); electroporation (e.g., US Pat. No. 5,384,253; Tur-Kaspa, Mol. Cell. Biol., 6:716, 1986; Potter, Proc. Nat'l. Acad. Sci. USA, 81:716, 1984); calcium phosphate precipitation (Graham & Van Der Eb, Virology, 52:456, 1973; Rippe, Mol. Cell. Biol., 10:689, 1990); DEAE-dextran followed by polyethylene glycol (Gopal, Mol. Cell. Biol., 5:1188, 1985); direct sonic loading (Fechheimer, Proc. Nat'l Acad. Sci. USA, 84:8463, 1987); liposome mediated transfection (Nicolau & Sene, Biochim. Biophys. Acta, 721:185, 1982; Fraley, Proc. Nat'l. Acad. Sci. USA, 76:3348, 1979; Nicolau Methods Enzymol., 149:157, 1987; Kaneda, Science, 243:375, 1989) and receptor-mediated transfection (Wu & Wu, Biochemistry, 27:887, 1988; Wu & Wu, J. Biol. Chem., 262:4429, 1987) and any combination of such methods.

In CE of the disclosure, brake pedal/potency and accelerator pedal/differentiation constructs and modified viable reporter ESCs can be described as follows: multimerized Oct4 promoters and multimerized Rex1 promoters that drive green fluorescent protein (GFP) and red fluorescent protein (RFP) respectively can be expressed in living ESCs. Following exposure to stress, the Rex1 potency loss should be greater and more permanent than Oct4 loss. These results can be determined by immunoblots for biochemical measures and FACS and microplate reader for visual assays. Microplate readers also provide for HTS for various stresses.

Modified ESCs with a GFP knocked into one of the endogenous Pdgfra loci differentiation reporters have been tested. In these embodiments of the disclosure, GFP expression is driven by the endogenous promoter as it is activated by normal or stressed mechanisms. The specificity of Pdgfra-GFP induction can be validated using other co-markers of 1^{st} lineage induced during stressed differentiation and later lineages that are induced during growth factor removal but suppressed during stressed differentiation. Thus DAB2, LRP2, and GATA6 are appropriate co-markers of 1^{st} lineage ExEndo, FGF5, and SPARC are appropriate markers of other later lineages suppressed by stress-induced differentiation but activated during normal differentiation by removing potency-maintaining growth factors.
CE of ESC reporter cell lines of the disclosure include: (1) Potency measuring viable reporters (decreased with stress): (a) Oct4 multimerized promoter-GFP ("Oct4-GFP"); (b) Rex1 multimerized promoter-RFP ("Rexl-FRP"); (c) Oct4-GFP/Rex1-RFP co-expressing ESCs ("Oct4-GFP/Rex1-RFP"); (2) Differentiated 1^{st} lineage measuring viable reports (increased with stress): (a) Pdgfra promoter-GFP knockin ("Pdgfra-GFP"); (b) Sox17 Endo promoter-GFP knockin ("Sox17-GFP"); (c) DAB2, LRP2 Endo promoter-GFP; (d) GATA6 Endo promoter-GFP; (3) Later lineage measuring promoters (decreased with stress): (a) Brachyury Endo promoter-GFP knockin ("Brachy-GFP"); (b) FGF5 promoter-GFP knockin ("FGF5-GFP"); (4) Stress enzymes that regulate potency loss (AMPK) and 1^{st} lineage gain (SAPK/JNK): (a) Constitutive promoter AMPKAR (AMPK viable activity reporter); and (b) Constitutive promoter JNKAR (SAPK/JNK viable activity reporter).

In CE of the disclosure, modified viable reporters such as PL1 promoter-RFP can be used to measure transcription factors marking the differentiation to 1^{st} lineage available to TSCs.

CE of the disclosure utilizing Rex1-RFP for brake pedal, potency-loss testing and Pdgfra-GFP for accelerator pedal, 1^{st} lineage differentiation-increase testing represent preferred viable ESC-HTS reporters. Because submorbid, runting doses that cause differentiation are used, it is important, and technically feasible to add a nuclear stain to the color plate reader excitation and band pass acquisition and assay the level of diminished stem cell population expansion. At any stress dose, taken together or individually (i) decreased potency, (ii) increased 1^{st} lineage, and (iii) diminished cell population expansion should give an accurate analysis of stress impacts on embryonic and fetal development in response to toxicological stress and is completely unique amongst stem cell assays for toxicology.

In generating an expression profile, in CE a sample is assayed to generate an expression profile that includes expression data for at least 1 gene/protein, sometimes a plurality of genes/proteins, where by plurality is meant at least 2 different genes/proteins, and often at least 3, at least 4, at least 5, or at least 10 different genes/proteins, etc. The Luminex Xmap system (Luminex Corp, Austin, TX) allows for, e.g., 500 simultaneous fluorescent frequency multiplexing.

In the broadest sense, the expression evaluation may be qualitative or quantitative. As such, where detection is qualitative, the S/M provide an assessment, of whether or not the target analyte is present in the sample being assayed. In other embodiments, the S/M provide a quantitative detection of whether the target analyte is present in the sample, e.g., an assessment of the actual amount or relative abundance of the target analyte

S/M can include comparisons to reference levels. Reference levels can be obtained from a dataset. A reference level from a dataset can be derived from previous measures derived from a population. A "population" is any grouping of stem cells of like specified characteristics. The grouping could be according to, e.g., previous exposure to a stress condition or lack thereof.

A "dataset" as used herein is a set of numerical values resulting from evaluation of a sample (or population of samples) under a desired condition. The values of the dataset can be obtained, e.g., by experimentally obtaining measures from a sample and constructing a dataset from these measurements. As is understood by one of ordinary skill in the art, the reference level can be based on e.g., any mathematical or statistical formula useful in the art for arriving at a meaningful aggregate reference level from a collection of individual datapoints; e.g., mean, median, median of the mean, etc. Alternatively, a reference level or dataset to create a reference level can be obtained from a service provider such as a laboratory, or from a database or a server on which the dataset has been stored.

In CE, conclusions are drawn based on whether a measure is statistically significantly different or not statistically significantly different from a reference level. A measure is not statistically significantly different if the difference is within a level that would be expected to occur based on chance alone. In contrast, a statistically significant difference or increase is one that is greater than what would be expected to occur by chance alone. Statistical significance or lack thereof can be determined by any of various S/M used in the art. An example of a commonly used measure of statistical significance is the p-value. The p-value represents the probability of obtaining a given result equivalent to a particular datapoint, where the datapoint is the result of random chance alone. A result is often considered significant (not random chance) at a p-value less than or equal to 0.05.

Embodiments can be used with high throughput screening (HTS). In HTS, numerous different compounds can be tested for potential stress effects quickly. For example, stem cells (e.g., modified stem cells) can be provided to wells of a microtiter plate, including, e.g., a 96- or 384-well plate that can be read in a fluorescent microplate reader. The stem cell lines in the wells can then be exposed to different compounds and/or different concentrations of compounds. In additional embodiments, stem cells can be contacted with a single test agent, or, to facilitate HTS, to at least 2, at least 5, at least 10, at least 100, at least 1,000, at least 10,000, at least 100,000 or at least at least 1,000,000 test compounds at a time. Environmental conditions can similarly be screened, and test compounds and environmental conditions can be screened in various combinations. If the stem cells score positive for stress, a different set of stem cells can be subsequently tested with a subset of the test compounds or environmental conditions until the compounds or conditions creating the stress effect are identified.

High-throughput screening systems are commercially available from, e.g., Zymark Corp., Hopkinton, Mass.; Air Technical Industries, Mentor, Ohio; Beckman Instruments, Inc. Fullerton, Calif.; Precision Systems, Inc., Natick, Mass., Luminex Corp., etc.. These systems typically automate entire procedures including all sample and reagent pipetting, liquid dispensing, timed incubations, and final readings of the microplate in detector(s) appropriate for the assay. These configurable systems provide high throughput and rapid start up as well as a high degree of flexibility and customization. The manufacturers of such systems provide detailed protocols for the various S/M of high throughput.

Kits to practice the S/M are also provided (which are not part of the invention). Kits can include one or more containers including modified stem cells, primer pairs, probe sequences, binding proteins (e.g., antibodies) and/or reagents or supplies to assess expression of a gene of interest described herein. Components of the kit can be prepared for storage and later use. Associated with such container(s) can be a notice in the form prescribed by a governmental agency regulating the manufacture, use, or sale of the kit, which notice reflects approval by the agency of manufacture, use, or sale, when required.

Optionally, the kits further include instructions for using the kit in the methods. In various embodiments, the instructions can include appropriate reference levels to interpret results associated with using the kit; proper disposal of the related waste; and the like. The instructions can be in the form of printed instructions provided within the kit or the instructions can be printed on a portion of the kit itself. Instructions may be in the form of a sheet, pamphlet, brochure, CD-Rom, or computer-readable device, or can provide directions to instructions at a remote location, such as a website.

The Examples below describe the optimization of the methods. These Examples are included to demonstrate CE of the disclosure..

### Examples.

Example 1. As an embryo implants into the uterus, stress can diminish placental TSCs proliferation inducing differentiation to create more essential differentiated product/cells. Xie, 2014, Xie, Int. Rev. Cell. Mol. Biol., 287:43, 2011 (Xie, 2011); Awonuga Mol. Reprod. Dev., 78:519, 2011 (Awonuga, 2011); Zhong, Reproduction, 140:921, 2010 (Zhong, 2010). This "compensatory" differentiation thus further depletes the size of the population of multipotent TSC. Most human embryos are lost before birth and the greatest loss occurs soon after implantation. Cross, Science, 266:1508, 1994. Understanding the cellular and molecular mechanisms of TSC stress responses informs strategies to ameliorate their consequences during implantation.

Implantation site O₂ is normally 2%, when O₂ levels are optimized, highest growth and least stress (activation of stress-activated protein kinase/SAPK) is at 2% O₂. Zhou, 2011. Also, dose responses of SAPK activitation in response to TNFalpha, dioxin suggest that these stressors could activate compensatory and prioritized differentiation in TSC (Xie, 2008, Zhong, Reprod. Sci., 14:534, 2007 (Zhong, 2007)). Cigarette smoke condensate and IFNgamma also may activate compensatory differentiation through Rex1 loss in mouse ESCs.

Although stress at 20% is greater than at 2% O₂, only O₂<2% activates high levels of SAPK that are comparable to other stresses at 20% O₂, describing a U-shaped curve for stress that is proportional to levels of SAPK and inversely proportional to growth. Hypoxic stress for stem cells at 0.5% O₂ combined with FGF4 is maximal for priming TSC for differentiation by producing the lowest levels of three potency factors (Inhibitor of differentiation (ld)2 Caudal related homeobox Cdx(2), Estrogen receptor related, (Err)beta) and highest levels of three nuclear factors (heart and neural differentiation Hand(1), glial cells missing Gcm(1), and trophoblast specific binding protein Tpbp(a) that mediate TSC differentiation.

Fibroblast growth factor (FGF)4 is synthesized in the implanting embryo (Xie 2011) and is necessary for maintaining TSC proliferation and potency in culture (Tanaka, 1998) and in the embryo (Chai, 1998). To better emulate preimplantation stress effects, TSC culture was performed in this Example for 24 hr in the presence of FGF4. Furthermore, to study the longer period of postimplantation development, culture was extended to 7 days with FGF4 removed to stimulate normal differentiation.

During normal postimplantation placental development, the 1^{st} lineage differentiating from TSC is parietal trophoblast giant cells (pTGC) at E4.5 (4.5 days after fertilization), followed by spongiotrophoblasts at E7.0 and labyrinthine, chorionic syncytiotrophoblasts (synT) and sinusoidal TGC (sTGC) at E7.5. Simmons, BMC Genomics, 9:352, 2008; Simmons, Dev. Biol., 304:567, 2007. SAPK is needed to mediate stress-induced suppression of placental cell cycle and TSC differentiation to the earliest lineage, pTGC, in placental cells. Xie, Mol. Hum Reprod., 13:473, 2007 (Xie, 2007); Awonuga, 2011. Without being bound by theory, it is believed that stress "prioritizes" differentiation as hyperosmolar stress induces TSCs to differentiate into the earliest postimplantation lineage/marker (e.g., pTGC/Hand1) but not later lineages/markers (e.g., synTA/Gcm1; Xie, 2011, supra). Because SAPK mediates hyperosmolar stress-induced Hand1 and Hand1-dependent PL1 (Awonuga, 2011), whether stress-induced lineage choice during hypoxia was SAPK-dependent was tested. Because hyperosmolar stress induces the 1^{st} lineage but not later lineages from TSC (Liu, Placenta, 30:66, 2009), and SAPK mediates the induction of 1^{st} lineage markers (Awonuga, 2011), it was hypothesized that SAPK will mediate induction of the 1^{st} lineage/marker (pTGC/Hand1) and suppression of later lineage/marker (synTA/Gcm1) induced by hypoxic stress.

Here the effects on potency and lineage differentiation in cultured TSC of FGF4 removal during TSC culture with O₂ levels at 20, 2, 0.5, and 0% was tested. It was found that FGF4 maintains Warburg metabolism (suppression of mitochondrial electron transport chain and activation of aerobic glycolysis) at all O₂ levels, but during hypoxic stress at 0.5% O₂ differentiation was induced by O₂ levels that were insufficient to support increased mitochondrial activity or suppress aerobic glycolysis. This led to a rapid but incomplete differentiation and lineage imbalance as two lineages at the maternal interface of the later-developing chorionic placenta are largely suppressed at all O₂ levels <20%. Failure of these lineages is associated with spontaneous miscarriage, preeclampsia, intrauterine growth retardation, and preterm labor.

Materials and Methods. Materials. Reagents for maintaining TSC, delivering (O₂)s and for SAPK and actin antibodies and SAPK inhibitors were described previously (Awonuga, 2011; Zhou, 2011). The primary antibodies for pyruvate kinase embryonic form (PKM)2 (CS3198), PKM2 Tyr105 (CS3827) were from Cell signaling (Danvers MA), and pyruvate kinase adult form (PKM)1 antibody (AP7476b) was purchased from Abgent (San Diego, CA). Cyto-oxygenase (COX) subunit I antibody (MS404) was purchased from MitoSciences (Eugene, OR). The mitochondrial membrane potential (ΔΨₘ) stains tetramethylrhodamine methyl ester (TMRM), MitoTracker red, and 5,5',6,6'-tetrachloro-1,1',3,3'-Tetra-ethylbenzimidazolylcarbo-cyanine iodide (JC1) were purchased from Molecular Probes (Grand Island, NY). Mitochondrial inhibitors antimycin A and cyanide, the mitochondrial activator dichloroacetate (DCA), and the OxPhos uncoupler Carbonyl cyanide 4-(trifluoromethoxy)phenylhydrazone (FCCP) were purchased from Sigma (St. Louis, MO). FGF4 and heparin were from Sigma Chemical Co. (St. Louis, MO). Fetal bovine serum and RPMI1640 were from Gibco (Grand Island, NY). SAPK inhibitor SP600125 and LJNKI1 SAPK inhibitor were purchased from Calbiochem-EMD (Emanuel Merck, Darmstadt)(San Diego, CA). Anaerobic bags to create 0% O₂ were purchased from Hardy Diagnostics (AN010C, Santa Maria, Ca).

Cell lines and culture conditions. The mouse TSC sources and culture conditions were described previously. Awonuga, 2011; Zhou, 2011. Briefly, mouse TSC isolated from E3.5 and E6.5 conceptuses were from Dr. Rossant (Lunenfeld Research Institute, Ontario, Canada) and cultured. Cells were cultured with premixed gas at 0.5%, 2% (O₂/CO₂/N₂ levels of 0.5/4.5/95, and 2/5/93, respectively, Praxair, Warren, MI), and 20% O₂ levels were obtained by mixing 5% CO₂ from a 100% CO₂ gas tank with ambient N₂ and O₂ levels. Media were pre-equilibrated under O₂ levels for TSC culture overnight prior to culture. Quantitative removal of gas phase O₂ requires 30 min in anaerobic bags, i.e., there was only a minimal period when 20% O₂ was ambient to media that was pre-equilibrated under 0, 0.5, or 2% O₂. Since 12-24 hr is required to equilibrate gas phase to complex media/liquid phase, there was no appreciable exposure of TS cells to 20% O₂ again after the switch to 0, 0.5, or 2% O₂.

RNA isolation and quantitative reverse transcription-PCR (qRT-PCR). All steps in RNA isolation, cDNA synthesis and qPCR performance and analysis were described previously (Zhou, 2011). Briefly, DNase-treated total RNA was isolated following cDNA preparation using QuantiTect Reverse Transcription Kit iScript (Qiagen). Assays were performed using Fast SYBR Green on a System 7500 instrument (Applied Biosystems), and a one-step program for 40 cycles. Each experiment was carried out in triplicate, and all three genes for TSC multipotency were normalized against 18S rRNA.

Primers used are shown in the following Table 1:

| Table 1. List of primers used in qPCR assays | | |
|---|---|---|
| Gene abbreviati on | Gene Name | Primer sequence |
| TSC Multipotency Markers | | |
| Errβ | Estrogen-related receptor-β | F 5' GGGAGCTTGTGTTCCTCATC 3' (SEQ ID NO:96) R 5' CTACCAGGCGAGAGTGTTCC 3' (SEQ ID NO:97) |
| Cdx2 | Caudal type homeobox-2 | F 5' CCAGCTCTTTGCCTCTCTGT 3' (SEQ ID NO:98) R 5' TGCCTCTGGCTCCTGTAGTT 3' (SEQ ID NO:99) |
| Id2 | DNA-binding protein inhibitor2 | F 5' TCAGCCTGCATCACCAGAGA 3' (SEQ ID NO:100) R 5'CTGCAAGGACAGGATGCTGAT 3' (SEQ ID NO:101) |
| Transcription Factors (Hand1, Tfeb, Gcm1) and other marker (Tpbpa) | | |
| HAND1 | Heart- and neural crest derivatives expressed protein 1 | F 5' GGATGCACAAGCAGGTGAC3' (SEQ ID NO:102) R 5'CACTGGTTTAGCTCCAGCG3' (SEQ ID NO:103) |
| Tpbpa (4311) | Trophoblast specific protein alpha | F 5' CGGAAGGCTCCAACATAGAA 3' (SEQ ID NO: 104) R 5' TCAAATTCAGGGTCATCAACAA 3' (SEQ ID NO:105) |
| Tfeb | Transcription factor EB | F 5' AACAAAGGCACCATCCTCAA 3' (SEQ ID NO:106) R 5' CAGCTCGGCCATATTCACAC 3' (SEQ ID NO:107) |
| Gcm1 | Glial cells missing-1 | F 5' AACACCAACAACCACAACTCC 3' (SEQ ID NO: 108) R 5' CAGCTTTTCCTCTGCTGCTT 3' (SEQ ID NO:109) |
| Terminal Differentiation Markers for Giant Cell | | |
| PI-I | Placental lactogen-1 | F 5' TGGTGTCAAGCCTACTCCTTT 3' (SEQ ID NO:110) R 5' CAGGGGAAGTGTTCTGTCTGT 3' (SEQ ID NO:111) |
| PI-II | Placental lactogen-2 | F 5' CCAACGTGTGATTGTGGTGT 3' (SEQ ID NO: 112) R 5' TCTTCCGATGTTGTCTGGTG 3' (SEQ ID NO:113) |
| Plf | Proliferin | F 5' TGAGGAATGGTCGTTGCTTT 3' (SEQ ID NO:114) R 5' TCTCATGGGGCTTTTGTCTC 3' (SEQ ID NO:115) |
| Ctsq | Cathepsin Q | F 5' AACTAAAGGCCCCATTGCTAC 3' (SEQ ID NO:116) R 5' CAATCCCCATCGTCTACCC 3' (SEQ ID NO:117) |
| Terminal Differentiation Markers for Syncytiotrophoblast | | |
| synA | Syncytin-A | F 5' CCCTTGTTCCTCTGCCTACTC 3' (SEQ ID NO:118) R 5' TCATGGGTGTCTCTGTCCAA 3' (SEQ ID NO:119) |
| Loading Control | | |
| 18S | 18S ribosomal subunit | F 5' CGCGGTTCTATTTTGTTGGT 3' (SEQ ID NO:120) R 5' AACCTCCGACTTTCGTTCTTG 3' (SEQ ID NO:121) |

Mitochondrial staining by MitoTracker dye. TSC were cultured at different O₂ levels at least 24 hr stained with DAPI (D21490, Invitrogen) alone or with either MitoTracker Red FM (Invitrogen), TMRM (T668), or JC-1 (T3168, Invitrogen) alone or with the uncoupler FCCP (Sigma), and analyzed under a DM-IRE2 fluorescence microscope (Leica, Germany) using Simple PCI image acquisition software (Hamamatsu, Sewickley, PA).

In more detail, TSC were cultured on coverslips in 12-well plates at different O₂ levels either with or without FGF4, for 48 hr. Before experiments were conducted at low O₂ tension, TSC were adapted in a hypoxia chamber (Billups-Rothenberg, CA) at the given O₂ level described above for at least 24 hr. Nuclear dye 25µg/ml DAPI (D21490, Invitrogen) was applied as a counterstain 1 hr before TSC were incubated alone with either 50nM MitoTracker Red FM (M22425m, Invitrogen) for 30 min, 20nM TMRM (T668) for 30 min, or 3.25nM JC-1 (T3168, Invitrogen) for 45 min, or in combination with the uncoupler FCCP (1nM, Sigma, C2920). After staining, the TSC were washed with PBS 2 times and immediately analyzed under a DM-IRE2 fluorescence microscope (Leica, Germany) using Simple PCI image acquisition software (Hamamatsu, Sewickley, PA).

Western analysis. TSC were lysed, protein assayed, separated by PAGE-SDS, immunoblotted and films exposed and analyzed as done previously. Zhou, 2011. In more detail, cytochrome c oxidase (COX) levels were determined by Western analysis. Proteins were extracted with lysis buffer (Cell signaling, #9803). Forty µg protein was loaded per well of precast gels (Bio-rad, 161-1101). Proteins were transferred onto nitro-cellulose membranes and then incubated with anti-mouse monoclonal COX subunit I antibody (1:500; MitoSciences, MS404) overnight. After incubation with secondary antibody, signal was detected by chemiluminescence using ECL Advance (RPN2135, GE Healthcare Biosciences, Pittsburgh PA).

**ATP** measurements. TSC were washed, collected, centrifuged, frozen on dry ice, and stored. Cell pellets were boiled, lysed, iced, and solubilized by ultrasonication. (ATP)s were determined using the ATP bioluminescence assay kit HS II (Roche Applied Science) by the manufacturer's protocol. Data were standardized to the protein concentration. In more detail, TSC were washed with PBS once, collected by scraping, centrifuged at 10,000 rpm for 30 sec and then PBS was quickly removed. Cells were immediately frozen on dry ice and stored at - 80°C. Cell pellets were supplemented with 300 µl boiling buffer (100 mM Tris, pH 7.75, 4 mM EDTA) and immediately transferred to a boiling water bath, lysed for 2 min, put on ice, and solubilized by ultrasonication. ATP concentrations were determined using the ATP bioluminescence assay kit HS II (Roche Applied Science) according to the manufacturer's protocol. Data were standardized to the protein concentration.

ROS measurements at 20% O₂. TSC were trypsinized, centrifuged, washed, and incubated with the ROS-sensitive probe 2' ,7' -dichlorodihydrofluorescein diacetate (Molecular Probes), and analyzed on an Ascent Fluoroskan plate reader. Experiments were performed in triplicate and data standardized to cell number. As a control, cells were incubated in the presence of 100 µM H₂O₂.

In more detail, after differentiation at 20% O₂, TSC were trypsinized, collected by centrifugation, washed, and incubated with the ROS-sensitive probe 2' ,7' - dichlorodihydrofluorescein diacetate (H₂DCFDA; D-399, Molecular Probes) at a final concentration of 7.5 µM for 20 min at 37°C in the dark. Cells were collected by centrifugation, supplemented with media, and incubated in the dark for 20 min at 37°C. As a control, cells were incubated in the presence of 100 µM H₂O₂. Cells were collected by centrifugation, resuspended in PBS, transferred to a 96-well plate (Costar 3603; 200 µL per well), and analyzed on an Ascent Fluoroskan plate reader (488 nm excitation; 527 nm emission). Experiments were performed in triplicate and data standardized to cell number counted with a hemocytometer. Table 2. Estimation of changes in PKM2 and COX subunit 1 activity caused by removal of FGF4 at 20% or 0.5% O₂.

Dual regulation of rate-limiting steps in ETC and glycolysis shown by removing FGF4 at 20% and 0.5% O₂:

| | | | | | | |
|---|---|---|---|---|---|---|
| day 2 | 20% | | 0.5% | | | |
| | 20% anabolic | | ETC | 0.5% | | |
| | | | activity | | | carbon |
| usage | | | | | | |
| Total COX | FGF4+ 1 | FGF4-2.3 | delta +2.3x | FGF4+ 0.4 | FGF4-0.4 | delta 1 |
| pCOX total activity delta | 1 | 0.4 | +2.5x | 0.85 | 0.25 | +3.4 |
| | +3.4 x 0.4=+1.36 | | +5.8 | x2.3=+13.34 | | |
| Total PKM2 | 1 | 1 | 1 | 3.3 | 4.7 | +1.4 |
| pPKM2 | 1 | 0.35 | -3x | 0.9 | 0.3 | -3x |
| total activity delta | | | -3x x 1=-3x | | | |
| | | | -4.2 x 4.7=-19.74 | | | |
| Values from Figure 7 ETC activity is a product of changes with FGF4 removal of total protein (dependent on O₂ levels) multiplied by the change in posttranslational regulation (dependent on FGF4) and this product is multiplied by the total protein levels after FGF4 removal that is acted upon. | | | | | | |

Statistical analysis. The data in this Example are representative of three independent experiments and indicated as mean ± s.e.m.. Data were analyzed with SPSS v. 19.0 using the Kruskal-Wallis non-parametric ANOVA for not normally distributed values, and if significant, the pairs of treatments were tested using the Mann-Whitney U-test with a Bonferroni correction for multiple comparisons. Groups were considered to be significantly different if p<0.05.

Results. SAPK increases the 1^{st} lineage and decreases later lineages proportional to the deviation from the optimal O₂ at 2%. Two SAPK inhibitors with different mechanisms, SP600125 and LJNKI1, were used to limit possible off-target effects. Both SAPK inhibitors prompted a 3-7-fold increase in the later chorionic lineage (Gcm1), with the highest inhibition occurring at 0.5% and 20% O₂ (FIGs. 1A and 2). Both SAPK inhibitors also increased later spongiotrophoblast lineage (Tpbpa) at all O₂ levels. In contrast, both inhibitors suppressed early lineage pTGC (Hand1) mRNA (FIGs. 1A and 2) with suppression being highest at 0.5% and 20% O₂. Furthermore, both SAPK inhibitors suppressed later lineages (Tpbpa and Gcm1) and increased the earliest lineage (Hand1, FIG. 1B and FIG. 2, 3A and 3B). The smallest effects on Hand1, Tpbpa, and Gcm1 occurred at 2% O₂, but as O₂ levels deviated from 2% the mRNA levels are influenced by SAPK level upward for Hand1 and downward for Gcm1 (FIG. 1B).

The mRNA levels for 3 multipotency factors (Cdx2, Id2, and Errβ) were unaffected by 24 hr of O₂ at 2-20% but were greatly reduced at 0.5% O₂, despite FGF4. Neither SAPK inhibitor affected multipotency factors maintenance at 2-20% O₂, or the loss of the 3 multipotency factors at 0.5% O₂ (FIG. 1C). Hence during hypoxic stress, SAPK mediated prioritized differentiation (FIGs. 4A-4D), but not multipotency loss.

Loss of potency and gain of differentiation are O₂-dependent, and hypoxia suppresses labyrinthine placenta. It was 1^{st} tested whether the most stressful 0.5% O₂ level induces most loss of potency factor transcripts and increased differentiation after FGF4 removal. It was found that following FGF4 removal, the caudal homeobox related (Cdx2), Inhibitor of Differentiation (ld)2, and estrogen receptor related (Err)β - potency-mediating mRNA - were the most rapidly lost by 24 hr at 0-0.5% O₂ (FIG. 4A, 2). In contrast by day 7 0.5% O₂ sustained the highest potency factor mRNA levels while the lowest average level of the potency factors were observed at 20% O₂.

At 24 hr, 2 markers for the 1^{st} lineage pTGC showed stress-induced differentiation (PL1, Plf, FIG. 4B and 4C, FIG. 3A and 3B). First lineage markers PL1 and PIf were 2-3-fold higher at 0-0.5% than at 20% O₂ after 24 hr, Two later lineages found at the placental surface, sTGC (PL2+CtsQ) and syncytiotrophoblast A cells (synTA) were not increased (FIG. 4C). Transcription factors supporting 1^{st} and secondary pTGC, Hand1 and Tpbpa were upregulated in the first 24 hr at O₂<2% (FIG. 3A and 3B). Thus the early response to hypoxic stress <2% O₂ is to differentiate to the 1^{st}, but not later lineages. A marker of 1^{st} lineage (PL1) was induced by O₂<2%, but differentiation did not increase greatly at low O₂ and by day 7 PL1 was most highly expressed at 20% O₂ (FIG. 4D, FIG. 5). In contrast a marker of a later lineage (synTa) was not disproportionally upregulated by O₂<2% and was always most highly expressed at 20% O₂.

Through day 4, the average differentiation increased threefold at all O₂ levels over day 0. However, by day 7, while 0-0.5% O₂ maintained this threefold increase, increases of 10-fold and 25-fold were observed for 2% and 20% O₂, respectively. First lineage markers PL1 and PIf were much more highly induced at 2-20% than at <2% O₂ but by day 7 all five differentiation markers were highest at 20% O₂ (FIG. 5). Thus, after a fast, early increase in differentiation of especially the 1^{st} lineage, differentiation stalled at hypoxic 0-0.5% O₂ but increased greatly at 2-20% O₂.

Proliferin (Plf), placental lactogen (PL)1, CtsQ, and synTa had a maximal increase of 7-10 fold, and 1 marker (placental lactogen, (PL)2) had a 70-fold increase by day 7 at 20% O₂ (FIG. 4B,C). The two placental surface lineages, sTGC and synTA cells appear least supported by O₂ at <20%. Markers of these two lineages were suppressed by hypoxic stress at 0.5% O₂ to 10% of levels at 20% O₂ (FIG. 4C).

Hyperosmolar stress that induces differentiation at 20% O₂ cannot induce differentiation at hypoxic 0.5% O₂. O₂ at 0.5% does not support differentiation but also does not activate as much SAPK as 100 mM sorbitol at >20% O₂⁶. Thus, 100 mM sorbitol, which increases SAPK 5 fold and induces differentiation at 20% O₂ despite FGF4 was added. Awonuga, 2011; Zhong, 2010; Liu, supra; Zhong, 2007. However, the added stress did not increase mRNA expression of any of the 3 terminal differentiation markers (PL1, Plf, and synTa) during hypoxia (FIG. 6A). Thus, 0.5% O₂ cannot support differentiation after FGF4 removal or sorbitol addition.

Multipotent TSCs that differentiated for 2 days after FGF4 removed showed increased mitochondrial membrane potentials (Δ_{Ψm}). The roles of FGF4 and O₂ on Δ_{Ψm} associated with ATP production was determined. TSCs were incubated for 2 days with or without FGF4 and stained with the Δ_{Ψm}-sensitive probes MitoTracker (FIG. 7A-7D), JC1, or TMRM in the presence or absence of the Δ_{Ψm} uncoupler FCCP (FIG. 8A-8F). At 2 or 20% O₂, FGF4 maintained mitochondria with low Δ_{Ψm}. Some cells had high Δ_{Ψm} at epithelial edges despite FGF4. Similar to the MitoTracker stain findings (FIG. 7A-7D), both JC1 and TMRM stains showed increased Δ_{Ψm} levels only after FGF4 removal (FIG. 8A-8F). FCCP completely depolarizes the A_{Ψm}, showing the specificity of the Δ_{Ψm} effects. Thus, FGF4 maintains mitochondrial inactivity in TSCs at all O₂ levels and O₂ does not play a role in mitochondrial initiation to full activity.

FGF4 and hypoxia co-regulate low Δ_{Ψm}, but Δ_{Ψm} does not increase with FGF4 removal during hypoxia after 7 days. The JC1 stain was used to assay Δ_{Ψm} and to test whether hypoxia limited Δ_{Ψm} generation when differentiation was maximal after FGF4 removal for 7 days (as in FIG. 4A-4D). There were 4 levels of JC1 stain color and intensity. FCCP produced no fluorescence, indicating complete Δ_{Ψm} depolarization (FIG. 6B). TSCs cultured with FGF4 at 0.5% O₂ showed a low level of green fluorescence, indicating low Δ_{Ψm}, but TSCs cultured at 2% or 20% O₂ with FGF4 removed showed two levels of increasing red fluorescence, indicating higher Δ_{Ψm}. FGF4 and hypoxia play additive, regulatory roles for Δ_{Ψm} but even with FGF4 removed, 0.5% O₂ could not sustain high Δ_{Ψm} levels.

Hypoxia induced morphological changes. At 0.5% O₂, stress significantly increases nuclear size despite FGF4 and nearly all TSCs with small nuclei were lost and all differentiated cells were TGC (FIG. 6B, 6C). Hypoxic stress-induced giant cells in (C) with FGF4 were the same size as those induced by FGF4 removal at 2% or 0.5% O₂ (E,F), but 20% O₂ sustained the largest giant cells (D). This finding is similar to hyperosmolar stress at 20% O₂, where all cells lost Id2 and expressed the TGC marker PL1. Awonuga, 2011; Zhong, 140:921-930, 2010. Multinuclear synTA cells and giant cells were visible at 20% O₂ with FGF4 removed, but synTA cells were not observed or extremely rare at 0.5% O₂. Although hypoxic stress initiates differentiation to TGC morphologically while suppressing syncytiotrophoblasts, terminal differentiation does not complete at 7 days (FIG. 4B) and size of TGC is lower at 0.5%<20% O₂. Thus, FGF4 is most important in suppressing Δ_{Ψm} when O₂ is ≥2%, but O₂≤0.5% limits Δ_{Ψm} generation, and morphological differentiation of TSCs is limited at 0-0.5% O₂.

Cytochrome c oxidase (COX) and pyruvate kinase embryonic form M2 (PKM2) are co-regulated by FGF4 and hypoxia, and their total protein levels do not change during hypoxia despite FGF4 removal. Without being bound by theory, a likely mechanism for decreased differentiation at 0.5% O₂ is insufficient mitochondrial OxPhos, which normally consumes >90% of cellular O₂. COX is the terminal, proposed rate-limiting enzyme of the electron transport chain (ETC). Villani, J. Biol. Chem., 273:31829, 1998; Pacelli, Mitochondrion, 11:334, 2011. COX, with other ETC complexes, generates the Δ_{Ψm} which is required for increased ATP synthesis, but also increased mitochondrial ROS. To biochemically corroborate the findings regarding Δ_{Ψm} inactivity, the amount of COX protein and its inactive Tyr304-phosphorylated form was assayed. Lee, J. Biol. Chem., 280:6094, 2005; Samavati, J. Biol. Chem., 283:21134, 2008.

At all O₂ levels COX is maintained at highest, phosphorylated (inactivated) state by FGF4 (FIG. 9A), corresponding to low Δ_{Ψm} in FIG. 6B, Total COX is low at O₂≤2% levels, and highest at 20% O₂ when FGF4 is removed (FIG. 9B). Significantly, total COX levels do not increase with FGF4 removal at 0.5% O₂.

Highly proliferative stem cells require high anabolic rates that require aerobic glycolysis. Aerobic glycolysis, as is probable when FGF4 is present in (FIG. 6B), is mediated by pyruvate kinase embryonic form PKM2. Christofk, Nature, 452:181, 2008; Hitosugi, Sci. Signal, 2:ra73, 2009. PKM2 mediates the irreversible terminal step of glycolysis. In its Tyr105 form, however, PKM2 does not homotetramerize, which is necessary to bind phospho-enol pyruvate and catalyze pyruvate production. PKM2 is also a nuclear cofactor for genes involved in glycolysis and glucose uptake. Similar to COX phosphorylation, FGF4 maintained phosphorylation of PKM2 on Tyr105 at all O₂ concentrations (compare FIG. 9A and 9C). However, for PKM2, O₂≤ 2% without FGF4 resulted in the highest protein levels, with less PKM2 protein at O₂>2% (FIG. 9E). At 20% O₂, PKM2 protein decreased after FGF4 removal. The adult splice variant, PKM1, was higher with 2-20% O₂, but there was no coherent pattern of regulation by FGF4 or O₂ (FIG. 9D). Despite FGF4 removal, hypoxic O₂ at 0.5% O₂ maintains high glycolysis by maintaining high PKM2 protein levels.

Mitochondrial antagonists and an agonist show that mitochondrial function is necessary and sufficient to mediate the differentiation of two labyrinthine placental lineages. To test the necessity and sufficiency of mitochondrial function for TSC differentiation, 2 mitochondrial inhibitors and an agonist were used. The mitochondrial activator dichloroacetate (DCA) was tested at 0.5%-20% O₂ (FIG. 10A). There were no effects at 0.5% or 20% O₂. But at 2% O₂, of 5 markers only 2 markers increased 2-fold with DCA treatment; PL2 and synTa. These are markers of sTGC and synTA cells of the labyrinthine placenta, although PL2 is detected in other cell types. The other 3 markers were equally highly expressed at 20% and 2% O₂, suggesting maximal differentiation at 2% O₂ that could not be increased. Thus, DCA increased the 2 lineages that could be increased at 2% O₂. In contrast 0.5% O₂ is likely to be insufficient to support increases in differentiation induced by DCA.

Cyanide and antimycin A inhibit COX (part of mitochondrial complex IV) and complex III, respectively, and were used to inhibit the ETC and thus the generation of Δ_{Ψm} for 7 days of culture at optimal differentiation conditions (FIG. 10B). Sublethal concentrations of inhibitors were used that had significant effects on mitochondria. For all 5 differentiation markers tested, both mitochondrial inhibitors decreased mRNA levels. Markers emphasized (PL2; inhibited 98% and 56%) or specific (Ctsq; inhibited 81% and 71%) for labyrinthine sTGCs were decreased greatly by antimycin and cyanide respectively (FIG. 10B). A marker specific for labyrinthine synTA cells (synTa; inhibited 42% and 34%) was inhibited by antimycin and cyanide respectively (FIG. 10B). Thus, mitochondrial antagonists block differentiation of 2 lineages of the labyrinthine placenta under conditions mediating maximal differentiation, whereas a mitochondrial agonist increased those lineages at 2% O₂.

ATP decreases in an O₂-dependent manner as TSCs differentiate, but highest ATP accumulation occurs where hypoxic differentiation is lowest. To understand energy use during O₂-dependent differentiation, TSCs were cultured for 1-7 days without FGF4, and ATP levels were determined on days 1, 2, 4, and 7, the same as those used to test differentiation (FIG. 4B,4C). At 0%, 2%, and 20% O₂, ATP continuously decreased from days 1-7, with the lowest ATP in TSCs cultured at 2% or 20% O₂ at day 7 (FIG. 11). Thus, energy levels were lowest when differentiation was highest.

In contrast, in TSCs cultured at 0.5% O₂ ATP increased from days 1-7. The greatest decrease in ATP at 2-20% O₂ occurred between days 4 and 7, when differentiation increased most (FIG. 4B,4C) but cells were already confluent (data not shown) and proliferation does not consume ATP. Highest ATP consumption correlates with highest differentiation at 20% O₂, but surprisingly during hypoxic stress when differentiation is lowest ATP accumulates. Thus ATP is not the only factor that limits differentiation during hypoxia.

FGF4 suppresses ROS generation by TSCs. FGF4 regulates mitochondrial function through COX1 and PKM2 phosphorylation, which reduces ETC by preventing carbon entry into mitochondria. Mitochondria produce ROS, which can endanger DNA and are suppressed in stem cells. Simon and Keith, Nat. Rev. Mol. Cell Biol., 9:285, 2008. TSCs produce twice as much ROS (H₂O₂) 48 hr after FGF4 removal (FIG. 12). Thus FGF4 maintains low ROS.

Discussion. Since the seminal work from the Fisher lab, O₂ effects during imbalanced or blocked placental stem cell differentiation have focused on the insufficient "hypoxia" at 2% O₂ (Genbacev, Science, 277:1669, 1997) which does not support differentiation. Those findings are corroborated and extended here; 2% O₂ provides for insufficient placental differentiation for almost all lineages. Specifically 2% O₂ is insufficient for the full development of two lineages at the surface of the labyrinthine placenta; gas exchange-mediating synTA cells and secretory sTGCs. But hypoxia also does not support continuing increase in PL1 from days 4-7 after an early disproportionate peak at day 1.

However, it is emphasized that "hypoxic" stress for stem cells is at 0-0.5% O₂ where these lower levels fail to support differentiation but also cause differentiation, particularly of the 1^{st} lineage. Hypoxic stress creates an approximation of the higher stress levels (SAPK activation) for TSC caused by sorbitol or benzopyrene at 20% O₂. Awonuga, 2011; Zhong, 2010; Xie, Mol. Reprod. Dev., 77:533, 2010 (Xie, 2010). Like other stresses at 20% O₂, hypoxic stress at 0.5% O₂ induces potency factor loss and gain of differentiation factors. Specifically, hypoxia-induced Hand1 mediates differentiation of the 1^{st} lineage, pTGC. Rapid loss of potency factors and gain of differentiation factors are induced dominantly by stress with or without FGF4. However, 20% O₂ provides for an exit from aerobic glycolysis and an increase in mitochondrial membrane charge (Δ_{Ψm}) that supports the most energy-requiring lineages from 4-7 days after FGF4 removal. The average differentiation at all O₂ levels is equivalent at 4 days after FGF4 removal. But, differentiation increases to much higher levels only at 2-20% O₂ where free ATP levels approach zero as highest differentiation is reached at day 7. The highest level of Δ_{Ψm}, highest level of differentiation, and lowest ATP suggests that the final increase in differentiation requires the levels of energy supplied by efficient mitochondrial OxPhos, not aerobic glycolysis. It has been suggested that proliferation requires aerobic glycolysis to preserve carbon for proliferation, but cells are confluent at 2-20% O₂ by day 4. Lack of cell death or proliferation at day 7 suggests nearly all ATP would support differentiation and that ATP production limits differentiation.

It is only at 20% O₂ that total COX protein increases and total PKM2 protein decreases. It is only after FGF4 removal that tyrosine phosphorylation of COX and PKM2 proteins decreases. This loss of phosphorylation of COX and PKM2 leads to increased Δ_{Ψm} and ATP production, and decreased aerobic glycolysis, respectively. Thus 0.5% O₂ cannot support the differentiation initiated by hypoxic stress or added hyperosmolar stress that induces robust differentiation of TSC at 20% O₂. A key problem during the hypoxic stress at 0-0.5% O₂ is that it induces TSC differentiation in the 1^{st} 24-48 hr increase, marked by increases in cell size and PL1 and Plf levels (early lineage markers), but these markers are less than at 20% O₂ between days 4-7 due to mitochondrial insufficiency. The tendency is that 1^{st} lineage markers, which are favored early with hypoxic stress here and by all stresses at 20% O₂ as reported here and previously (Awonuga, 2011; Rappolee, Syst. Biol. Reprod. Med., 58:33, 2012), have a low "ceiling" of differentiation at 0.5% O₂ after 7 days with or without sorbitol. This suggests that energy or another mitochondrial function is rate-limiting.

Several lines of evidence suggest that mitochondrial insufficiency and failure to leave glycolysis (Warburg metabolism) are the fundamental limits to normal differentiation after FGF4 removal. These lines of evidence also pinpoint the two lineages at the labyrinthine surface as the highest energy- and mitochondrial function-requiring lineages. First although FGF4 is dominant over oxygen at all oxygen levels in maintaining phosphorylation of COX subunit 1 and PKM2, oxygen is dominant over FGF4 at low oxygen levels where total COX cannot increase and PKM2 cannot decrease with FGF4 removal. This suggests that mitochondrial ETC cannot increase at low O₂ and the inefficiency of glycolytic production of ATP cannot change at low O₂, even after FGF4 is removed and differentiation can begin. Also, both mitochondrial inhibitors decrease all differentiated lineages but the largest effects are on the labyrinthine surface lineages. But the highest inhibition occurs in the order 20>2>>0.5% O₂ after FGF4 removal, suggesting that mitochondrial function is required only at levels of O₂ that support full development of Δ_{Ψm} and OxPhos. In addition the mitochondrial agonist has no effects at 20% or 0.5% O₂. At 20% O₂, this suggests that differentiation is already maximized 7 days after FGF4 removal to the point that ATP is depleted and rate-limiting. At 0.5% O₂, this suggests there are no agonist's effects because mitochondria are inactive due to high PKM2 diverting carbon from the mitochondria, and insufficient COX and O₂ to enable OxPhos. Taken together, these lines of evidence suggest that full mitochondrial activity is needed to enable full differentiation after the mitochondria are fully active three days after FGF4 removal.

It should be noted that the highest accumulation of ATP after 7 days of FGF4 removal occurs at 0.5% O₂ suggesting that other factors besides ATP insufficiency truncate differentiation. Two candidates are residual high potency factors or induced hypoxia inducible factors (HIFs) that attenuate TSC differentiation. Caniggia, Placenta, 2000;21 Suppl A:S25-30. HIFs are a prime candidate because they maintain glucose uptake and aerobic glycolysis (Semenza, Cold Spring Harbor Symp. Quant. Biol., 76:347, 2011; Qing, Cancer Res., 70:10351, 2010) and may contribute to the mechanism that prevents PKM2 loss with FGF4 removal at 0.5% O₂. Another candidate is reactive oxygen species (ROS) generation by mitochondria which is attenuated during hypoxia and supports differentiation of some stem cells. Testing of these candidates is underway, but underlying mitochondrial ATP insufficiency due to hypoxia remains a fundamental problem aside from the other candidates. Despite FGF4, at 20% O₂ hyperosmolar stress induces TSCs to differentiate to the 1^{st} lineage, producing Hand1 positive pTGC and Hand1-dependent PL1. Awonuga 2011. During induction of pTGC hyperosmolar stress does not induce later lineages such as sTGCs or synTA cells at 20% O₂, suggesting that stress prioritizes TSC differentiation. Liu, supra. Hyperosmolar stress induced-differentiation of Hand1 and PL1 proteins require SAPK enzyme function. Awonuga, 2011.

Similarly departure from the optimum at 2% O₂ increased stress, SAPK activity, and SAPK-dependent increase in Hand1 mRNA transcripts. But, here it is shown for the 1^{st} time that SAPK also mediates a decrease in Gcm1 mRNA transcripts proportional to the departure from the optimum O₂ for stemness at 2%. Although the SAPK effects are not the dominant effects, possibly because of mitochondrial insufficiency limiting some lineages, this is the 1^{st} evidence for an enzyme that mediates prioritized (aka "default") differentiation. SAPK knockouts do not affect placentation in a normal vivarium (Xie, 2011; Rappolee, Reproduction, 145:R139, 2013 (Rappolee, 2013) so it will be important to study the effects of gestational hypoxic stress in SAPK knockouts.

Gestational hypoxia has revealed that placental and uterine hormones that are unnecessary during normal development become essential during hypoxic stress *in vivo.* Xie, 2011; Rappolee, 2013. In addition, gestational hypoxia produces a phenotype *in vivo* that has similarities with the phenotype observed here during hypoxia in culture. In both cases there was an increase in pTGC and a suppression of labyrinthine placenta.

A key interpretation is that stress leads to decreased stem cell growth and the further depletion of stem cell potency to enable differentiation of early essential function. A similar outcome is seen in preeclamptic human placenta where proliferative cytotrophoblasts are depleted while differentiated villous surface function is maintained. Longtine, Placenta, 33:352, 2012.

Stress induces differentiation but hypoxia for stemness, 0.5% O₂, truncates this and leads to rapid but incomplete differentiation that suppresses all lineages but two at the labyrinthine surface are suppressed the most. The mechanisms mediating the suppression are important to understand because diseases such as intrauterine growth retardation and preeclampsia feature labyrinthine dysfunction. It is important to test for these hypoxic effects in human 1^{st} trimester villous explants or human TSCs.

Example 2. Introduction. Transcription factor (TF) expression and therefore lineage identity in the peri-implantation embryo and its stem cells may be influenced by extracellular stresses. Fauque, PLoS One, 5:e9218, 2010; Zhong, 2010. Perturbations of the embryo during the critical period of implantation frequently lead to loss of the pregnancy. Smart, Clin. Reprod. Fertil., 1:177, 1982; Wilcox, N. Engl. J. Med., 319:189, 1988. Understanding the integration of stress enzyme signaling of the developing embryo may help to improve early pregnancy success rates, and avoid or mitigate long-term negative effects on the health of offspring.

*In vivo,* the earliest placental lineage to differentiate after embryo implantation is trophoblast giant cells (TGCs). TGCs maintain early pregnancy by producing the hormones that stimulate uterine changes necessary to support an embryo. When placental trophoblast stem cells (TSCs), precursors to TGCs, were confronted with hyperosmotic stress in vitro, the stress enzymes that were activated modulated lineage TF expression. Zhong, 2010; Xie, 2014; Xie, Stem Cells Dev., 22:1564, 2013 (Xie, 2013)); Awonuga, 2011. Nearly all surviving TSCs terminally differentiated to 1^{st} -lineage TGCs (Xie, 2014; Awonuga, 2011; Liu, *supra*) and later lineages were suppressed. Xie, 2014; Liu, supra. This would hypothetically provide for the nutritional needs of the implanting embryo but leave insufficient stem cells to populate the other necessary placental lineages, jeopardizing long-term survival of the embryo.

Murine embryonic stem cells (mESCs) derived from the inner cell mass (ICM) of an E3.5 blastocyst are also highly sensitive to extrinsic signaling. Lander, J. Biol., 8:70, 2009. Extracellular signal regulated kinase (ERK) signaling can induce differentiation of mESCs; its suppression allows pluripotent stem cells to be derived from refractory mouse strains, and also allows the self-renewal of mESCs in culture. Burdon, Cells Tissues Organs, 165:131, 1999. Phosphoinositide 3-kinase (PI3K) regulates both the proliferation and pluripotency of mESCs, in part by its ability to maintain Nanog expression. Storm, J. Biol. Chem., 282:6265, 2007. p38MAPK signaling is necessary for mesoderm development (Duval, Cell Death Differ., 11:331, 2004; Barruet, Stem Cells Dev., 20:1233, 2011), and mESCs lacking c-Jun N-terminal kinase (JNK)1 fail to undergo neuronal differentiation. Amura, Mol. Cell. Biol., 25:10791, 2005. All of these enzymes may be activated by external stressors, such as hyperosmotic stress. Sheikh-Hamad and Gustin, Am. J. Physiol. Renal Physiol., 287:F1102, 2004. Therefore extrinsic stress signaling through stress enzymes may influence the kinetics and/or lineage allocation of differentiating mESCs.

Pluripotency in both murine embryonic stem cells (mESCs) and human embryonic stem cells (hESCs) is maintained by a network of TFs-Oct4, Sox2, and Nanog-which suppress the differentiated state. Boyer, Cell, 122:947, 2005; Lee, Cell, 125:301, 2006. The TF Rex1 is another common marker of the pluripotent state. Sharova, Dev. Biol., 307:446, 2007. Toxicological stressors can decrease potency in hESCs via a decrease in Oct4, Sox2, and Rex1 that potentially leads to abnormal differentiation. Pal, J. Cell. Physiol., 226:1583, 2011).

Oct4 maintains pluripotency in part by suppressing trophectoderm in both the ICM of the embryo and in the derivative mESCs in culture. Nichols, Cell, 95:379, 1998; Niwa, Nat. Genet., 24:372, 2000 (Niwa, 2000). A loss of 50% of Oct4 levels results in differentiation to trophectoderm, while a 50% increase above normal expression triggers differentiation to the early appearing primitive endoderm (PrEndo). Niwa, 2000. This is a reflection of the transient higher levels of Oct4 in the delaminating primitive endoderm derived from ICM of the E3.5 blastocyst. Palmieri, Dev. Biol., 166:259, 1994. Recent evidence suggests that Oct4 is required for differentiation of extraembryonic endoderm (ExEndo) by non-cell autonomous fibroblast growth factor (FGF)4 function and by cell autonomous upregulation of ExEndo TFs. Frum, Dev. Cell., 25:610, 2013; Frankenberg, Dev. Cell., 21:1005, 2011. Thus, small, transient changes in Oct4 levels change the potency of mESCs and stress may contribute to transient Oct4 regulation.

Nanog suppresses PrEndo and its derivative ExEndo expression in the blastocyst. High Nanog expression is found only in pluripotent cells; low expression sensitizes mESCs to differentiation signals, committing them to PrEndo and later ExEndo lineages. Chambers, Nature, 450:1230, 2007; Singh, Stem Cells, 25:2534, 2007. Rex1 expression correlates strongly with pluripotency in mESCs (Sharova, supra); its expression is lost as mESCs differentiate to either PrEndo/ExEndo or the later-appearing embryonic ectoderm (EmEcto). Rathjen, J. Cell. Sci., 112 (Pt 5):601, 1999; Lake, J. Cell. Sci., 113 (Pt 3):555, 2000; Toyooka, Development, 135:909, 2008. In contrast to Oct4, Rex1-homozygous-null-mutant mESCs can be isolated, but have a higher spontaneous differentiation rate to all lineages. Scotland, Dev. Dyn., 238:1863, 2009. *In vivo,* Rex1-null embryos express some visceral endoderm markers at lower levels (Masui, BMC Dev. Biol., 8:45, 2008 (Masui, 2008)), suggesting a role for Rex1 in ExEndo differentiation.

Therefore, cells of the ICM and its immediate successor lineages, PrEndo and EmEcto, may be identified by measuring the relative quantitative expression of potency TFs. The current Example used hyperosmotic stress to concurrently activate multiple stress enzyme signaling pathways in mESCs. The stress-enzyme-dependent changes in TFs Oct4, Nanog, Sox2, and Rex1 were subsequently measured to reveal the impact of stress on pluripotency. After screening 21 signaling kinases in 11 subfamilies using 14 inhibitors, 5 protein kinases (PKs) were revealed to affect the kinetics and/or the allocation of lineages from differentiating mESCs. While hyperosmotic stress did not trigger morphological differentiation of mESCs cultured in monolayer, it primed mESCs to initiate 1^{st} -lineage PrEndo/ExEndo differentiation, altering the response of mESCs to differentiation cues.

Materials and Methods. Reagents.MG132, lactacystin, and sorbitol were from Sigma (St. Louis, MO). Enzyme inhibitors LY294002, U0126, PD98059, SB202190, AKTi, and L-JNKi-1 were from Calbiochem (La Jolla, CA). Amido black was from MP Biomedicals (Solon, OH). Rabbit polyclonal to mouse Oct4 (sc-5279), goat anti-human Sox2 (sc-17320), anti-rabbit MEK1 (sc-219), and anti-mouse MEK2 (sc-13159) antibodies were from Santa Cruz Biotechnology (Santa Cruz, CA). Antirabbit Nanog was from Chemicon/Millipore (AB5731; Billerica, MA) and Rex1 antibodies were from Abcam (AB28141; Cambridge, MA). Anti-rabbit p38 (CS9212), phospho-p38 (Thr180/Tyr182 CS9211), JNK (CS9252), phospho-SAPK (Thr183/Tyr185 CS9251), phospho-MEK1/2 (Ser217/221 CS9121), ERK1/2 (CS9102), phospho-ERK1/2 (Thr202/ Tyr204, CS9106), phospho-AKT (Ser473 CS2965), b-actin (CS4967), and cleaved-caspase 3 (CS9664) antibodies were from Cell Signaling (Beverly, MA). For real-time quantitative PCR (RT-qPCR) the RNeasy Mini Kit was used for RNA isolation and QuantiTect Reverse Transcription Kit, both from Qiagen (Germantown, MD), and Fast SYBR Green Master Mix from Applied Biosystems (Foster City, CA). RNA primers (Oct4,Nanog, Rex1, Dab2, Lrp2, and Fgf5) were from Integrated DNA Technologies (Coralville, IA).

Cell culture and stimulation. mESC-D3 cells (ATCC, Manassas, VA) were cultured in the absence of feeder cells in Dulbecco's modified Eagle's medium (Gibco, Grand Island, NY) supplemented with 15% mESC-screened fetal bovine serum (HyClone, Logan, UT), 2mM L-glutamine, 1mM sodium pyruvate, 1mM nonessential amino acids, 0.1mM 2-mercaptoethanol (Sigma), and 1,000 U/mL murine leukemia inhibitory factor (LIF; Millipore, Temecula, CA) on 0.1% gelatin-coated dishes at 37°C in humidified air with 5% CO₂. Masui, 2008. mESCs were cultured overnight after passaging before stimulation with sorbitol. Osmolality was determined by freezing point depression using Advanced Instruments Wide Range Osmometer 3W2 (Advanced Instruments, Norwood, MA). One liter of a very dilute aqueous solution at room temperature very nearly has a mass of 1 kg, so at low solute concentrations (< 0.50 M), osmolarity and osmolality are considered equivalent.

Enzyme inhibition. Enzyme inhibitors were chosen according to the specificity reported in the kinase inhibitor literature. Alessi, J. Biol. Chem., 270:27489, 1995; Davies, Biochem. J., 351:95, 2000. Inhibitor concentrations were determined following dose response experiments for each inhibitor based on the range of concentrations determined from the mESC and kinase inhibitor literature. Alessi, supra; Davies, supra; Gross, Mol. Reprod. Dev., 70:324, 2005); Hamazaki, Mol. Cell. Biol., 26:7539, 2006; Bain, Biochem. J., 408:297, 2007; Lee, Cell Prolif., 41:230, 2008. The single doses selected for use in ongoing experiments and shown herein were the lowest doses impacting Oct4 protein expression following 4 hr of sorbitol exposure (as determined by western blot) with minimal toxicity (as determined by phase microscopy). Inhibitors were suspended in dimethyl sulfoxide (DMSO), diluted to the proper concentrations, and preloaded for 1 hr prior to sorbitol stimulation. Vehicle-only control experiments were performed and expression of Oct4, Nanog, and Rex1 was determined by western blot to be not significantly different from nonvehicle control (data not shown). Inhibitor-only experiments were performed for 24 hr to determine the impact of enzyme inhibition on Oct4, Nanog, and Rex1 expression.

**Table 3. Inhibitor only effects on Oct4, Nanog, and Rex1 expression.**

| Table 3 | | | |
|---|---|---|---|
| Inhibitor | Oct4 | Nanog | Rex1 |
| AKTi (10µM) | 0.88±0.03 | 0.97±0.04 | 1.1±0.12 |
| LY294002 (10µM) | 1.34±0.06 | 1.02±0.08 | 1.11±.12 |
| L-JNKi-1 (2µM) | 0.90±0.06 | 0.63±0.16 | 0.96±0.10 |
| SB202190 (10µM) | 0.91±0.28 | 0.71±0.20 | 0.78±0.18 |
| U0126 (40µM) | 1.16±0.31 | 0.94±0.15 | 0.72±0.06 |
| PD98059 (20µM) | 1.24±0.21 | *1.54±0.10 | 0.93±0.06 |
| mESC were cultured for 24 hr in the presence of inhibitor only. Values shown represent percentage of protein expression compared to 24 hr, no inhibitor (set to 100%). ANOVA with Student-Newman-Keul post hoc test, n>=3, p<0.05. *There was a significant effect on Nanog expression during 24 hr of PD98059 inhibitor-only treatment (p=0.0007). However in Table 4, PD98059 targets had no significant effect on the recovery of Nanog during 24 hr of stress, so the biological significance of PD98059 inhibitor-only is nil. | | | |

In 14/18 conditions, inhibitors had no effect on potency TF levels; in 2 conditions, the inhibitors decreased expression by 20% and, in the remaining 2 conditions, TF expression was increased above baseline. Inhibitor-only experiments were performed for 4 and 24 hr to determine the impact of enzyme inhibition on Oct4, Nanog, and Rex1 expression. There were no significant differences in expression after 4 hr of inhibitor-only treatment (Oct4 P= 0.78, Nanog P= 0.81, and Rex1 P= 0.55; data not shown). There were no significant differences in expression of Oct4 (P = 0.23), Rex1 (P= 0.053), or 5/6 of the Nanog inhibitors (P= 0.19) after 24 hr of inhibitor-only inhibition (Table 3). There was a significant effect on Nanog expression during 24 hr of PD98059-inhibitor-only treatment (P< 0.001). However in Table 4, PD98059 targets had no significant effect on the recovery of Nanog during 24 hr of stress, the biological significance of PD98059 inhibitor only is nil. DMSO-only controls were also done. DMSO at 0.6% did not impact Oct4, Nanog, and Rex1 expression at 4 or 25 hr. This (0.6%) was higher than the highest diluent concentration in any of the described experiments (0.4% in the U0126 experiments).

**Table 4. TF expression levels during 24 hr of hyperosmotic stress +/- enzyme inhibition in mESC monolayer culture.**

| Table 4 | | | |
|---|---|---|---|
| 24 hr inhibition | Oct4 | Nanog | Rex1 |
| Unstressed | 1.00 ± 0.00 | 1.00 ± 0.00 | 1.00 ± 0.00 |
| Stress only (S) | 1.03 ± 0.16 | 1.15 ± 0.20 | 0.35 ± 0.08 * |
| PD98059 (MEK1) + S | 0.78 ± 0.06 | 0.80 ± 0.09 | 0.51 ± 0.01 * |
| U0126 (MEK1/2) + S | 0.27 ± 0.03 *, a | 0.67 ± 0.32 | 0.31 ± 0.12 * |
| SB202190 (p38) + S | 0.51 ± 0.07 *, a | 0.10 ± 0.01 *, a | 0.19 ± 0.07 * |
| L-JNKi-1 (JNK) + S | 0.91 ± 0.15 | 0.71 ± 0.15 | 0.89 ± 0.08 a |
| LYZ94002 (PI3K) + S | 0.65 ± 0.08 | 0.44 ± 0.07 *, a | 0.00 *, a |
| AKTI + S | 0.84 + 0.19 | 0.97 + 0.03 | 0.44 ± 0.04* |
| Values represent percent of unstressed expression. '*' means statistically different from unstressed. 'a' means statistically different from 'stress only' , n>/=3, ANOVA and Student-Newman-Keul's post hoc tests, p<0.05. | | | |

Cell accumulation and apoptosis. Cell accumulation was assayed by counting cells using a hemocytometer following trypan blue exclusion. MESCs were trypsinized, plated, and cultured overnight to allow for adaptation after passage. Time-zero counts were taken at least 1 day after passage and all subsequent counts were normalized to this. Apoptosis was measured by immunoblot for cleaved caspase 3. Mullen and Critser, Methods Mol. Biol., 254:393, 2004.

Microscopy. Indirect immunocytochemistry was performed as described previously. Wang, Dev. Dyn., 231:72, 2004; Xie, Mol. Reprod. Dev., 71:1, 2005. Photomicrography was performed using a Leica DM IRE2 automated epifluorescence microscope (Wetzlar, Germany) controlled electronically by Simple PCI Al software (Hamamatsu Corporation, Sewickley, PA). All micrographs were taken at a magnification of 100 X, except where indicated.

Western blot analysis. Sodium dodecyl sulfate-polyacrylamide gel electrophoresis and western immunoblot analysis of mESC lysates were performed as previously described. Xie, Mol. Hum. Reprod. 13:473-81 (2007). Cells were harvested with cold lysis buffer (Cell Signaling) and protein was quantified by BCA assay (Pierce, Rockford, IL). Ten to twenty micrograms of aliquots were fractionated on 10% polyacrylamide precast gels (Bio-Rad, Hercules, CA), transferred to nitrocellulose membranes (Amersham Biosciences, Aylesbury, United Kingdom), probed overnight with primary antibodies, and developed as previously described. Xie, supra, 2005. Protein bands were visualized using the ECL Advance Western Blotting Detection Kit (GE Healthcare, Waukesha, Wl), blot was scanned to obtain an electronic image, and intensity of protein bands was quantified using Image J analysis software (rsbweb.nih.gov) and normalized to amido black staining. Aldridge, J. Neurosci. Methods, 172:250, 2008. Data are expressed as the change in expression relative to no treatment at time zero.

RT-qPCR analysis. mESCs were trypsinized and harvested for RT-Qpcr analysis using a 7500 Fast Real Time PCR System (Applied Biosystems). Total RNA was isolated from cell lysates using RNeasy Mini Kit (Qiagen). RNA content was measured using ND-1000 Spectrophotometer (NanoDrop and ThermoScientific, Wilmington, DE). Complementary DNA (cDNA) was synthesized from 50 to 100 ng of total RNA using QuantiTect Reverse Transcription Kit (Qiagen) according to the manufacturer's instructions, and diluted 1:5. One microliter of cDNA template was added to 1 mL of both the forward and reverse primers for each specific transcript and 10 mL of Fast SYBR Green Master Mix (Applied Biosystems) for the RT-qPCR. Primer sequences are shown in Table 5.

**Table 5. RT-qPCR primer sequences.**

| Table 5 | | |
|---|---|---|
| Gene | Forward primer sequence | Reverse primer sequence |
| Oct4 | AGTTGGCGTGGAGACTTTGC (SEQ ID NO:122) | CAGGGCTTCATGTCCTGG (SEQ ID NO:123) |
| Nanog | TCTTCCTGGTCCCCACAGTTT (SEQ ID NO:124) | GCAAGAATAGTTCTCTCGGGATGAA (SEQ ID NO:125) |
| Rex1 | CCCTCGACAGACTGACCCTAA (SEQ ID NO:126) | TCGGGGCTAATCTCACTTTCAT (SEQ ID NO:127) |
| Dab2 | CCCCTGAACGGTGATACTGAT (SEQ ID NO:128) | AAGTCCTGCTTTACGCCATTC (SEQ ID NO:129) |
| Lrp2 | AAAATGGAAACGGGGTGACTT (SEQ ID NO:130) | GGCTGCATACATTGGGTTTTCA (SEQ ID NO:131) |
| Fgf5 | TGTGTCTCAGGGGATTGTAGG (SEQ ID NO:132) | AGCTGTTTTCTTGGAATCTCTCC (SEQ ID NO:133) |
| Gapdh | CATGTTCCAGTATGACTCCACTC (SEQ ID NO:134) | GGCCTCACCCCATTTGATGT (SEQ ID NO:135) |
| 18s ribosomal subunit | SEQ ID NO:120 | SEQ ID NO: 121 |

Primer pairs were checked for specificity using BLAST analysis and were checked by both agarose gel electrophoresis and thermal dissociation curves to ensure amplification of a single product, and to rule out formation of primer dimers during the RT-qPCR. The RT qPCR cycling parameters were as follows: enzyme activation, 95°C for 20 s; denature, 95°C for 3 s; and anneal/extend, 60°C for 30 s for a total of 40 cycles. The expression of the target genes was quantified against that of two internal reference genes, glyceraldehyde-3-phosphate dehydrogenase (GAPDH) and 18s ribosomal RNA subunit (18s rRNA). GAPDH was determined to be the most stable of 10 reference genes tested during mESC differentiation (Murphy and Polak, Tissue Eng., 8:551, 2002; Willems, Int. J. Dev. Biol., 50:627, 2006), and 18s rRNA was selected due to its stability during hyperosmolar conditions in TSCs. Liu, supra. Fold change was determined using the ddCt method. Livak and Schmittgen, Methods, 25:402, 2001. Data are expressed as the fold change in expression relative to no treatment at time zero.

Statistical analysis. Results of these investigations were described as the mean-standard error of at least three independent experiments. Data were analyzed using SPSS v. 19.0. In some cases, hypotheses were restated and additional replicates with increased numbers of controls were performed to obtain higher statistical confidence. Thus, the sample size for each data point in some figures may vary. Statistical analysis included ANOVA with Student-Newman-Keuls post hoc tests, or Kruskal-Wallis nonparametric ANOVA tests (due to non-normal distribution of data), followed by Mann-Whitney tests on each pair of groups with Bonferroni correction of the P-value. Groups were considered to be significantly different if P< 0.05.

Results. mESC growth and colony morphology during hyperosmotic stress. 400 mM sorbitol is the dose that induces the highest levels of stress enzyme activity and function in mouse TSCs and embryos. Xie, 2007; Zhong, 2007; Xie, Mol. Reprod. Dev., 75:689, 2008. The described mESC studies therefore began with this dose, but it proved to be lethal in mESCs (FIG. 13A). 200 mM sorbitol was then established as the nonlethal experimental dose, and the effects of this dose were tested on mESC proliferation and apoptosis. Osmolality was measured at 330±4 mOsm/kg H₂O before sorbitol addition (described in "Materials and Methods" section; Zhong, 2007), and 531±7 mOsm/kg H₂O after addition of 200 mM sorbitol. At low solute concentrations, osmolality and osmolarity can be considered equivalent because the mass of 1 L of a dilute aqueous solution is very close to 1 kg. The osmolarity of mouse uterine fluid has been reported as 330 mOsm. Harris, Theriogenology, 64:992, 2005. Thus the described baseline media were isotonic with uterine fluid.

To establish the optimal, nonmorbid hyperosmotic dose, mESCs were cultured for 72 hr in three experimental conditions: isosmotic media +LIF, isosmotic media -LIF, and hyperosmotic media +LIF. Cell counts were done (FIG. 13B) and micrographs were taken at 24, 48, and 72 hr of culture (FIG. 13D). Stress slowed growth rates from an overall doubling rate of 17.4 hr in untreated cells +LIF to 30.8 hr in treated mESCs (FIG. 13C).

To test whether the reduced accumulation of cells during stress was the result of slowing of the cell cycle or apoptosis, apoptosis was assayed for by probing for the small cleavage product generated when caspase 3 is activated. It has been reported that mESCs do not express a functional death ligand (Fas/FasL) system. Brunlid, Stem Cells, 25:2551, 2007. Nevertheless, caspase 3 is activated in both the extrinsic (i.e., extracellular induction) and intrinsic (mitochondrial) apoptotic pathways. This cleavage product was detected following 1-2 hr of stress (14% of cells; FIG. 13E), and occasionally at 4 h, but by 6-24 hr the remaining cells had adapted to the stress with cleaved-caspase 3 detected in fewer than 5% of cells by immunofluorescence, and no protein detected by immunoblot. mESC survival in hyperosmotic conditions was dependent on PI3K signaling (FIG. 13F); inhibition of PI3K with LY294002 led to massive cell death of up to 90% of mESCs during 24 hr of hyperosmotic stress. Thus this level of hyperosmotic stress produced transient apoptosis followed by diminished but positive cell growth and survival that was PI3K dependent.

Hyperosmotic activation of signaling enzymes. A small subset of the 500 PKs in the kinome typically respond to stress stimuli, suggesting these as candidates for mediating cellular responses to hyperosmotic stress. Zhong, 2010; Xie, 2011. After screening 21 signaling kinases in 11 subfamilies using 14 inhibitors, 5 kinases in the mitogen activated protein kinase (MAPK) and PI3K families were identified as producing differentiation-priming effects in hyperosmotic conditions. To determine the kinetics of activation of these five kinases in the described system, mESCs were treated with 200 mM sorbitol for 1 hr. Expression levels of the activated (i.e., phosphorylated) enzymes were assayed by western blot analysis (FIG. 14), including phospho-p38MAPK, phospho-JNK, phospho-MEK1/ 2, and phospho-AKT (the downstream effector of PI3K signaling). Activated p38MAPK was 1^{st} detected at 10 min of stimulation; activation persisted throughout 1 hr of stimulation. Similarly, activated JNK was 1^{st} detected at 20 min of stimulation, and persisted throughout 1 hr of stimulation. Activated MEK1/2 and activated AKT were present endogenously at low levels at time zero; sorbitol stimulated higher activation levels of each. Increased MEK1/2 activation was stimulated within the 1^{st} 10 min of stress exposure, but returned to baseline levels by 20 min. In contrast, increased AKT activation occurred by 20-30 min of stimulation, and persisted through 1 hr of sustained stress exposure.

Efficacy of enzyme inhibitors. Efficacy of the pharmacological inhibitors for these enzymes was tested under stress conditions (FIG. 15). LY294002 and AKTi inhibited AKT activation by an average of 88% and 95%, respectively. PD98059 directly inhibits new MEK1 activation; its efficacy was determined by measuring the activation of MEK1's downstream targets, ERK1/2. PD98059 inhibited ERK1/2 activation by an average of 30%. This is consistent with the literature that describes the mechanism of action of PD98059; it binds to inactive MEK1 thus preventing new activation, but does not shut off endogenously active MEK1. Dudley, Proc. Natl. Acad. Sci. USA, 92:7686, 1995; Goueli, Promega Notes, 69:6, 1998. U0126 inhibited ERK1/2 activation by an average of 87%. SB202190 inhibited p38MAPK activation by an average of 60%. L-JNKi-1 inhibited JNK activation by an average of 50%. Thus highest inhibition and specificity was identified and attained in line with the encyclopedic testing by Bain, Biochem. J., 371:199, 2003.

Hyperosmotic stress modulated expression of TF markers of pluripotency. mESCs were treated with 200 mM sorbitol for 24 hr to test for stress-induced differentiation through loss of potency TFs and/or gain of TFs that mark differentiated lineages PrEndo/ExEndo or EmEcto. FIG. 16A illustrates the relative expression of these TFs in the undifferentiated ICM, modeled by mESCs, and each of the potential downstream lineages of mESCs, PrEndo that gives rise to other ExEndo lineages, and EmEcto. Expression levels of Oct4, Sox2, Nanog, and Rex1 were determined at multiple time points up to 24 hr by western blot analysis and RT-qPCR. Hyperosmotic stress rapidly activated the differentiation program, with expression of Oct4, Nanog, Rex1, and Sox2 TF proteins decreasing within the 1^{st} hour and reaching a nadir between 2 and 4 hr of continued stress (FIG. 16B). The nadir of Nanog expression was more variable than the other three TFs, occurring as early as 2 hr in some experiments or as late as 6 hr of stress. By 6 h, the overt differentiation program was aborted, and the decline in Oct4, Sox2, and Nanog expression halted. Over the next 2-20 h, expression of these three TF proteins rebounded toward the unstressed baseline with both Sox2 and Nanog achieving complete, robust recovery to their prestressed protein levels (FIG. 16B). Because Nanog, Oct4, and Rex1 expression was adequate to identify mESC or subsequent lineages, Sox2 expression was not continued. Nanog and Oct4 recovery was maintained through 72 hr of ongoing stress (FIG. 16E, Oct4, data not shown). Recovery at the mRNA level also occurred, with both Oct4 and Nanog moving toward their unstressed baseline, although this recovery had not reached significance at 24 hr (FIG. 16C).

In contrast, Rex1 protein levels did not rebound, remaining at < 40% of its unstressed levels throughout the 24-h time course (FIG. 16B). Rex1 mRNA levels, however, recovered to 70% of their unstressed levels by 24 hr (FIG. 16C).Overall, stress induced a rapid, transient loss of protein and mRNA in potency TFs, but this loss was reversed in some TFs as mESCs adapted to the stress. Rex1 protein was exceptional in its persistent stress-induced suppression through 24 hr.

Hyperosmotic effects on lineage markers during monolayer cell culture. The persistent suppression of Rex1 during hyperosmotic stress suggested that some mESCs were differentiating to PrEndo/ExEndo or EmEcto. RT-qPCR was therefore used to look at markers of these lineages after 24 hr of hyperosmotic stress in a monolayer culture system (FIG. 16D). Lrp2, which arises in the E3.5 PrEndo (Frankenberg, supra; Gerbe, Dev. Biol., 313:594, 2008), and Dab2, which arises in the E4.5 ExEndo (Frankenberg, supra; Yang, Dev. Biol., 251:27, 2002) were selected as PrEndo/ExEndo markers, and Fgf5, which arises in the E5.5 EmEcto (Haub & Goldfarb, Development, 112:397, 1991; Hebert, Development, 112:407, 1991), was selected as the EmEcto marker (FIG. 16A). mRNA expression was compared in stressed mESCs with LIF with that of both unstressed mESCs cultured in the presence of LIF (a pluripotent control) and unstressed mESCs cultured following LIF removal (a differentiation control).

Sorbitol induced significantly higher levels of Lrp2 and Dab2 expression compared with either of the time-matched controls (FIG. 16D). In contrast, although 24 hr of LIF removal was sufficient for unstressed cells to upregulate the EmEcto marker Fgf5, the presence of sorbitol suppressed this upregulation in stressed cells. Thus, the stress-induced loss of Rex1 correlated with upregulation of markers of the early appearing PrEndo/ExEndo lineages, but suppression of the later EmEcto lineage marker.

Hyperosmotic-stress-induced loss of potency TFs due to proteasomal degradation. To determine the mechanism of TF protein loss during stress, mESCs were treated with one of two proteasome inhibitors, MG132 (FIG. 17) and lactacystin (FIG. 18A-18D). Both inhibitors prevented the stress-induced loss of Oct4, Nanog, and Rex1. Oct4 loss was reversed by 75%, Nanog by 93%, and Rex1 by 310% in 4 hr of proteasome inhibition by MG132.

MEK1 and other enzymes trigger mESC initial stress response: initiation of differentiation program. mESCs were cultured in the presence of pharmacological inhibitors and hyperosmotic stress for 4 hr to identify the enzymes that mediated the stress-induced loss of potency TF proteins, reported in FIG. 16B. The complete results of p38, JNK, MEK1/2, and PI3K inhibition during 4 hr of hyperosmotic stress in mESCs are summarized in Table 6.

**Table 6. TF expression levels during 4 hr of hyperosmotic stress +/- enzyme inhibition in mESC monolayer culture.**

| Table 6 | | | |
|---|---|---|---|
| 4 hr inhibition | Oct4 | Nanog | Rex1 |
| Unstressed | 1.00 ± 0.00 | 1.00 ± 0.00 | 1.00 ± 0.00 |
| Stress only (S) | 0.52 ± 0.03 * | 0.65 ± 0.07 * | 0.63 ± 0.06 * |
| PD98059 (MEK1) + S | 0.93 ± 0.09 a | 2.57 ± 0.50 *,a | 1.20 ± 0.05 a |
| U0126 (MEK1/2) + S | 0.44 ± 0.13 * | 0.24 ± 0.02 *, a | 0.98 ± 0.04 a |
| SB202190 (p38) + S | 0.81 ± 0.21 | 0.52 ± 0.12 * | 0.59 ± 0.23 * |
| L-JNKi-1 (JNK) + S | 0.21 ± 0.04 *, a | 1.08 ± 0.11 a | 0.47 ± 0.04 * |
| LY294002 (PI3K) + S | 0.92 ± 0.12 a | 0.55 ± 0.10 * | 0.36 ± 0.14 * |
| AKTi + S | 0.57 ± 0.05 * | 0.47 ± 0.06 * | 0.43 ± 0.14* |
| Values represent percent of unstressed expression. '*' means statistically different from unstressed. 'a' means statistically different from 'stress only', n>/=3, ANOVA and Student-Newman-Keul's post hoc tests, p<0.05. | | | |

MEK1 activation triggered a loss of expression of all three TFs; when its stress-induced activation was prevented by PD98059, expression of both Oct4 and Rex1 remained at the unstressed baseline, while Nanog expression increased to 2.5 times its unstressed level (FIG. 19A). This effect was repeated for Rex1 during inhibition with the MEK1/2 inhibitor U0126 (FIG. 19B); due to inhibition of both MEK1 and MEK2 during stress exposure, Rex1 expression remained at unstressed levels. This suggests that MEK1 was the regulator of Rex1 protein destruction during stress.

In contrast, MEK1/2 inhibition with U0126 did not prevent Oct4 or Nanog loss during stress for 4 hr (Table 6). Instead, inhibition of MEK1/2 kinase activity allowed even greater loss of these two TFs. Therefore a U0126 target that is not targeted by PD98059, putatively MEK2, protected Oct4 and Nanog from being completely lost during stress. This indicates that the targets of U0126 and PD98059 produce opposite effects on pluripotency, and perhaps interact negatively with each other.

Several other enzymes were also implicated in the stress-induced loss of potency TFs. PI3K signaling led to loss of Oct4 expression during the 1^{st} 4 hr of stress; its inhibition with LY294002 prevented the stress-induced loss of Oct4 (FIG. 19B). This effect was not mediated through the AKT pathway, as specific inhibition of AKT did not prevent Oct4 loss (Table 6). When both PI3K and MEK1 signaling was inhibited simultaneously, Oct4 levels did not rise significantly above baseline (data not shown). This suggests that both PI3K and MEK1 use a common pathway to destroy Oct4 protein under stress conditions. A third stress enzyme, JNK, was involved in the loss of Nanog expression during stress; its inhibition allowed Nanog expression to be preserved during stress (FIG. 19B).

p38MAPK and other signaling kinases produced mESC adaptive response to hyperosmotic stress: differentiation program aborted, and pluripotency restored at 24 hr. The enzymatic mechanisms that mediated the recovery of potency TF proteins during stress that persisted for 24 hr was next tested. mESCs were cultured in the presence of hyperosmotic stress for 24 hr with or without pharmacological inhibitors of PKs; the complete results of p38MAPK, JNK, MEK1/2, and PI3K inhibition during 24 hr of hyperosmotic stress are summarized in Table 4. p38MAPK signaling mediated the recovery of Oct4 and Nanog proteins to their unstressed baselines during 24 hr of stress (FIG. 20A). When stress-induced activation of p38MAPK was prevented by SB202190, Oct4 expression did not recover from its 4-h nadir of 50% of unstressed levels. Similarly, Nanog expression during ongoing stress decreased to 10% during p38MAPK inhibition. Micrographs of p38MAPK-inhibited mESCs are shown in FIG 8B; although 24 hr is not adequate to see outright differentiation, the colonies do appear to show the initial signs of differentiation, with some migration of cells away from the colonies, larger cobblestone cells characteristic of ExEndo (Brown, PLoS One, 5:e12016, 2010), and more projections of the cells along the colony borders. Thus, p38MAPK inhibition affected the stress-induced changes in morphology and appeared to enable stress-induced differentiation.

Inhibition of both MEK1 and MEK2 with U0126 also prevented Oct4 recovery (FIG. 20C), whereas inhibition of only MEK1 with PD98059 did not prevent Oct4 recovery (Table 4). When taken together with the U0126 results at 4 hr of hyperosmotic stress, this suggests that the unique U0126 target(s), putatively MEK2, plays a role in reserving pluripotency during adaptation to stress conditions after 4 hr. Similarly, PI3K signaling played a role in Nanog recovery during ongoing stress; its inhibition prevented Nanog recovery.

JNK-dependent signaling produced persistent suppression of Rex1 during 24 hr of hyperosmotic stress. In contrast to the p38MAPK-dependent effects on Oct4 and Nanog during 24 hr of hyperosmotic stress, p38MAPK signaling was not able to reverse Rex1 suppression, neither was PI3K signaling (FIG. 21A). Instead, JNK signaling suppressed Rex1 expression during 24 hr of hyperosmotic stress (FIG. 21A). To test whether the stress-induced suppression of Rex1 was reversible, mESCs were subjected to only 4 hr of hyperosmotic stress and then returned to iso-osmotic media. Rex1 expression at 24 hr recovered to baseline; this indicates that continued stress was required for persistent Rex1 suppression (FIG. 20B). The persistent suppression of Rex1 throughout the duration of stress stimulation suggests that at least a subpopulation of mESCs may be primed by stress for differentiation by maintaining chronic Rex1 loss as well as transient loss of Oct4, Nanog, and Sox2.

Discussion. In this Example, the level of hyperosmotic stress analyzed slowed mESC accumulation due to slowing of the cell cycle, not ongoing apoptosis. PI3K signaling was responsible for cell survival under these stress conditions. Stress initially triggered mESC differentiation through MEK1, JNK, and PI3K signaling, leading to proteasomal degradation of Oct4, Nanog, Sox2, and Rex1 proteins. Concurrent with this posttranscriptional effect were the stress-induced decreased levels of their mRNA transcripts. In addition to the increase in degradation of potency TF proteins and decrease in mRNA transcript levels reported here, suppression of new translation is also caused by the levels of hyperosmotic stress used here. Patel, Eur. J. Biochem., 269:3076, 2002. Simultaneous increases in protein degradation rates and decreases in synthesis rates enable rapid changes in the cellular "stemness" program. However, as stress at levels studied here continued beyond 4 h, cells adapted, cell cycle resumed, and Oct4 and Nanog mRNA and protein expression returned to near normal levels by 24 hr. The recovery of potency TF proteins was mediated by p38MAPK and PI3K signaling, as well as by that of an unknown MEK1/2 inhibitor target. Rex1 protein levels, however, did not recover; its persistent suppression was due to stress-induced JNK signaling. Table 7 summarizes the enzyme effects reported in this Example.

**Table 7. Summary of enzyme effects on Oct4, Nanog, and Rex1 following hyperosmotic stress in mESC monolayer culture.**

| Table 7 | | |
|---|---|---|
| Transcription Factor | Enzymes regulating initial stress responses; loss of TF expression at 4 hr. | Enzymes regulating long-term adaptive stress responses at 24 hr. |
| Oct4 | Mek1 >Mek2 PI3K | p38MAPK (rescue of expression) Mek2 (putatively) |
| Nanog | Mek1 >Mek2 JNK | p38MAPK (rescue of expression) PI3K |
| Rex1 | Mek1 | JNK (ongoing suppression) (no rescue enzyme identified) |

Stress-induced loss and regain of four potency TFs in mESCs by enzymes was characterized for the 1^{st} time in these studies. MEK1 mediated potency loss at the 4-h nadir of Oct4 and played a role in regain of Oct4, Nanog, and Rex1 from 4 to 24 hr. The mediator of regain of Oct4 by MEK1/2 is not clear. However, MEK1 is a weaker enzyme that blocks the stronger enzyme MEK2 in a heterodimer (Catalanotti, Nat. Struct. Mol. Biol., 16:294, 2009), and both MEK1 and MEK2 are expressed in TSCs/ESCs/blastocysts (Wang, supra) and should heterodimerize. One possible interpretation is that PD98059 suppresses MEK1 and reactivates MEK2 that reverses stress-induced Oct4 loss from 0 to 4 hr. U0126 inhibits both but importantly MEK2, blocking Oct4 recovery from 4 to 24 hr. It was hypothesized that MEK2 protects Oct4 from 0 to 4 hr unless MEK1 activity is dominant and reverses Oct4 loss from 4 to 24 hr if MEK2 activity becomes dominant (Table 7). Activity and chemical inhibitor data for MEK1 and MEK2 support this but further attempts to test this hypothesis using siRNA to MEK1 and MEK2 failed due to insufficient specificity of the knockdowns (data not shown).

JNK mediates both Nanog loss from 0 to 4 hr and continuing Rex1 loss between 4 and 24 hr. Both JNK-dependent effects would mediate predisposition toward endoderm, which is suppressed by Nanog (Frankenberg, supra; Hamazaki, J. Cell. Sci., 117:5681, 2004; Mitsui, Cell, 113:631, 2003), although Rex1 may mediate later ectoderm and specific subtypes of extraembryonic endoderm. Scotland supra; Masui, 2008. Interestingly, JNK prioritizes differentiation of TSCs stressed by hyperosmotic sorbitol, benzopyrene, or hypoxic O₂ below 2%, by inducing 1^{st} lineage (Xie, 2014; Awonuga, 2011; Abdallah, Fertil. Steril., 92(3):S136, 2009) and suppressing later lineages. Xie, 2014. JNK appears to mediate stress-induced endoderm, the 1^{st} lineage arising from ESCs.

In stressed ESCs, 1^{st} -lineage markers show that nutrient acquisition is a function associated with the increased expression of Lrp2 and Dab2 in the primitive endoderm. Lrp2 marks the earliest allocation of primitive endoderm in the E3.5 blastocyst and is accompanied by a suite of other proteins, such as cubilin and megalin (e.g., SEQ ID NO:69), that are also expressed in the earliest ExEndo in the blastocyst and are part of lipid uptake mechanisms shared by many absorptive epithelia. Frum, supra; Maurer & Cooper, J. Cell. Sci., 118:5345, 2005; Assemat, Biol. Reprod., 72:1079, 2005; Smith, BMC Dev. Biol., 6:30, 2006; Drake, Anat. Rec. A Discov. Mol. Cell. Evol. Biol., 277:163, 2004. Dab2 arises during primitive endoderm lineage fixation at E4.5 and anchors the absorptive complex in the apical surface where it may function to feed the endoderm and adjacent ICM in the blastocyst or inner stem cells subjacent to the outer visceral endoderm of embryoid bodies. Frum, supra; Yang, J. Biol. Chem. 282:13114, 2007. Biochemical evidence and evidence from the 1^{st} peri-implantation-null lethals that were endoderm specific resulted in death of the overlying primitive ectoderm by E6.0 suggesting that the early endoderm feeds the embryonic ectoderm (reviewed in Rappolee, Mol. Reprod. Dev., 52:234, 1999). Thus, induction of both Lrp2 and Dab2 by stress suggests that a primary function of stress-induced differentiation is to provide function that becomes essential rapidly after blastocyst implantation. Hyperosmotic stress begins to induce this absorptive capability in mESCs in monolayers, similar to the induction by several stressors of the placental hormone PL1 from TSCs (Xie, 2014; Awonuga, 2011; Liu, supra; Rappolee, 2013) that increases nutritional supply to the fetal-maternal interface *in vivo.*

PI3K and p38MAPK protect potency after adaptation to stress, leading to recovery of Nanog/Rex1 and Nanog/Oct4 between 4 and 24 h, respectively (Table 6). Oct4 recovery may occur for several reasons. One is that a sufficient, successful stress response occurs during the 1^{st} 4 hr and allows substantial ESC proliferation and accumulation, and thus induction of full differentiation is not required to compensate for fewer cells. Alternately, the function of early stem cells is to retain potency and divide exponentially between days E4.0 and E11.0 (Embryogenesis in mammals. (1976). Elsevier, Amsterdam; New York; McLaren. Embryo growth during the immediate postimplantation period. In Embryogenesis in mammals. (1976). Elsevier, Amsterdam; New York) so stress-induced differentiation of all cells would not be tolerated. A third hypothesis is that Oct4 function is required to mediate the ongoing stress response as established also for Oct1. Kang, Trends Biochem. Sci., 34:491, 2009; Kang, Genes Dev., 23:208, 2009; Tantin, Cancer Res., 65:10750, 2005; Riley, Dev. Biol., 284:377, 2005) If the function of transient Oct4 loss is to enable sufficient ExEndo differentiation, then this may have been accomplished and longer culture in monolayer of embryoid bodies would show that a larger subpopulation of these cells arises. The ESC survival effects corroborate previous studies for PI3K function in ESCs, TSCs, and embryos (Gross, supra; Riley, supra), but the role of mediating Oct4 loss from 0 to 4 hr is novel and unexpected.

As in the described Example, Mao, found that mESCs maintained a pluripotent phenotype during long-term exposure to hyperosmolarity, although proteins involved in both protein synthesis and degradation via the ubiquitin-proteasome system were decreased. Mao, J. Proteome Res., 7:3968, 2008. In many cell types, such as cardiomyocytes, hyperosmotic stress at these levels causes an 80% decrease in new translation within the 1^{st} 30 min. Patel Eur. J. Biochem., 269:3076, 2002. When stress decreases new translation and increases potency factor degradation, rapid, adaptive programmatic changes are possible.

The ubiquitin-proteasome system has been reported to have a role in the self-renewal and differentiation of both human and mouse ESCs (reviewed in Naujokat & Saric, Stem Cells, 25:2408, 2007). Inhibition of proteasomes decreased levels of four: Oct4, Sox2, Nanog, and Rex1 mRNAs (Vilchez, Nature, 489:304, 2012) and Oct4 and Nanog mRNAs in unstressed human ESCs and thus proteasome function is proposed to be part of potency maintenance. It is not clear from these studies or the Examples described here whether stress would increase proteasome-dependent protein destruction in human ESCs or proteasome-dependent increase in potency mRNA occurs in unstressed mouse ESCs, respectively. The activity of the 20S subunit of the proteasome is normally upregulated during mESC differentiation (Hernebring, Proc. Natl. Acad. Sci. USA, 103:7700, 2006); its suppression during hyperosmotic stress supports the finding that mESCs are not enabled to differentiate fully during extended hyperosmotic stress.

**The** reported half-life of Oct4 protein ranges from 1.5 to 8 hr. A half-life of 90 min has been reported for Oct4 protein in undifferentiated P19 (Sax, PLoS One, 4:e4467, 2009), 6-8 hr in NIH3T3-overexpressing Oct4 protein (Wei, J. Biol. Chem., 282:21551, 2007), 6.9 hr for Oct4 mRNA in mouse ESCs (Sharova, supra), and a few hours for Oct4 mRNA in differentiated cells undergoing reprogramming. Taranger, Mol. Biol. Cell, 16:5719, 2005. Oct4 is required to exclude Cdx2 in nonpolar inner cells of the blastocyst (Niwa, Cell, 123:917, 2005) and is required for FGF4 secretion that sustains adjacent trophectoderm (Arcuri, Mol. Cell. Endocrinol., 141:13, 1998; Ma, Dev. Biol., 154:45, 1992) and primitive endoderm cells in the three-dimensional (3D) blastocyst. Frum, supra; Frankenberg, supra; Cho, Development, 139:2866, 2012; Krawchuk, Dev. Biol., 384:65-71, 2013. However, Oct4 also plays a transient, cell-autonomous role in establishing primitive endoderm. Frum, supra. Importantly, Oct4 is a stress response factor that controls stem cell metabolism in mESCs in culture before and during stress (Kang, Trends Biochem. Sci., 34:491, 2009; Kang, Genes Dev., 23:208, 2009) and metabolism of cultured mouse embryos. Frum, supra. Thus, the cohort of potency TFs with Oct4 at its apex is dynamically poised under normal circumstances to regulate potency, allocation, stem cell metabolism, and stress responses.

Nanog has a half-life of 2 hr in human ESCs, and is controlled by proteasomal regulation via the PEST motif. Ramakrishna, Stem Cells Dev., 20:1511, 2011. In mESCs, a half-life of 5.2 hr has been reported for Nanog mRNA. Sharova, supra. Rex1 half-life has been reported to be from 30 min (Gontan, Nature, 485:386, 2012) to 2.2 hr (Sharova, supra) in mESCs (Gontan, supra). In mESC system, inhibition of the proteasome for 4 hr led to a 3-fold increase in Rex1 expression, suggesting a short half-life of this protein. The rapid turnover of Rex1 as compared with Oct4 and Nanog allows it to respond more quickly to changing conditions. That Rex1 was the only pluripotency marker that did not recover to normal expression levels during stress is unique, as it normally recovers quickly during fluctuations in expression. Toyooka, supra. This may be due to increased protein destruction, JNK-dependent silencing of the Rex1 promoter despite Oct4 (Boyer, supra), or JNK-dependent phosphorylation of Oct4 that decreases binding to the Rex1 promoter. These data suggest that the stressed mESC monolayer may act more like the epithelial embryonic ectoderm of E5.5 that has lost Rex1 since differentiating from the ICM, but still expresses Oct4, Nanog, and Sox2. During normal, unstressed mESC culture, subpopulations of both low-Rex1- and high-Rex1-expressing cells exist. Toyooka, supra. Low-Rex1-expressing cells have poor ability to differentiate into primitive endoderm, and predominantly differentiate to primitive ectoderm lineages. High-Rex1-expressing mESCs were pluripotent and, upon reinjection to embryos, they contributed to multilineage chimeras. Toyooka, supra. These populations were interconvertible during culture without added stress; low Rex1 expressors could revert back to high Rex1 expression regaining developmental potential, and vice versa.

In the current Example, hyperosmotic stress maintains low Rex1 expression in mESCs and suppresses interconversion back to high Rex1 expression. This result led to the expectation that the stressed mESCs would induce epiblast markers, such as FGF5, due to their inverse regulation. Pelton, J. Cell. Sci., 115:329, 2002. This did not prove to be the case, however, as epiblast was suppressed while primitive endoderm markers were induced. Rex1 expression is required for complete development of extraembryonic lineages. Rex1-negative mESCs were defective in some visceral endoderm markers although the major marker, α-feto protein (AFP), was expressed (Masui, 2008) and Rex1-/-F9 teratocarcinoma stem cells were only able to differentiate to parietal endoderm. Thompson & Gudas, Mol. Cell. Endocrinol., 195:119, 2002. The data suggest that Rex1 plays a role in directing lineages of endoderm differentiation in F9 cells but data should be interpreted cautiously as, unlike mESCs, AFP requires Rex1 expression and F9 cells may not emulate complex regulatory mechanisms of mESCs. In this Example, persistence of some Rex1 expression in stressed cells presumably allows mESC priming toward primitive endoderm. However, the persistent suppression of Rex1 expression raises the question of whether visceral endoderm downstream of primitive endoderm will be decreased in favor of parietal endoderm. Brown, supra.

In the current Example, JNK activation by continuous hyperosmotic stress suppressed Rex1 expression, but was not adequate to trigger outright differentiation of mESCs. JNK may not be activated long enough to irreversibly commit ESCs to differentiation, or there may be insufficient activation of other enzymes needed to complement JNK-induced differentiation, or insufficient 3D interactions may occur. Unlike TSCs, ESCs may require the 3D interactions available in embryoid bodies. However, it was not defined whether JNK directly decreased Rex1 protein or indirectly suppressed it during induction of differentiation. In each case of stress-induced differentiation of mESCs and TSCs, JNK was active within a pathway required to produce a new lineage, but in none of the cases was it capable of initiating differentiation on its own. JNK may work with other differentiation cues and enzymatic mechanisms. But during the stress response, the activation of multiple pathways with competing effects prevented outright or complete differentiation of large cell subpopulations. For example, JNK suppressed Rex1 expression during 24 hr of stress, while PI3K signaling was simultaneously maintaining Rex1, preventing total Rex1 protein loss. This additional signaling masked but did not negate JNK's action on Rex1. Of course if a single ESC lost the three potency TFs undergoing transient stress-induced loss, and the continuing loss of Rex1 for a long enough period, then it might differentiate irreversibly.

Finally, the stress-induced differentiation of TSCs occurred even in the presence of FGF4 signaling that sustains their multipotent state. Xie, 2014; Xie, 2013; Awonuga, 2011; Liu, supra; Zhou, 2011. Stress signaling was dominant over FGF4 signaling at doses where stem cell populations expanded, but at diminished rates. In the current Example, mESCs integrated stress-response signals with potentially competing signals from exogenous LIF and bone morphogenetic protein (BMP). The cytokine LIF promotes mESC self-renewal by activating the TF STAT3. BMPs are a serum component that induces expression of inhibitor of differentiation genes that block expression-lineage-specific TF function and facilitate the self-renewal response to LIF/STAT3. Ying, Cell, 115:281, 2003. In the described system, the integration of all these signals during hyperosmotic stress led to preservation of a pluripotent population; stress was not dominant in overt changes in mESC monolayers. However, *in vivo* the pluripotent ICM is a transient stage that stem cells move through to populate the lineages that will eventually make up the embryo and its support cells. LIF in the four-cell-stage embryo and interleukin-6 at the blastocyst stage are necessary to maintain phosphorylated STAT3 and Oct4 in order to maintain pluripotency *in vivo.* Do, Genes Dev., 27:1378, 2013. If stress occurred in an environment that was more characteristic of the nonepithelial ICM, one where differentiation was not repressed, then the stress activation of the differentiation program may proceed unimpeded. Thus, stress may drive preimplantation ICM to Ex-Endo but suppress other lineages that arise at gastrulation.

The data suggest that the period when stem cells 1^{st} allocate at E3.5, differentiate soon after, and mediate gastrulation at E6.5 is critical and can be affected critically by stress. The understanding of how stress enzymes mount successful adaptation to stress through regulation of potency and differentiation-mediating TFs during this critical period is of profound importance. The goal is to understand the mechanisms and thresholds between healthy, adaptive and unhealthy, maladaptive responses that are important in the general population but also during medical procedures such as in vitro fertilization that alleviate infertility.

Example 3. The embryo undergoes stress *in vivo* and in vitro and this decreases developmental rates, stem cell growth rates and causes potency loss (Puscheck, Molecular Biology of the Stress Response in the Early Embryo and its Stem Cells. In: Cell Signalling During Mammalian Early Embryo Development. Henry Leese DB ed. Springer, 2015 (Puscheck, 2015, MB); Xie, 2011). ESCs respond to hyperosmotic stress with transient loss of Oct4 and long-term loss of Rex1 proteins in a proteasome-dependent manner and this correlates with induction of 1^{st} lineage and suppression of later lineages during stress (Example 2; Slater, Stem Cells Dev., 23:3049, 2014). Early embryos at the 2-cell and blastocyst-stage also undergo stress-induced potency loss as do placental trophoblast stem cells (TSCs) derived from the blastocyst (Xie, 2013; Zhou, 2011).

Oct4 and Rex1. Oct4 is a DNA-binding transcription factor that mediates stemness in gametes and early embryos and in pluripotent cells through the start of gastrulation (Pesce & Scholer, Mol. Reprod. Dev., 55:452, 2000). Oct4 null lethality occurs at the blastocyst stage when ICM lose pluripotency and fail to synthesize FGF4 (Nichols, Cell, 95:379, 1998) that maintains adjacent polar trophectoderm (Chai, 1998). Oct4 promoter methylation is diminished in oocytes undergoing in vitro maturation (IVM) (Milroy, Fertil. Steril., 95t:2094, 2011) and Oct4 expression is decreased in embryos derived from smoke-exposed mouse females (Huang, Fertil. Steril., 92:1456, 2009). Oct1 and Oct4 have been used to test for toxic stress in HTS of embryonic stem cells ESCs *(*Pal, J. Cell. Physiol., 226:1583, 2011) and both transcription factors have stress domains that prepare stem cells for stress and are phosphorylated and regulate the stress response (Kang, Genes Dev., 23:208, 2009). Rex1 is also a transcriptional factor that is lost from ICM as they differentiate to either extra-embryonic primitive endoderm or embryonic primitive ectoderm fates (Toyooka, supra) but its null is not lethal at the blastocyst stage and it is not required to initiate and maintain pluripotency of ESCs (Rezende, Dev. Biol., 356:370, 2011). Taken together the data suggest that Rex1-driven reporters should be more useful than Oct4-driven reporters to create HTSs for toxicants or other stressful stimuli that negatively affect embryo and stem cell stemness and differentiation, thus depleting stem cells and imbalancing differentiation.

In order to produce modified reporter mESCs without the stress of electroporation or cationic lipids, lentivirus infection can be used (Kaufman, Theriogenology, 82:1043, 2014) and in place of antibiotic selection ESCs are passaged in the normal manner but fluorescence activated cell sorted (FACS) to select stem cells reporting pluripotency. This creates viable potency reporter ESC lines with normal growth rates and without cell loss during infection and selection. These mESCs also report potency factor activity loss using 1) visual microscopic inspection, 2) microplate readers, 3) FACS, and 4) immunoblots. The assays provide similar results as in Example 2. The analysis by flow cytometry also provides important new information on the size of the bright (potent) subpopulation that goes to zero potency and the microplate gives a platform of high throughput analysis of toxicants or other potential stressors that may induce potency loss and for performing high throughput screens to further elucidate the mechanisms of potency loss.

Materials. Germline competent mESC-D3 cells were purchased from ATCC (Manassas, VA). DMEM medium was from HyClone (Logan, UT). Gibco^{™} glutamine and sodium pyruvate supplement solutions were from Life Technologies (Grand Island, NY). ESC-qualified EmbryoMax fetal bovine serum, 0.1% gelatin solution and ESGRO^{™} Mouse LIF medium supplement were from EMD Millipore (Billerica, MA). Oct4 promoter reporter lentiviral particles that express the green fluorescent protein copGFP and TranDux^{™} transduction reagent were from System Biosciences (Mountain View, CA). Rex1 promoter reporter lentiviral particles that express the red fluorescent protein mApple were from Allele Biotechnology (San Diego, CA). Rabbit anti-Rex1 antibody was from Abcam (Cambridge, MA). Rabbit anti-cleaved caspase 3 antibody, Rabbit anti-p-actin antibody and HRP-conjugated second antibodies were from Cell Signaling Technology (Danvers, MA). Pierce RIPA lysis buffer, protease inhibitor cocktail and BCA protein assay reagents were from Thermo Scientific (Rockford, IL). ECL chemiluminescence reagent was from GE Healthcare Bio-Sciences (Pittsburgh, PA). MEM nonessential amino acid solution, sorbitol, 2-mercaptoethanlol and other chemicals were from Sigma (St. Louis, MO).

Low stress production of single and double viable potency activity reporter mouse ESCs. mESCs were cultured in 24-well plates pre-coated with 0.1% gelatin. The starting confluence of cells was 20% or 100,000 cells/well. The cells were incubated in 37°C for 2-3 hr for attachment and readiness for virus infection. Virus infection medium was made by mixing Oct4 and/or Rex1 promoter reporter lentiviral particles into regular growth medium supplemented with TransDux. The final concentration of the viral particles was 10⁶/ml. The infection medium was applied to mESCs at 400 µl per well. After infection, the cells were cultured for 3 days to allow the expression of the fluorescent reporter proteins. After expansion of the total cell number by passaging, the infected cells were subjected to flow cytometry sorting using BD FACS Vantage SE cell sorter (BD Biosciences, San Jose, CA) at the Wayne State University Microscopy, Imaging and Cytometry Resources (MICR) core facility. Pure, fluorescent cells were obtained after two FACS repeats. But, as indicated in FIG. 25A and 25B and as previously reported by Toyooka and colleagues Toyooka, supra) heterogeneity reestablishes after sorting and culture and normally a small population becomes Rex1 dim.

mESC culture and stimulation. Germline competent mESC-D3 cells (ATCC, Manassas, VA, (Doetschman, J. of Embryology and Experimental Morphology, 87:27, 1985)) were cultured in the absence of feeder cells in DMEM (Gibco, Grand Island, NY) supplemented with 15% mESC-screened fetal bovine serum (HyClone, Logan, UT), 2mM L-glutamine, 1 mM sodium pyruvate, 1 mM nonessential amino acids, 0.1 mM 2-mercaptoethanol (Sigma, St. Louis, MO), and 1000 U/mL murine leukemia inhibitory factor (LIF; Millipore, Temecula, CA) on 0.1% gelatin-coated dishes at 37°C in humidified air with 5% CO₂ (Masui, 2008). mESCs were cultured overnight after passaging before stimulation with sorbitol. Osmolality of ESC media with and without added 200 mM sorbitol was determined as in Example 2.

Fluorescence determination by plate reader of pluripotency reporters. mESCs that stably express Oct4-copGFP and/or Rex1-mApple promoter reporters were plated on 96-well plates at a starting confluence of 5%. Four identical plates were made for the time course study. Cells were grown for 24 hr in incubator till 10% confluence was reached; an addition of stimuli was designated time zero. The cells were stressed for a time period selected from 1, 2, or 3 days. At each time point, the medium was removed and the cells were rinsed with D-PBS. At 50 µl/well, ESCs were measured for fluorescence using the Synergy H1 microplate reader (BioTek, Winooski, VT). The excitation and detection wavelengths for Oct4-copGFP were 485nm and 528nm, respectively. The excitation and detection wavelengths for Rex1-mApple were 568nm and 611nm, respectively. After live-cell reading, the cells were lysed with 50 µL of lysis buffer (1% NP-40, 0.5% TX-100, 100 mM NaCl, 2mM EDTA, 50 mM Tris-HCI, and pH 7.5) and measured for fluorescence with the same parameters above. After fluorescence determination, the cell lysates were further measured for total protein concentrations by adding 100 µl of Pierce BCA protein assay reagent (Thermo Scientific Inc.) following the manufacturer's instructions. Finally, the fluorescence readings from either live-cells or lysates were normalized to total protein concentration readings, in which higher readings represent higher pluripotency of the cells.

Flow cytometric analysis of mESCs expressing pluripotency reporters. Fluorescence determination by the plate reader reported an averaged biochemical result from a population of cells inside the tissue culture vessel. In order to get insight into the heterogenic nature of the cells that respond to the same stress condition, a flow cytometry assay that provides quantification of cells that show different levels of pluripotency under stress was used. mESCs expressing potency reporter Oct4-CopGFP or Rex1-mApple were plated at a starting confluence of 10% and grown for 24 hr until reaching 25% confluence. This time point was designated as time zero. The cells were then treated with different sorbitol concentrations for 1-3 days. At each time point, cells were trypsinized and re-suspended in ice-cold D-PBS for flow cytometry assay using an LSR Fortessa flow cytometer (BD biosciences) and the FACS Diva Software version 7.0 (BD biosciences). Non-stressed mESCs that express Oct4-copGFP and/or Rex1-mApple and the non-modified, parental mESCs were used to set up the parameters that define the fluorescent cells and non-fluorescent cells.

From the histogram of FACS, two peaks are seen. The right peak represents the bright fluorescent potent ESCs. The left peak represents the dim fluorescent non-potent ESCs. An arbitrary vertical line is drawn here at the 5 x 10³ position on the PE-A axis to separate the low potency ESCs. The peak areas were calculated using Image J by counting the area pixels.

Immunoblot. After treatment, the ESCs were rinsed with PBS, lysed with RIPA buffer supplemented with protease inhibitors and sonicated briefly. Total protein concentration was determined using Pierce BCA protein assay reagent admixed with Laemmli sample buffer, and boiled for 5min. Equal total protein amounts from the lysates were size-fractionated by 5-20% gradient SDS-PAGE and transferred onto nitrocellulose membrane. Rex1 immunofluorescent signals were detected using ECL reagent with X-ray films and quantified using ImageJ 1.48v software (NIH, USA).

Statistics. Data collected over at least, three independent experiments were analyzed using SPSS Version 22.0 and presented as means ± SEM. Data distributions met the assumptions of independent *t*-test and one-way ANOVA. Dunnett's post hoc test was performed following significant ANOVA test. For FIG. 23A and 23B, Independent *t*-tests were performed to examine the difference between control and treatment for Oct and Rex. Significance was detected for Rex (*p*=0.019) but not for Oct. For FIG. 24, One-way ANOVA was performed followed by Dunnett's post hoc test using 0 mM as reference. All treatments were different from the control (highlighted p-value in excel). For FIGs. 25 and 26, One-way ANOVA was performed followed by Dunnett's post hoc test using 0 mM as reference for parental dim, intermediate dim, and bright separately. Differences were detected for parental dim and bright groups, but not for intermediate dim (highlighted p-value in excel). For FIG. 27: One-way ANOVA was performed followed by Dunnett' s post hoc test using 0 mM as reference. Differences between treatments and/or groups were considered insignificant if p ≧0.05 and significant if *p*< 0.05.

Results. It was previously shown that Oct4, Sox2, and Nanog transcription factor protein underwent stress-induced transient loss at 4 hr, rebounding to baseline pre-stressed condition by 24 hr and later. However, Rex1 protein loss due to stress is not transient but chronic. Here Rex1-RFP ESCs were developed and their kinetic and dose-dependent responses to stress were tested. To create the Rex1-RFP vector, and compare it with Oct4-GFP activity in response to stress, ESCs were infected with lentivirus with either single vector or both vectors (FIG. 22A, 22B, 22C). Note that single infected cells were red for Rex1-RFP or Oct4-GFP, but expression of both produced yellow emissions (FIG. 22C). This was done with no cell loss in comparison with huge cell death using either electroporation or cationic lipids. In a second step modified ESCs, were trypsinized as for a normal passage, and a fluorescence-activated cell sorter (FACS) was used to purify Rex1-RFP (FIG. 22D), Oct4-GFP (FIG. 22E), and dual Rex1-RFP/Oct4-GFP expressing ESCs (FIG. 22F). To test for the stress response of these three modified ESCs they were incubated for 3 days with LIF and with or without 200 mM sorbitol, the sub-morbid dose that created a strong Rex1 protein loss as in Example 2. Oct4-GFP fluorescence intensity was similar without (FIG. 22G) or with (FIG. 22J) stress, but Rex1-RFP fluorescence was clearly higher without stress (FIG. 22H) than with stress (FIG. 22K). In corroboration,-d culture of Rex1-RFP/Oct4-GFP dual expressing cells were not affected by culture without stress (FIG. 221), but stress decreased Rex1-RFP, removing red from yellow and re-exposing green fluorescence from Oct4-GFP (FIG. 22L). Thus, the visual data suggest that Rex1-RFP activity reporter is decreased in viable ESCs after 3 days of hyperosmotic stress.

The amount of stress-induced loss of Rex1-RFP or Oct4-GFP was next quantitated using a microplate reader. It was found that stress mediated by 200 mM sorbitol, despite the presence of LIF, induces a significant loss of Rex1-RFP but not Oct4-GFP (FIG. 23A,23B). This is in general agreement with the loss of Rex1 protein but not Oct4 protein after 24 hr of stress. However, the loss of Rex1 protein is greater than the loss of Rex1 activity detected by the multimerized Rex1 response elements driving RFP fluorescence assay in the microplate reader.

In the test of Oct4-GFP and Rex1-RFP reporter ESCs 200 mM sorbitol was used as described in Example 2. However, higher doses of sorbitol created increasing potency loss and toxicity. To test for higher dose-dependent stress, increases were used in increments of 25mM from 200-300 mM sorbitol for three days in ESC culture with LIF present. A dose-dependent increased Rex1-RFP loss was found in triplicate biological experiments in which stress was dominant to Rex1 activity as measured by the microplate reader (FIG. 24). Although Rex1-RFP activity was less at 200 mM sorbitol, by 300 mM the 60% loss of Rex1 activity measured here was as high as the Rex1 protein loss reported previously and in FIG.27.

To further define the nature of the response of ESCs to hyperosmotic stress, changes in the potency of ESC subpopulations using flow cytometry were tested. It was found that there was an increase in the dim subpopulation that was at 10² arbitrary intensity units, the level of fluorescence of non-modified parental ESCs (FIG. 25A and 25B, FIG. 26A and 26B). Unstressed ESCs had 8.6+/-1.0% dim cells but this increased to 24.4+/-2.9% by 300 mM sorbitol, despite the presence of LIF. In contrast 83.3+/-3.9% of unstressed cells were in the bright group (near 10⁴ arbitrary intensity units), but this decreased to 61.8+/-3.7% at 300 mM. There was a small increase in intermediate dim cells, from 10.7+/-1.2 % to 13.8+/-2.1%. The 60% decrease in Rex1-RFP from 0 to 300 mM sorbitol determined by microplate reader in FIG. 24 is matched by a total change toward intensity decrease of 40.4% of cells; 3.1 % increase in intermediate dim ESCs, 15.8% increase in parental dim ESCs, and decrease of 21.5% in the bright group (FIG. 25A and 25B, FIG. 26A and 26B). The additional decrease in brightness determined by microplate reader compared with subpopulation change producing decreased brightness may be a result of increased changes in brightness/cell.

Finally it was tested whether endogenous Rex1 protein levels decreased commensurately with decreases in Rex1-RFP activity levels assayed in FIG. 24. Rex1-RFP ESCs were cultured for 3 days with 0-300 mM sorbitol and assayed by immunoblot for endogenous Rex1 levels normalized to ACTB loading controls and with time zero unstressed Rex1 set to 100 as a baseline level. Whereas stress-induced decrease in Rex1 activity in FIG. 24 were 20% and 70% at 200 mM and 300 mM sorbitol, respectively endogenous Rex1 protein losses were 30% and 70% at 200 mM and 300 mM, respectively (FIG. 27). Through the series from 200 mM to 300 mM sorbitol there was generally good agreement between activity level and endogenous protein level for Rex1 at 3 days of stress and with Rex1 protein loss at 1 day (Example 2). To correlate stress-induced Rex1 potency loss with stress-induced diminished cell growth rates, cell counts were performed at time zero and at 3 days over the same sorbitol dose range as in FIG. 27 and FIG. 24 and FIG. 26A and 26B. As in the past, potency loss was directly proportional to diminished growth, suggesting that the differentiation enabled by potency loss would compensate for fewer cells. Stress-diminished cell counts were also multiplied by stress-increased subpopulations of intermediate dull and parental dull stem cells that had loss potency and for several doses from 200-250 mM sorbitol the product, which equaled total numbers of cells which lost potency, was constant (Table 8, doses from 200, 225, 250 have constant numbers of cells with lost Rex1-RFP; see Brief Description of the Figures for Table 8). Interestingly, in the 200-250 mM sorbitol range cell doubling rates decreased but in a lineage range between 34.1-39.3 hr, but total numbers of intermediate + parental dim potency loss cells decrease at 275-300 mM where doubling rates fell off to 51.4-103.4 hr. This is also predicted by the theory of stress-induced compensatory differentiation (Puscheck, 2015, MB; Xie, 2014). Taken together, dose-dependent stress effects on cell growth rates, Rex1 endogenous protein levels, decreases in Rex1 active subpopulation and increases in Rex1 low activity subpopulations suggest that stress induces potency loss in a coherent manner.

The goals of this Example were to 1) test whether four lines of evidence show consistent outcomes for potency factor activity reporter ESCs and biochemical data for stress-induced potency loss, 2) validate these reporter ESCs for use in HTS for toxicology, and 3) to further test the theory of stress-induced compensatory differentiation of stem cells.

The 4 lines of evidence confirm that Rex1-RFP potency activity reporter ESCs show consistency between micrographic analysis, biochemical averages from immunoblots and microplate reader, and subpopulation studies using flow cytometry. Endogenous Rex1 protein levels are consistent with Rex1-RFP activity levels measured in the same cells under the same stress exposures.

Rex1-RFP is a sensitive stress reporter and will enable an HTS for developmental toxicology of manufactured compounds, environmental pollutants, new Pharma and cosmetics and fluid collected during IVF ART protocols. These include follicular, vaginal, uterine, amniotic fluids and spent media. Hyperosmotic stress, which is a "gold standard" for discovery and characterization of stress enzymes *(Xie,* 2011), was used in this Example. There is also an exemplar set of toxicants used for the only government-validated ESC toxicology assay (Genschow, European Centre for the Validation of Alternative Methods. Altern Lab Anim, 30:151, 2002, and these will be used to further test the validity of the Rex1-RFP HTS. Similar results are expected; more particularly, however, similar in "shape" of the dose response curve. Because Genschow, used an ESC cytotoxicity outcome which requires high doses, the compensatory differentiation assay should be more sensitive in that differentiation is induced at lower doses than cytotoxicity.

The theory of stress-induced compensatory differentiation (Xie, 2011; Puscheck, 2015, MB; Rappolee, Syst. Biol. Reprod. Med., 56:168, 2010) states that stress 1^{st} diminishes normal stem cell population expansion and then compensates for fewer stem cells by differentiating more of the remaining stem cells to produce essential 1^{st} lineage differentiated function. This occurs despite the presence of growth factors that would normally maintain proliferation and potency and thus stress destroys potency. There are three lines of evidence for the theory. One is that dose-dependent stress diminishes stem cell growth and that was observed previously in ESCs (Example 2) and TSCs (Zhou, 2011; Puscheck, 2015, MB; Xie, 2014; Awonuga, 2011; Zhong, 2010; Xie, 2010; Abdallah, Fertility and Sterility, 92(3):S136, 2009) and here. A second, previously-established line of evidence is that stress induces increases in stress enzymes proportional to average decreases in potency factors and increases in differentiation factors in biochemical assays such as immunoblots and qPCR. A third prediction is that there is a putative subpopulation of stem cells that decreases potency and increases differentiation. Because the modeled stressed embryo is under duress, this prediction includes the stipulation that the subpopulation cell number should be constant; a minimal size but not larger.

The stress dose-dependent cell number of low potency cells can be determined by multiplying the fractional subpopulation size of cells with potency loss, which increases with stress and the decreasing cell numbers with increasing stress. This was done in Table 8 and compared with a previously published Venn diagram for compensatory differentiation ( Puscheck, 2015, MB; Rappolee, 2013), modified to produce FIG. 28. In the stress-dose range where population size decreases, but increasing doubling rate is in a manageable range, the absolute size of the subpopulation of potency loss (intermediate and very low dim cells) is remarkably the same. At stress doses where very large doubling rate increases occur the subpopulation number with potency loss decreases, probably due to stem cell morbidity at these higher hyperosmotic stress levels (Example 2). Thus all three lines of evidence summarized here are consistent with the theory, but with limitations.

**A** Pdgfra-GFP ESC line can be tested for stress effects on differentiation of the 1^{st} lineage ESC-derived extra-embryonic endoderm that this line reports (Artus, Development, 137:3361, 2010). This will provide an even more direct test of stress-induced differentiation and the regulation and number of cells in the subpopulation undergoing stress dose-dependent potency loss and differentiation increase. Without being bound by theory, it is hypothesized that Pdgfra-GFP cells will derive primarily from the parental dim subpopulation of Rex1-RFP cells, but differentiated cells may arise from the intermediate dim or even bright groups of Rex1-RFP cells, but at lower frequencies.

It should be noted that a recent high impact paper claimed that a three-step stress regimen induces potency during reprogramming of adult differentiated cells (Obokata, Nature, 505:641, 2014) however this report was retracted (Obokata, Nature, 511:112, 2014). Many stresses were used (Xie, 2011); Puscheck, 2015, MB) and others have also shown (Soares, Trends Endocrinol. Metab., 18:114, 2007) that stress can induce differentiation in placental and embryonic stem cells TSCs and ESCs of the early rodent embryo. The modified stem cells (e.g., Rex1-RFP and Pdgfra-GFP potency and 1^{st} differentiated lineage ESC)) and their use in HTSs will be invaluable in rapidly compiling quantitative data on stress-induced differentiation from large sets of stressors of interest to toxicologists and reproductive scientists.

Any embodiment (e.g., modified stem cells, assays, HTSs) can be used in combination with any of the following markers of interest for pregnancy outcome including Cofillin (NM_ 005507), DIAPH1 (NM_001079812, NM_005219), ECT2 (NM_018098), MYLC2/MYL5 (NM_ 002477), DGCR8 (NM_022720), Dicer/DICER1 (NM_030621, NM_177438), TARBP2 (NM_ 004178, NM_134323, NM_134324), CPEB1 (NM_001079533, NM_001079534, NM_ 001079535, NM_030594), Symplekin/SYMPK (NM_004819), YBX2 (NM_015982), ZAR1 (NM_ 175619), CTNNB1 (NM_001098209, NM_001098210, NM_001098210, NM_001904), DNMT3B (NM_006892, NM_175848, NM_175849, NM_175850), TERT (NM_198253, NM_ 198255), YY1 (NM_003403), IFGR2/IFNGR2 (NM_005534), BTF3 (NM_001037637, NM_ 001207), and NELF (NM_001130969, NM_001130970, NM_001130971, NM_015537).

As will be understood by one of ordinary skill in the art, each embodiment disclosed herein can comprise, consist essentially of or consist of its particular stated element, step, ingredient or component. Thus, the terms "include" or "including" should be interpreted to recite: "comprise, consist of, or consist essentially of." As used herein, the transition term "comprise" or "comprises" means includes, but is not limited to, and allows for the inclusion of unspecified elements, steps, ingredients, or components, even in major amounts. The transitional phrase "consisting of' excludes any element, step, ingredient or component not specified. The transition phrase "consisting essentially of" limits the scope of the embodiment to the specified elements, steps, ingredients or components and to those that do not materially affect the embodiment. As used herein, a material effect would cause a statistically-significant reduction in the ability of a method to detect stress in a stem cell.

Unless otherwise indicated, all numbers expressing quantities of ingredients, properties such as molecular weight, reaction conditions, and so forth used in the specification and claims are to be understood as being modified in all instances by the term "about." Thus, unless indicated to the contrary, the numerical parameters set forth in the specification and attached claims are approximations that may vary depending upon the desired properties sought to be obtained by the present invention. At the very least, and not as an attempt to limit the application of the doctrine of equivalents to the scope of the claims, each numerical parameter should at least be construed in light of the number of reported significant digits and by applying ordinary rounding techniques. When further clarity is required, the term "about" has the meaning reasonably ascribed to it by a person skilled in the art when used in conjunction with a stated numerical value or range, i.e. denoting somewhat more or somewhat less than the stated value or range, to within a range of ±20% of the stated value; ±19% of the stated value; ±18% of the stated value; ±17% of the stated value; ±16% of the stated value; ±15% of the stated value; ±14% of the stated value; ±13% of the stated value; ±12% of the stated value; ±11% of the stated value; ±10% of the stated value; ±9% of the stated value; ±8% of the stated value; ±7% of the stated value; ±6% of the stated value; ±5% of the stated value; ±4% of the stated value; ±3% of the stated value; ±2% of the stated value; or ±1% of the stated value.

Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard deviation found in their respective testing measurements.

The terms "a," "an," "the" and similar referents used in the context of describing the invention (especially in the context of the following claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein is intended merely to better illuminate the invention and does not pose a limitation on the scope of the invention otherwise claimed. No language in the specification should be construed as indicating any non-claimed element essential to the practice of the invention.

Groupings of alternative elements or embodiments of the invention disclosed herein are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

CE of this invention are described herein, including the best mode known to the inventors for carrying out the invention. Of course, variations on these described embodiments will become apparent to those of ordinary skill in the art upon reading the foregoing description. The inventor expects skilled artisans to employ such variations as appropriate.

Furthermore, numerous references have been made to patents, printed publications, journal articles and other written text throughout this specification (referenced materials herein).

The particulars shown herein are by way of example and for purposes of illustrative discussion of the preferred embodiments of the present invention only and are presented in the cause of providing what is believed to be the most useful and readily understood description of the principles and conceptual aspects of various embodiments of the invention. In this regard, no attempt is made to show structural details of the invention in more detail than is necessary for the fundamental understanding of the invention, the description taken with the drawings and/or examples making apparent to those skilled in the art how the several forms of the invention may be embodied in practice.

Definitions and explanations used in the present disclosure are meant and intended to be controlling in any future construction unless clearly and unambiguously modified in the following examples or when application of the meaning renders any construction meaningless or essentially meaningless. In cases where the construction of the term would render it meaningless or essentially meaningless, the definition should be taken from Webster's Dictionary, 3^{rd} Edition or a dictionary known to those of ordinary skill in the art, such as the Oxford Dictionary of Biochemistry and Molecular Biology (Ed. Anthony Smith, Oxford University Press, Oxford, (2004)).

## Claims

1. A screening method of detecting stress in stem cells exposed to a compound to be screened, the method comprising:
measuring the expression or activity of one or more of Oct4; Sox2; Nanog; Rex1; TEAD4; Errβ; Lrp2; Dab2; Fgf5; Pdgfra; Sox17; Gata4/6; PAX8; NEFL; TSHR; Brachyury; Laminin; AFP; Nestin; Goosecoid; MEK1; MEK2; and AKT in the presence of fibroblast growth factor 4 (FGF4) for murine trophoblast stem cells (TSCs) or leukemia inhibitory factor (LIF) for murine embryonic stem cells (ESCs);
comparing the measurement to a reference level of expression or activity, which is derived from previous measures from a population of stem cells which have not been exposed to the compound to be screened; and
determining that the stem cells have experienced stress if the measurement is statistically significantly reduced or increased from the reference level,
wherein the method screens compounds for their stress effects on the stem cells, and
wherein a statistically significantly reduction in Oct4; Sox2; Nanog; Rex1; TEAD4; or Errβ is indicative of a stress experience, or
a statistically significantly increase in Lrp2; Dab2; Fgf5; Pdgfra; Sox17; Gata4/6; PAX8; NEFL; TSHR; Brachyury; Laminin; AFP; Nestin; Goosecoid; MEK1; MEK2; or AKT is indicative of a stress experience.

2. The method of claim 1 comprising measuring the expression or activity of Oct4 and/or Rex1 wherein a statistically significantly reduction in comparison to the reference level is indicative of a stress experience.

3. The method of claim 1 comprising measuring the expression or activity of Lrp2, Dab2, Fgf5, and/or Pdgfra wherein a statistically significantly increase in comparison to the reference level is indicative of a stress experience.

4. The method of claim 1 comprising measuring the expression or activity of Pdgfra, Sox17, Dab2, Lrp2 or Gata6 wherein a statistically significantly increase in comparison to the reference level is indicative of a stress experience.

5. The method of claim 1 comprising measuring the expression or activity of Brachyury or FGF5 wherein a statistically significantly increase in comparison to the reference level is indicative of a stress experience.

6. The method of claim 1 comprising measuring the expression or activity of Rex1 and Pdgfra wherein a statistically significantly reduction in of Rex 1 comparison to the reference level is indicative of a stress experience and a statistically significantly increase of Pdgfra in comparison to the reference level is indicative of a stress experience.

## Patentansprüche

1. Screening-Verfahren zum Erkennen von Stress in Stammzellen, die einer zu screenenden Verbindung ausgesetzt sind, wobei das Verfahren Folgendes umfasst:
Messen der Expression oder Aktivität von Oct4; Sox2; Nanog; Rex1; TEAD4; Errß; Lrp2; Dab2; Fgf5; Pdgfra; Sox17; Gata4/6; PAX8; NEFL; TSHR; Brachyury; Laminin; AFP; Nestin; Goosecoid; MEK1; MEK2; und/oder AKT in der Gegenwart von Fibroblasten-Wachstumsfaktor 4 (fibroblast growth factor- FGF4) für murine Trophoblasten-Stammzellen (trophoblast stem cells - TSCs) oder Leukämieinhibitionsfaktor (LIF) für murine embryonale Stammzellen (embryonic stem cells - ESCs);
Vergleichen der Messung mit einem Referenzwert von Expression oder Aktivität, der aus früheren Messungen von einer Population von Stammzellen abgeleitet ist, die der zu screenenden Verbindung nicht ausgesetzt waren; und
Bestimmen, dass die Stammzellen Stress erfahren haben, wenn die Messung gegenüber dem Referenzwert statistisch signifikant verringert oder erhöht ist,
wobei das Verfahren Verbindungen auf ihre Stresswirkungen auf die Stammzellen screent, und
wobei eine statistisch signifikante Verringerung von Oct4; Sox2; Nanog; Rex1; TEAD4; oder Errß eine Stresserfahrung anzeigt, oder
eine statistisch signifikante Erhöhung von Lrp2; Dab2; Fgf5; Pdgfra; Sox17; Gata4/6; PAX8; NEFL; TSHR; Brachyury; Laminin; AFP; Nestin; Goosecoid; MEK1; MEK2; oder AKT eine Stresserfahrung anzeigt.

2. Verfahren nach Anspruch 1, das das Messen der Expression oder Aktivität von Oct4 und/oder Rex1 umfasst, wobei eine statistisch signifikante Verringerung im Vergleich zu dem Referenzwert eine Stresserfahrung anzeigt.

3. Verfahren nach Anspruch 1, das das Messen der Expression oder Aktivität von Lrp2, Dab2, Fgf5 und/oder Pdgfra umfasst, wobei eine statistisch signifikante Erhöhung im Vergleich zu dem Referenzwert eine Stresserfahrung anzeigt.

4. Verfahren nach Anspruch 1, das das Messen der Expression oder Aktivität von Pdgfra, Sox17, Dab2, Lrp2 oder Gata6 umfasst, wobei eine statistisch signifikante Erhöhung im Vergleich zu dem Referenzwert eine Stresserfahrung anzeigt.

5. Verfahren nach Anspruch 1, das das Messen der Expression oder Aktivität von Brachyury oder FGF5 umfasst, wobei eine statistisch signifikante Erhöhung im Vergleich zu dem Referenzwert eine Stresserfahrung anzeigt.

6. Verfahren nach Anspruch 1, das das Messen der Expression oder Aktivität von Rex1 und Pdgfra umfasst, wobei eine statistisch signifikante Verringerung von Rex 1 im Vergleich zu dem Referenzwert eine Stresserfahrung anzeigt und eine statistisch signifikante Erhöhung von Pdgfra im Vergleich zu dem Referenzwert eine Stresserfahrung anzeigt.

## Revendications

1. Procédé de criblage pour détecter un stress dans des cellules souches exposées à un composé à cribler, le procédé comprenant :
la mesure de l'expression ou de l'activité d'un ou de plusieurs de Oct4 ; Sox2 ; Nanog ; Rex1 ; TEAD4 ; Errβ ; Lrp2 ; Dab2 ; Fgf5 ; Pdgfra ; Sox17 ; Gata4/6 ; PAX8 ; NEFL ; TSHR ; Brachyury ; Laminine ; AFP ; Nestine ; Goosecoid ; MEK1 ; MEK2 ; et AKT en présence de facteur de croissance des fibroblastes 4 (FGF4) pour les cellules souches trophoblastiques murines (TSC) ou le facteur d'inhibition de la leucémie (LIF) pour les cellules souches embryonnaires murines (ESC) ;
la comparaison de la mesure à un niveau de référence d'expression ou d'activité, qui est dérivé de mesures précédentes à partir d'une population de cellules souches qui n'ont pas été exposées au composé à cribler ; et
la détermination du fait que les cellules souches ont subi un stress si la mesure est réduite ou augmentée de manière statistiquement significative par rapport au niveau de référence,
dans lequel le procédé crible les composés pour leurs effets de stress sur les cellules souches, et
dans lequel une réduction statistiquement significative de Oct4 ; Sox2 ; Nanog ; Rex1 ; TEAD4 ; ou Errβ indique une expérience de stress, ou
une augmentation statistiquement significative de Lrp2 ; Dab2 ; Fgf5 ; Pdgfra ; Sox17 ; Gata4/6 ; PAX8 ; NEFL ; TSHR ; Brachyury ; Laminine ; AFP ; Nestine ; Goosecoid ; MEK1 ; MEK2 ; ou AKT indique une expérience de stress.

2. Procédé selon la revendication 1, comprenant la mesure de l'expression ou de l'activité d'Oct4 et/ou de Rex1, dans lequel une réduction statistiquement significative par rapport au niveau de référence indique une expérience de stress.

3. Procédé selon la revendication 1, comprenant la mesure de l'expression ou de l'activité de Lrp2, Dab2, Fgf5 et/ou Pdgfra dans lequel une augmentation statistiquement significative par rapport au niveau de référence indique une expérience de stress.

4. Procédé selon la revendication 1, comprenant la mesure de l'expression ou de l'activité de Pdgfra, Sox17, Dab2, Lrp2 ou Gata6 dans lequel une augmentation statistiquement significative par rapport au niveau de référence indique une expérience de stress.

5. Procédé selon la revendication 1, comprenant la mesure de l'expression ou de l'activité de brachyure ou de FGF5, dans lequel une augmentation statistiquement significative par rapport au niveau de référence indique une expérience de stress.

6. Procédé selon la revendication 1, comprenant la mesure de l'expression ou de l'activité de Rex1 et de Pdgfra, dans lequel une réduction statistiquement significative de la comparaison de Rex 1 par rapport au niveau de référence indique une expérience de stress et une augmentation statistiquement significative de Pdgfra par rapport au niveau de référence indique une expérience de stress.
